(19) Europäisches Patentamt
European Patent Office
Office européen des brevets

(11) EP 4 375 286 A1

(12) **EUROPEAN PATENT APPLICATION**
published in accordance with Art. 153(4) EPC

(43) Date of publication:
**29.05.2024 Bulletin 2024/22**

(21) Application number: **22852134.0**

(22) Date of filing: **01.08.2022**

(51) International Patent Classification (IPC):
***C07D 493/10*** *(2006.01)* ***A61K 31/35*** *(2006.01)*
***A61K 31/4433*** *(2006.01)* ***A61P 25/04*** *(2006.01)*

(52) Cooperative Patent Classification (CPC):
**A61K 31/35; A61K 31/4433; A61P 25/04; C07D 493/10**

(86) International application number:
**PCT/CN2022/109503**

(87) International publication number:
**WO 2023/011422 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2021 CN 202110888850**
**02.08.2021 CN 202110895052**

(71) Applicants:
- **Shujing Biopharma Co., Ltd**
  **Shanghai 201208 (CN)**
- **Jiangsu NHWA Pharmaceutical Co., Ltd**
  **Xuzhou, Jiangsu 221000 (CN)**

(72) Inventors:
- **FENG, Jiaquan**
  **Shanghai 201208 (CN)**
- **DENG, Xinshan**
  **Shanghai 201208 (CN)**
- **WAN, Zehong**
  **Shanghai 201208 (CN)**

(74) Representative: **Henderson, Helen Lee**
**Withers & Rogers LLP**
**2 London Bridge**
**London SE1 9RA (GB)**

# (54) OXASPIRO DERIVATIVE, AND PREPARATION METHOD THEREFOR AND USE THEREOF

(57) The present invention relates to an oxaspiro derivative, and a preparation method therefor and use thereof. Specifically, provided are a compound as represented by general formula (I), a stereoisomer thereof, a tautomer thereof, or a pharmaceutically acceptable salt thereof, and a preparation method therefor, the use thereof for activating the activity of an opioid receptor, and the use thereof in the preparation of an analgesic drug.

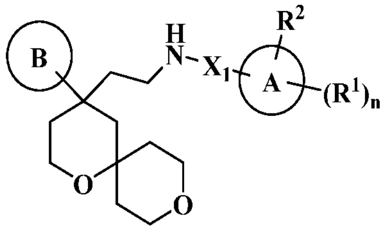

(I)

EP 4 375 286 A1

## Description

The present application claims priority to Chinese Patent Application Nos. 202110895052X and 202110888850X filed on Aug. 2, 2021, which is incorporated herein by reference in its entirety.

## TECHNICAL FIELD

The present invention belongs to the field of pharmaceuticals, and particularly relates to an oxaspiro derivative, a preparation method therefor and use thereof.

## BACKGROUND

Opioid receptors are one of the members of the G protein-coupled receptor (GPCR) family. At present, 9 opioid receptors, μ, δ, κ, ORL1, and the like, have been found to be widely expressed in central and peripheral nerves, as well as in neuroendocrine cells, immune cells, and endothelial cells. μ(MOR), δ(DOR), and κ (KOR) opioid receptors regulate a range of body behaviors through the central nervous system, including pain, emotion, stress response, and addictive behaviors. The μ, δ, and κ opioid receptors are classical opioid receptors, and the three types of receptors have the same basic structure, namely having an extracellular amino-terminal region and an intracellular carboxyl-terminal region. The three subtypes of opioid receptor agonists couple primarily to Gi-type G protein, dissociating the β and γ subunits from the α subunit of G protein. The β, γ and α subunits mediate activation of multiple intracellular signaling pathways, respectively, such as inhibition of adenylate cyclase activity and activation of G protein-coupled receptor kinase (GRK), protein kinase C (PKC), and mitogen-activated protein kinase (MAPK). The cerebral cortex and various regions under the cortex are involved in MOR-mediated antinociceptive responses. Some areas show special functions, primarily modulating sensory of pain (like ipsilateral nucleus accumbens and amygdala) or emotional (like anterior cingulate cortex) response. Studies have shown that activation of amygdala MOR mediates antinociceptive responses. In the ACC region, activation of MOR during persistent pain is negatively correlated with the pain-specific MPQ emotion score.

High levels of opioid receptors are expressed in the ACC region. Opioids are widely used in the treatment of persistent pain and have been suggested to play a role in the brain. It has been reported that morphine injected in the ACC region inhibited the pain emotional response, and as a result, no change was found in pain sensation of nerve-injured animals, suggesting that the mechanism of pain emotion and pain sensation were different. Later studies have demonstrated that injection of different doses of MOR agonist DAMGO inside the rostral portion of the anterior cingulate cortex can provide dose-dependent relief of the pain emotional response induced by injection of complete Freund's adjuvant. Other studies have found that after MOR in nucleus accumbens is activated, release of presynaptic glutamic acid is reduced, and the functional activity of an NMDA receptor is suddenly increased, so that the NMDA receptor in basolateral amygdala of amygdala influences pain emotional response of acute pain and chronic pain caused by formalin. A large number of NMDA receptors are also distributed in ACC encephalic region neurons, participate in learning and memory of the central nervous system, and have various functions such as pain sensation modulation.

The mechanism of the analgesic effect with respect to opioids is now clearly known: in the process of transmitting pain to the center, the pain stimulates sensory nerve endings and releases glutamic acid (Glu), and the glutamic acid acts on corresponding receptors to complete the transmission of pain impulses to the center to cause pain. Exogenous opioids or endogenous opioid peptides stimulate opioid receptors on presynaptic and postsynaptic membranes of sensory nerves, and inhibit adenylate cyclase from degrading ATP to generate cAMP through a G protein coupling mechanism so as to inhibit the release of presynaptic membrane neurotransmitters such as P substance and acetylcholine, promote the outflow of $K^+$, reduce the inflow of $Ca^{2+}$, and finally weaken the pain signal to produce an analgesic effect.

Many agonists and antagonists of opioid receptors have been discovered over years of research. Agonists of opioid receptors discovered so far are: morphine, DAMGO, endomorphin, fentanyl and derivatives thereof, and the like. Naloxone (Nal), CTOP, CTAP, naltrexone, and the like are antagonists of opioid receptors. Naloxone is characterized by short onset time, strong antagonistic ability, strong lipophilicity, and relatively short duration of drug effect. Naloxone is a potent, broad-spectrum opioid antagonist that can function by competing for opioid receptors (in an order of μ, κ, and δ) and is frequently associated with an agonistic effect, i.e., an agonistic-antagonistic combining effect. It can relieve the problems of intoxication caused by excessive opioids and postoperative continuous respiratory depression, and can identify and diagnose drug addicts. Remifentanil has similar analgesic, sedative, respiratory depressing effects and the like with other μ opioid receptor agonists, and has no obvious binding force with non-opioid receptors, while its binding to opioid receptors can be competitively inhibited by naloxone. The analgesic effect is dose-dependent and has a "ceiling effect", but common adverse effects still exist, such as hypotension, muscle rigidity, bradycardia, nausea and vomiting, respiratory depression that is easily caused during local anesthesia or postoperative analgesia, and the like. Moreover, the opioids have side effects such as tolerance, respiratory depression, and constipation after long-term use.

Therefore, there is an urgent need to develop a novel opioid receptor agonist that can act on the G protein

pathway, have fewer side effects and higher activity, effectively treat a related disease mediated by a μ-opioid receptor agonist, and particularly have a significant analgesic effect, so as to meet the huge market demand.

## SUMMARY

The present invention aims to provide a compound of general formula (I), a stereoisomer thereof, a tautomer thereof, or a pharmaceutically acceptable salt thereof,

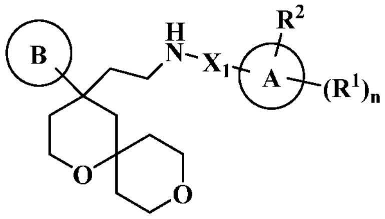

(I)

wherein:

ring A is $C_{6-10}$ aryl or 5- to 6-membered heteroaryl, wherein the 5- to 6-membered heteroaryl is 5- to 6-membered heteroaryl containing 1-3 heteroatoms selected from a nitrogen atom, an oxygen atom, and a sulfur atom;
$R^1$ are the same or different, and $R^1$ and $R^2$ are each independently hydrogen, halogen, hydroxy, amino, nitro, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl is optionally further substituted with one or more substituents selected from $C_{3-6}$ cycloalkyl, $C_{1-6}$ alkyl, and halogen, preferably hydrogen, halogen, hydroxy, $C_{1-3}$ alkyl, $C_{1-3}$ haloalkyl, $C_{1-3}$ alkoxy, or $C_{1-3}$ haloalkoxy, more preferably hydrogen, fluorine, chlorine, bromine, hydroxy, methyl, ethyl, propyl, methoxy, ethoxy, halomethyl, haloethyl, or halomethoxy, further preferably hydrogen, fluorine, chlorine, bromine, methyl, methoxy, or halomethoxy, still further preferably hydrogen, fluorine, chlorine, bromine, methyl, or methoxy;
ring B is phenyl or pyridinyl, optionally further substituted with 1-4 $R^3$;
$R^3$ are the same or different and are each independently hydrogen, halogen, hydroxy, amino, nitro, cyano, $C_{1-6}$ alkyl, $C_{1-6}$ haloalkyl, $C_{1-6}$ alkoxy, or $C_{1-6}$ haloalkoxy, preferably hydrogen, fluorine, chlorine, bromine, or $C_{1-3}$ alkyl, more preferably hydrogen, fluorine, chlorine, or bromine;
$X_1$ is $CR^aR^b$ or $CR^aR^bCH_2$, preferably $CH_2$, $CH(R^b)$, or $CH(R^b)CH_2$;
$R^a$ and $R^b$ are each independently hydrogen, halogen, or $C_{1-3}$ alkyl;
or $R^a$ or $R^b$ is linked to $R^2$ to form one cyclopentyl or cyclohexyl and is optionally further substituted with one or more substituents selected from fluorine, chlorine, bromine, methyl, ethyl, propyl, methoxy, ethoxy, halomethyl, haloethyl, halomethoxy, and haloethoxy; preferably, $R^a$ or $R^b$ is linked to $R^2$ to form one cyclopentyl;
n is 0, 1, 2, or 3.

In a further preferred embodiment of the present invention, for the compound of general formula (I), the stereoisomer thereof, the tautomer thereof, or the pharmaceutically acceptable salt thereof, ring A is phenyl.

In a further preferred embodiment of the present invention, for the compound of general formula (I), the stereoisomer thereof, the tautomer thereof, or the pharmaceutically acceptable salt thereof, $R^1$ and $R^2$ are each independently hydrogen, halogen, $C_{1-6}$ alkyl, or $C_{1-6}$ haloalkoxy, preferably hydrogen, halogen, $C_{1-3}$ alkyl, or $C_{1-3}$ haloalkoxy, more preferably hydrogen, fluorine, chlorine, bromine, methyl, ethyl, propyl, or halomethoxy, further preferably hydrogen, fluorine, chlorine, bromine, methyl, or fluoromethoxy.

In a further preferred embodiment of the present invention, for the compound of general formula (I), the stereoisomer thereof, the tautomer thereof, or the pharmaceutically acceptable salt thereof, $R^1$ is independently hydrogen, halogen, or $C_{1-6}$ alkyl, preferably hydrogen, halogen, or $C_{1-3}$ alkyl, more preferably hydrogen, fluorine, chlorine, bromine, methyl, ethyl, or propyl, further preferably hydrogen, fluorine, chlorine, bromine, or methyl.

In a further preferred embodiment of the present invention, for the compound of general formula (I), the stereoisomer thereof, the tautomer thereof, or the pharmaceutically acceptable salt thereof, $R^1$ is hydrogen, halogen, $C_{1-6}$ alkyl, $C_{1-6}$ alkoxy, or $C_{1-6}$ haloalkoxy, preferably hydrogen, halogen, $C_{1-3}$ alkyl, or $C_{1-3}$ haloalkoxy, more preferably hydrogen, fluorine, chlorine, bromine, methyl, ethyl, propyl, or halomethoxy, further preferably hydrogen, fluorine, chlorine, methyl, or fluoromethoxy.

In a further preferred embodiment of the present invention, for the compound of general formula (I), the stereoisomer thereof, the tautomer thereof, or the pharmaceutically acceptable salt thereof, $R^2$ is hydrogen, halogen, $C_{1-3}$

alkyl, or $C_{1-3}$ haloalkoxy, preferably hydrogen.

In a further preferred embodiment of the present invention, for the compound of general formula (I), the stereoisomer thereof, the tautomer thereof, or the pharmaceutically acceptable salt thereof, ring B is pyridinyl, optionally further substituted with one $R^3$.

In a further preferred embodiment of the present invention, for the compound of general formula (I), the stereoisomer thereof, the tautomer thereof, or the pharmaceutically acceptable salt thereof, $R^3$ are each independently hydrogen or halogen, preferably hydrogen, fluorine, chlorine, or bromine, more preferably hydrogen or fluorine.

In a further preferred embodiment of the present invention, for the compound of general formula (I), the stereoisomer thereof, the tautomer thereof, or the pharmaceutically acceptable salt thereof, $X_1$ is $CR^aR^b$ or $CR^aR^bCH_2$; $R^a$ and $R^b$ are each independently hydrogen, halogen, or $C_{1-3}$ alkyl, preferably hydrogen.

In a further preferred embodiment of the present invention, for the compound of general formula (I), the stereoisomer thereof, the tautomer thereof, or the pharmaceutically acceptable salt thereof, $X_1$ is $CR^aR^b$, preferably $CH_2$.

In a further preferred embodiment of the present invention, for the compound of general formula (I), the stereoisomer thereof, the tautomer thereof, or the pharmaceutically acceptable salt thereof, $X_1$ is $CR^aR^b$ or $CR^aR^bCH_2$; $R^a$ or $R^b$ is linked to $R^2$ to form one cyclopentyl or cyclohexyl, preferably cyclopentyl.

In a further preferred embodiment of the present invention, for the compound of general formula (I), the stereoisomer thereof, the tautomer thereof, or the pharmaceutically acceptable salt thereof, $X_1$ is $CH(R^b)$ or $CH(R^b)CH_2$; $R^b$ is linked to $R^2$ to form one cyclopentyl or cyclohexyl, preferably cyclopentyl.

In a further preferred embodiment of the present invention, for the compound of general formula (I), the stereoisomer thereof, the tautomer thereof, or the pharmaceutically acceptable salt thereof, n is 0, 1, or 2.

In a further preferred embodiment of the present invention, for the compound of general formula (I), the stereoisomer thereof, the tautomer thereof, or the pharmaceutically acceptable salt thereof, ring B is

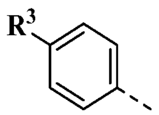, 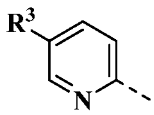, or 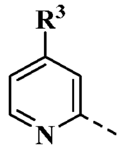,

preferably

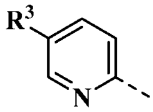,

more preferably

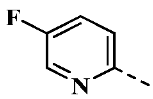 or 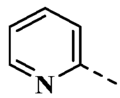,

further preferably

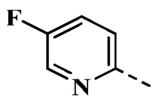.

A preferred embodiment of the present invention aims to provide a compound of general formula (II), a stereoisomer thereof, a tautomer thereof, or a pharmaceutically acceptable salt thereof,

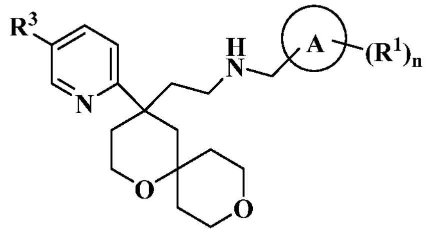

(II)

wherein:

ring A, $R^1$, $R^3$, and n are as described in general formula (I).

In a further preferred embodiment of the present invention, for the compound of general formula (II), the stereoisomer thereof, the tautomer thereof, or the pharmaceutically acceptable salt thereof, general formula (II) further has a structure represented by general formula (II-1):

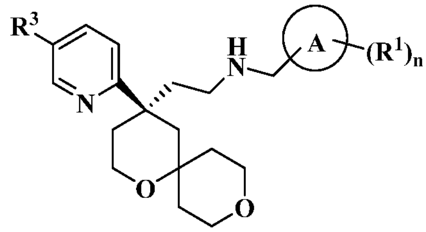

(II-1)

wherein:

ring A, $R^1$, $R^3$, and n are as described in general formula (I).

In a further preferred embodiment of the present invention, for the compound of general formula (II) or general formula (II-1), the stereoisomer thereof, the tautomer thereof, or the pharmaceutically acceptable salt thereof,

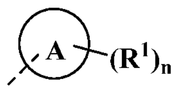

is

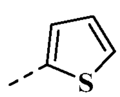 , 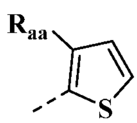 , 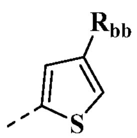 , 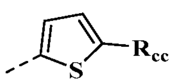 , 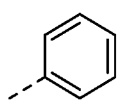 , 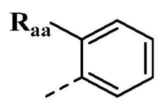 ,

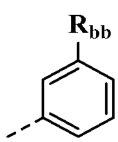 , 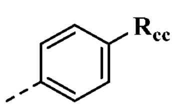 , 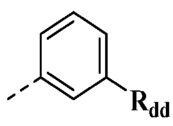 , 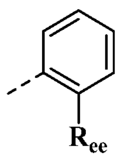 , 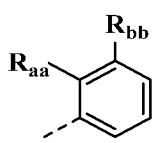 , 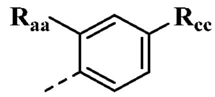 ,

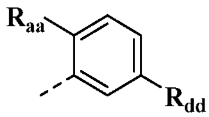, 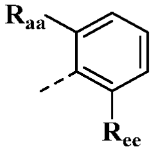, 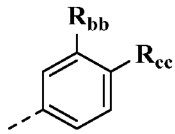, 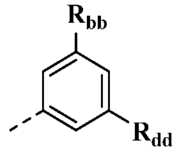, 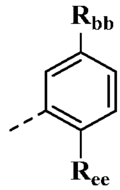, 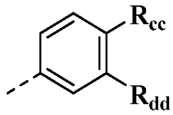,

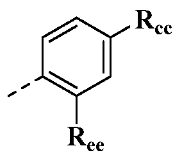, or 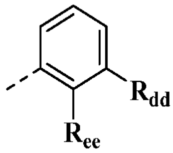;

preferably

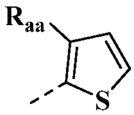, 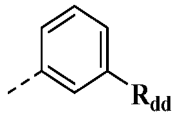, or 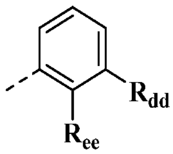;

wherein $R_{aa}$, $R_{bb}$, $R_{cc}$, $R_{dd}$, and $R_{ee}$ are each independently halogen, hydroxy, $C_{1-6}$ alkyl, $C_{1-6}$ haloalkyl, $C_{1-6}$ alkoxy, or $C_{1-6}$ haloalkoxy, wherein the $C_{1-6}$ alkyl is optionally further substituted with one or more substituents selected from $C_{3-6}$ cycloalkyl, $C_{1-6}$ alkyl, and halogen; preferably halogen, hydroxy, $C_{1-3}$ alkyl, $C_{1-3}$ haloalkyl, $C_{1-3}$ alkoxy, or $C_{1-3}$ haloalkoxy; more preferably fluorine, chlorine, bromine, hydroxy, methyl, ethyl, propyl, methoxy, ethoxy, halomethyl, haloethyl, or halomethoxy; further preferably fluorine, chlorine, bromine, methyl, methoxy, or halomethoxy; still further preferably fluorine, chlorine, bromine, methyl, or methoxy.

In a further preferred embodiment of the present invention, for the compound of general formula (II) or general formula (II-1), the stereoisomer thereof, the tautomer thereof, or the pharmaceutically acceptable salt thereof,

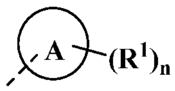

is

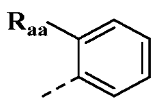, 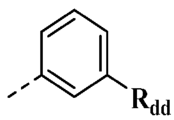, or 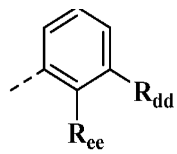.

In a further preferred embodiment of the present invention, for the compound of general formula (II) or general formula (II-1), the stereoisomer thereof, the tautomer thereof, or the pharmaceutically acceptable salt thereof, $R_{aa}$, $R_{bb}$, $R_{cc}$, $R_{aa}$, and $R_{ee}$ are each independently halogen, $C_{1-6}$ alkyl, $C_{1-6}$ alkoxy, or $C_{1-6}$ haloalkoxy, preferably halogen, $C_{1-3}$ alkyl, or $C_{1-3}$ haloalkoxy, more preferably fluorine, chlorine, bromine, methyl, ethyl, propyl, or halomethoxy, further preferably fluorine, chlorine, bromine, methyl, or fluoromethoxy.

In a further preferred embodiment of the present invention, for the compound of general formula (II) or general formula (II-1), the stereoisomer thereof, the tautomer thereof, or the pharmaceutically acceptable salt thereof, $R_{aa}$ is halogen or $C_{1-6}$ haloalkoxy, preferably halogen or $C_{1-3}$ haloalkoxy, more preferably fluorine, chlorine, bromine, or halomethoxy, further preferably chlorine or fluoromethoxy.

In a further preferred embodiment of the present invention, for the compound of general formula (II) or general formula (II-1), the stereoisomer thereof, the tautomer thereof, or the pharmaceutically acceptable salt thereof, $R_{dd}$ is halogen, more preferably fluorine, chlorine, or bromine, further preferably chlorine.

In a further preferred embodiment of the present invention, for the compound of general formula (II) or general formula (II-1), the stereoisomer thereof, the tautomer thereof, or the pharmaceutically acceptable salt thereof, $R_{ee}$ is halogen or $C_{1-6}$ alkyl, preferably halogen or $C_{1-3}$ alkyl, more preferably fluorine, chlorine, bromine, methyl, ethyl, or propyl, further preferably fluorine, chlorine, or methyl.

In a further preferred embodiment of the present invention, for the compound of general formula (II) or general formula (II-1), the stereoisomer thereof, the tautomer thereof, or the pharmaceutically acceptable salt thereof,

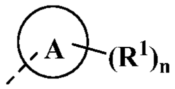

is

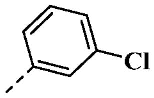 , 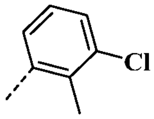 , 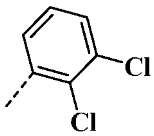 , 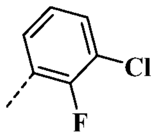 , 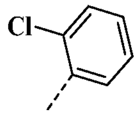 ,

or

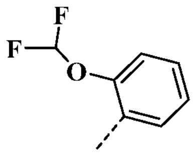 .

A preferred embodiment of the present invention aims to provide a compound of general formula (III), a stereoisomer thereof, a tautomer thereof, or a pharmaceutically acceptable salt thereof,

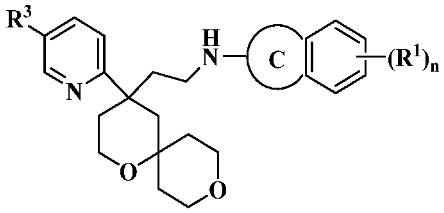

(III)

wherein:

ring C is cyclopentyl;
$R^1$, $R^3$, and n are as described in general formula (I).

In a further preferred embodiment of the present invention, for the compound of general formula (III), the stereoisomer thereof, the tautomer thereof, or the pharmaceutically acceptable salt thereof, general formula (III) further has a structure represented by general formula (IV) or general formula (V):

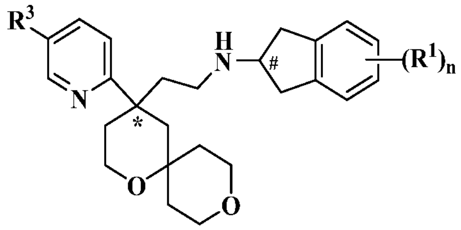
(IV)

or

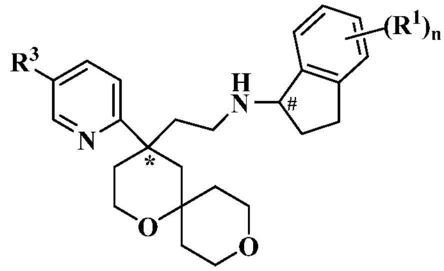
(V)

wherein:

- $R^1$, $R^3$, and n are as described in general formula (III);
- the carbon atom with "*" is a chiral carbon atom present in a form of a single (R) or (S) enantiomer or in a form enriched with a pair of enantiomers;
- the carbon atom with "#" is a chiral carbon atom present in a form of a single (R) or (S) enantiomer or in a form enriched with a pair of enantiomers.

In a further preferred embodiment of the present invention, for the compound of general formula (IV) or general formula (V), the stereoisomer thereof, the tautomer thereof, or the pharmaceutically acceptable salt thereof,

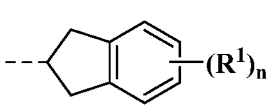 is 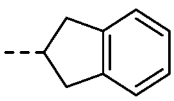, 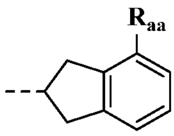, 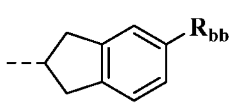, 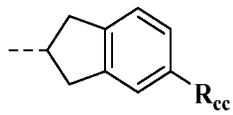,

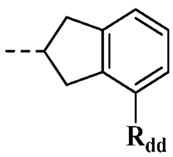, 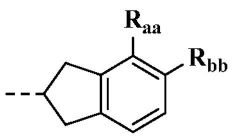, 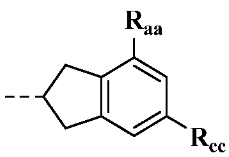, 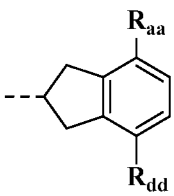, 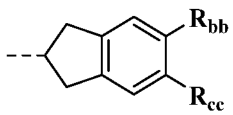,

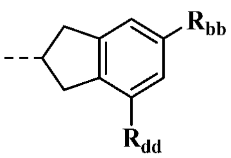, or 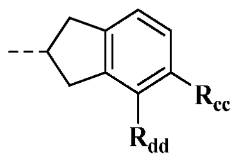,

preferably

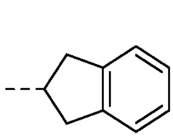, 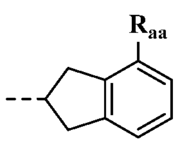,

or ;

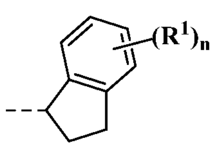

is

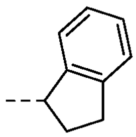, 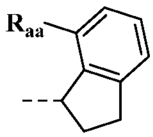, 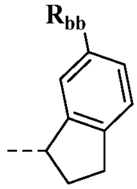, 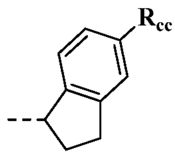, 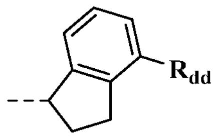,

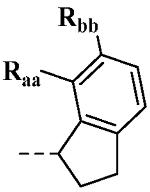, 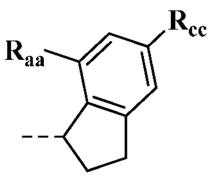, 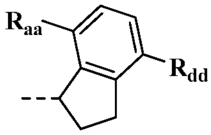, 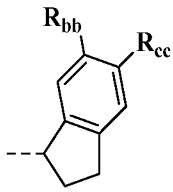, 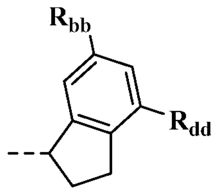,

or

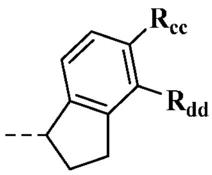,

preferably

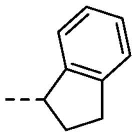, 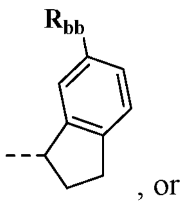, or 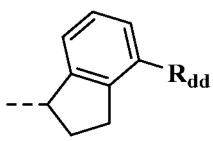;

wherein $R_{aa}$, $R_{bb}$, $R_{cc}$, and $R_{dd}$ are each independently halogen, hydroxy, $C_{1-6}$ alkyl, $C_{1-6}$ haloalkyl, $C_{1-6}$ alkoxy, or $C_{1-6}$ haloalkoxy, wherein the $C_{1-6}$ alkyl is optionally further substituted with one or more substituents selected from $C_{3-6}$ cycloalkyl, $C_{1-6}$ alkyl, and halogen; preferably halogen, hydroxy, $C_{1-3}$ alkyl, $C_{1-3}$ haloalkyl, $C_{1-3}$ alkoxy, or $C_{1-3}$ haloalkoxy; more preferably fluorine, chlorine, bromine, hydroxy, methyl, ethyl, propyl, methoxy, ethoxy, halomethyl, haloethyl, or halomethoxy; further preferably fluorine, chlorine, bromine, methyl, methoxy, or halomethoxy; still further preferably fluorine, chlorine, bromine, methyl, or methoxy.

In a further preferred embodiment of the present invention, for the compound of general formula (IV) or general formula (V), the stereoisomer thereof, the tautomer thereof, or the pharmaceutically acceptable salt thereof, $R_{aa}$, $R_{bb}$, $R_{cc}$, and $R_{dd}$ are each independently halogen or $C_{1-6}$ alkyl, preferably halogen or $C_{1-3}$ alkyl, more preferably fluorine, chlorine, bromine, methyl, ethyl, or propyl, further preferably fluorine, chlorine, bromine, or methyl.

In a further preferred embodiment of the present invention, for the compound of general formula (IV) or general formula (V), the stereoisomer thereof, the tautomer thereof, or the pharmaceutically acceptable salt thereof, $R_{aa}$ is halogen, preferably fluorine, chlorine, or bromine, more preferably chlorine or bromine.

In a further preferred embodiment of the present invention, for the compound of general formula (IV) or general formula (V), the stereoisomer thereof, the tautomer thereof, or the pharmaceutically acceptable salt thereof, $R_{bb}$ is halogen, preferably fluorine, chlorine, or bromine, more preferably fluorine or chlorine.

In a further preferred embodiment of the present invention, for the compound of general formula (IV) or general formula (V), the stereoisomer thereof, the tautomer thereof, or the pharmaceutically acceptable salt thereof, $R_{dd}$ is halogen or $C_{1-6}$ alkyl, preferably halogen or $C_{1-3}$ alkyl, more preferably fluorine, chlorine, bromine, methyl, ethyl, or propyl, further preferably fluorine, chlorine, bromine, or methyl.

In a further preferred embodiment of the present invention, for the compound of general formula (IV), the

stereoisomer thereof, the tautomer thereof, or the pharmaceutically acceptable salt thereof,

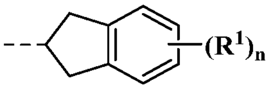

is

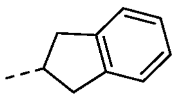, 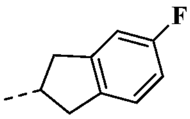, 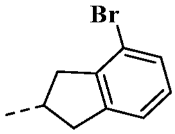, or 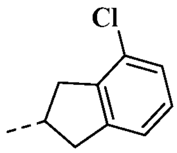.

In a further preferred embodiment of the present invention, for the compound of general formula (V), the stereoisomer thereof, the tautomer thereof, or the pharmaceutically acceptable salt thereof,

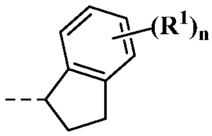

is

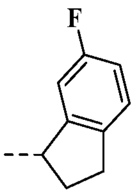, 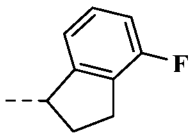, 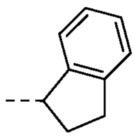, 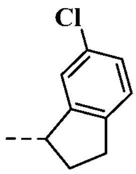, 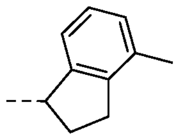,

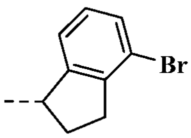, or 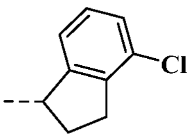.

In a further preferred embodiment of the present invention, for the compound of general formula (IV), the stereoisomer thereof, the tautomer thereof, or the pharmaceutically acceptable salt thereof, general formula (IV) further has a structure represented by general formula (IV-1):

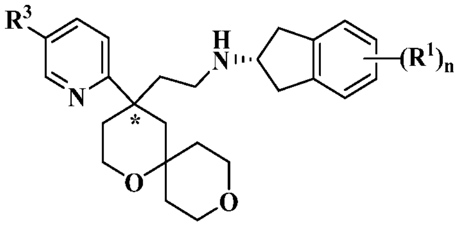

(IV-1)

wherein:

$R^1$, $R^3$, and n are as described in general formula (IV);

the carbon atom with "*" is a chiral carbon atom present in a form of a single (R) or (S) enantiomer or in a form

enriched with a pair of enantiomers.

In a further preferred embodiment of the present invention, for the compound of general formula (IV), the stereoisomer thereof, the tautomer thereof, or the pharmaceutically acceptable salt thereof, general formula (IV) further has a structure represented by general formula (IV-2):

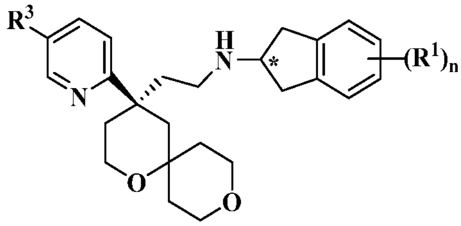

(IV-2)

wherein:

$R^1$, $R^3$, and n are as described in general formula (IV);
the carbon atom with "*" is a chiral carbon atom present in a form of a single (R) or (S) enantiomer or in a form enriched with a pair of enantiomers.

In a further preferred embodiment of the present invention, for the compound of general formula (IV-1), the stereoisomer thereof, the tautomer thereof, or the pharmaceutically acceptable salt thereof, general formula (IV-1) further has a structure represented by general formula (IV-1-1) or general formula (IV-1-2):

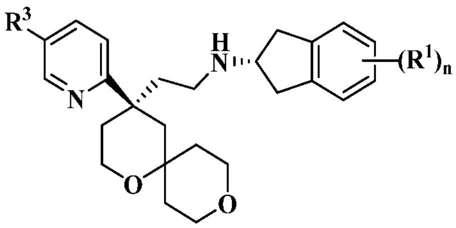

(IV-1-1)

or

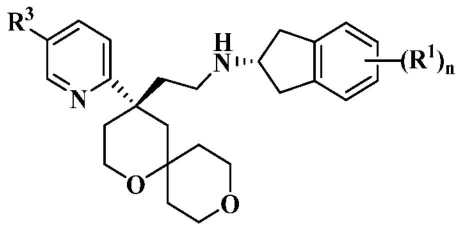

(IV-1-2)

wherein:
$R^1$, $R^3$, and n are as described in general formula (IV-1).

In a further preferred embodiment of the present invention, for the compound of general formula (V), the stereoisomer thereof, the tautomer thereof, or the pharmaceutically acceptable salt thereof, general formula (V) further has a structure represented by general formula (V-1):

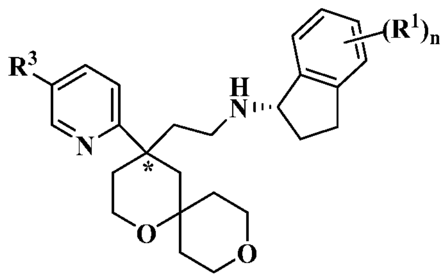

(V-1)

wherein:

$R^1$, $R^3$, and n are as described in general formula (V);
the carbon atom with "*" is a chiral carbon atom present in a form of a single (R) or (S) enantiomer or in a form enriched with a pair of enantiomers.

In a further preferred embodiment of the present invention, for the compound of general formula (V), the stereoisomer thereof, the tautomer thereof, or the pharmaceutically acceptable salt thereof, general formula (V) further has a structure represented by general formula (V-2):

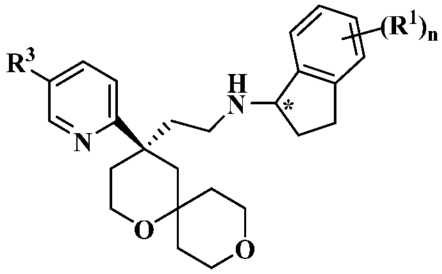

(V-2)

wherein:

$R^1$, $R^3$, and n are as described in general formula (V);

the carbon atom with "*" is a chiral carbon atom present in a form of a single (R) or (S) enantiomer or in a form enriched with a pair of enantiomers.

In a further preferred embodiment of the present invention, for the compound of general formula (V-1), the stereoisomer thereof, the tautomer thereof, or the pharmaceutically acceptable salt thereof, general formula (V-1) further has a structure represented by general formula (V-1-1) or general formula (V-1-2):

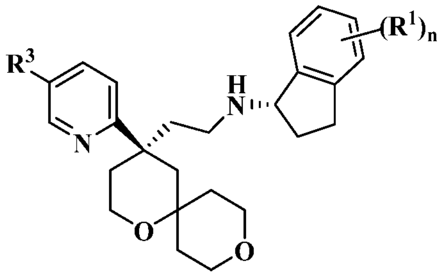

(V-1-1)

or

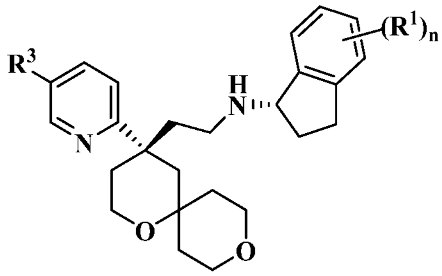

(V-1-2)

wherein:

$R^1$, $R^3$, and n are as described in general formula (V-1).

In one embodiment of the present invention, the compound is of any one of the following structures:

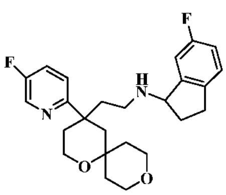

1

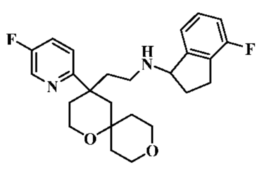

2

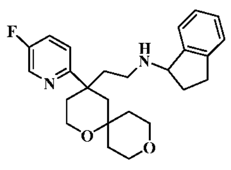

3

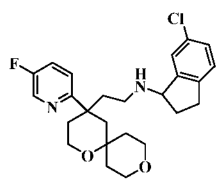

4

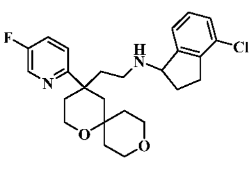

5

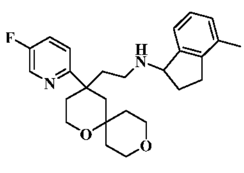

6

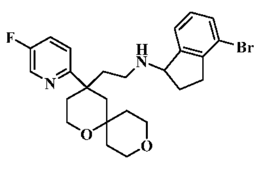

7

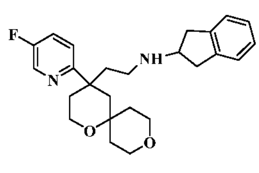

8

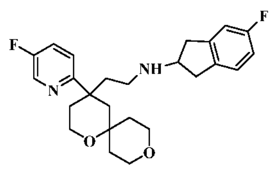

9

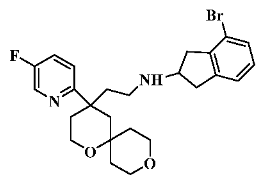

10

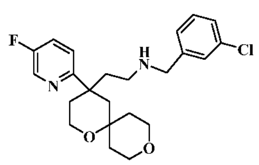

11

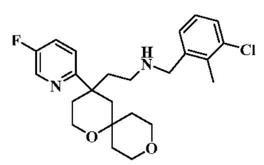

12

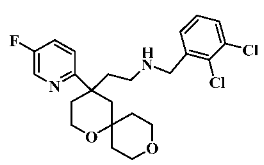

13

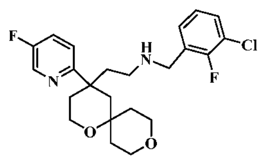

14

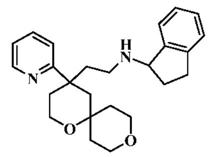

15

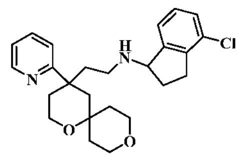

16

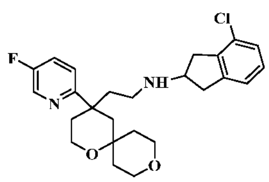

17

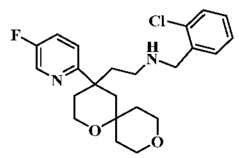

18

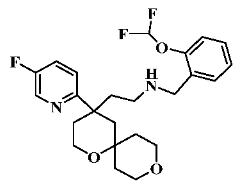

19

;

preferably, the compound is of any one of the following structures:

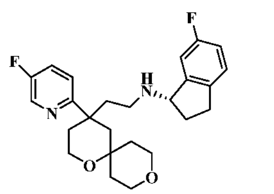

1-1

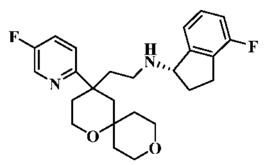

2-1

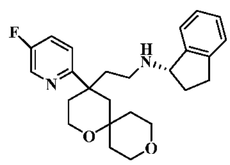

3-1

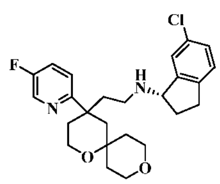

4-1

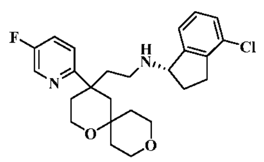

5-1

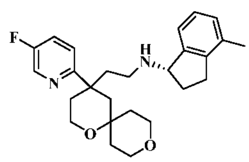

6-1

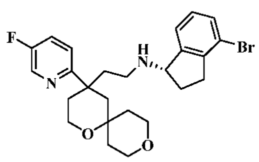

7-1

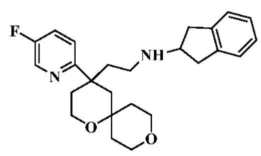

8

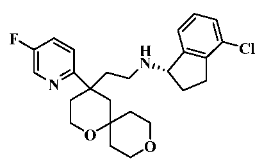

5-1

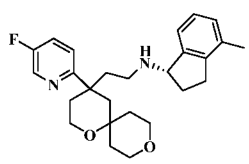

6-1

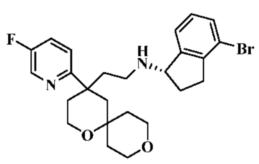

7-1

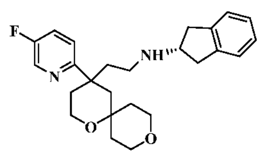

8-1

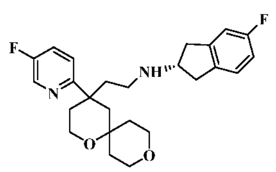

9-1

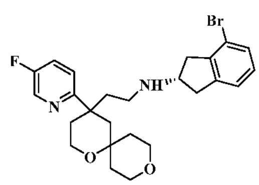

10-1

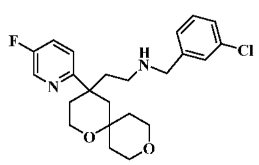

11

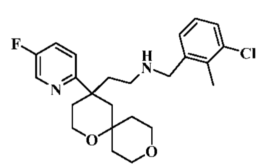

12

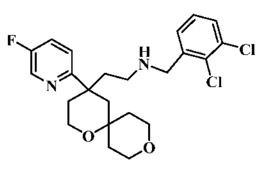
13

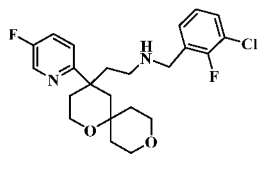
14

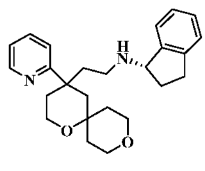
15-1

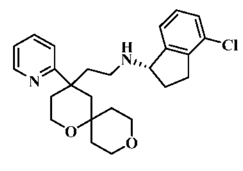
16-1

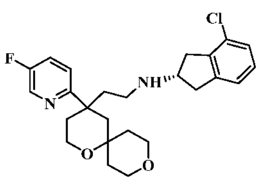
17-1

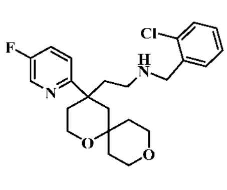
18

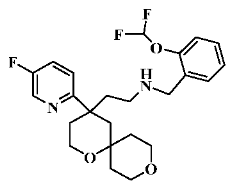
19

;

preferably, the compound is of any one of the following structures:

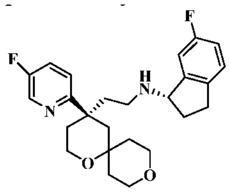
1-1-1

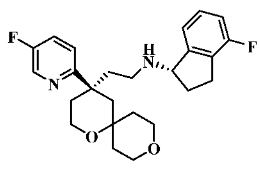
2-1-1

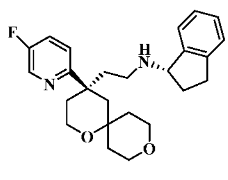
3-1-1

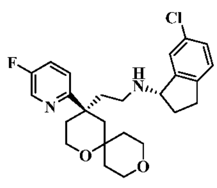
4-1-1

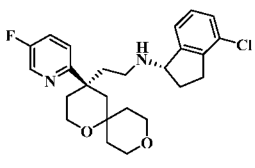
5-1-1

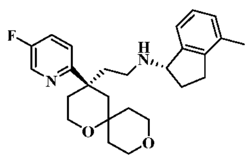
6-1-1

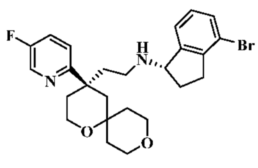
7-1-1

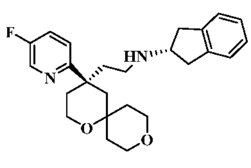
8-1-1

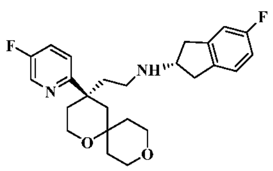
9-1-1

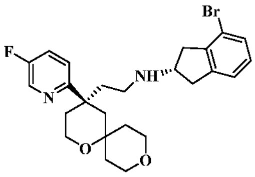
10-1-1

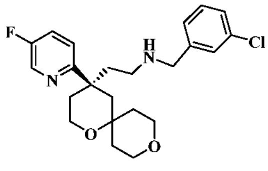
11-1

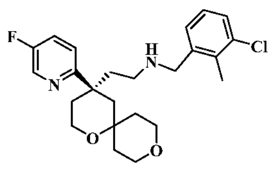
12-1

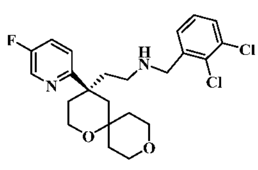
13-1

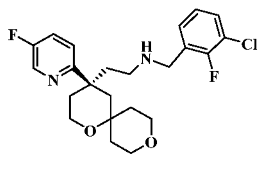
14-1

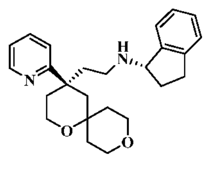
15-1-1

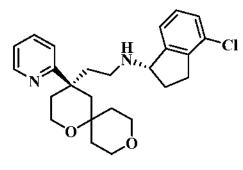
16-1-1

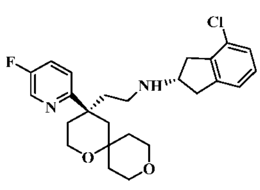
17-1-1

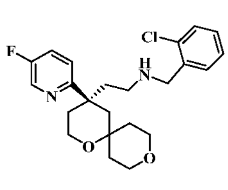
18-1

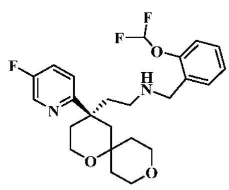
19-1

.

A preferred embodiment of the present invention aims to provide a method for preparing the compound of general formula (I), the stereoisomer thereof, the tautomer thereof, or the pharmaceutically acceptable salt thereof described above, comprising:

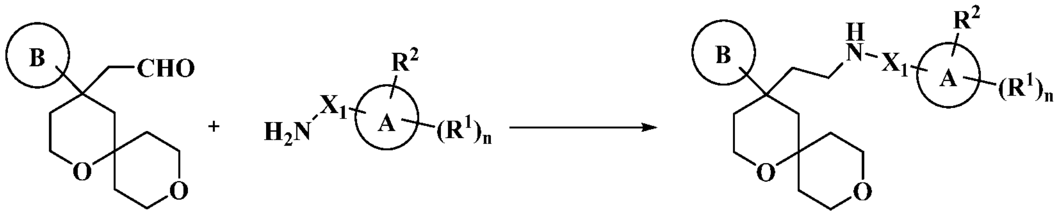

(I-A) (I-B) (I)

conducting a reductive amination reaction of a compound of general formula (I-A) with a compound of general formula (I-B) or a pharmaceutically acceptable salt thereof to give the compound of general formula (I), the stereoisomer thereof, the tautomer thereof, or the pharmaceutically acceptable salt thereof;
wherein ring A, $R^1$, $R^2$, ring B, $X_1$, and n are as described in general formula (I).

A preferred embodiment of the present invention aims to provide a method for preparing the compound of general formula (II), the stereoisomer thereof, the tautomer thereof, or the pharmaceutically acceptable salt thereof described above, comprising:

Method I:

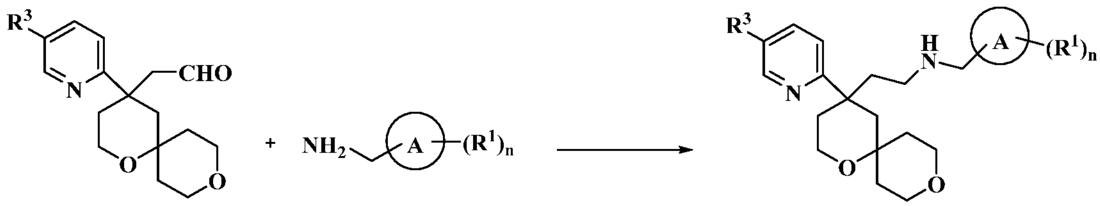

(II-A-1) (II-B-1) (II)

conducting a reductive amination reaction of a compound of general formula (II-A-1) with a compound of general formula (II-B-1) or a pharmaceutically acceptable salt thereof to give the compound of general formula (II), the stereoisomer thereof, the tautomer thereof, or the pharmaceutically acceptable salt thereof;
Method II:

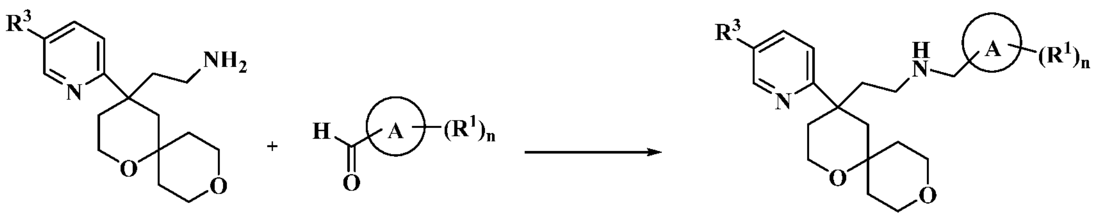

(II-A-2) (II-B-2) (II)

conducting a reductive amination reaction of a compound of general formula (II-A-2) with a compound of general formula (II-B-2) or a pharmaceutically acceptable salt thereof to give the compound of general formula (II), the stereoisomer thereof, the tautomer thereof, or the pharmaceutically acceptable salt thereof;
wherein ring A, $R^1$, $R^3$, and n are as described in general formula (II).

A preferred embodiment of the present invention aims to provide a method for preparing the compound of general formula (III), the stereoisomer thereof, the tautomer thereof, or the pharmaceutically acceptable salt thereof described above, comprising:

Method I:

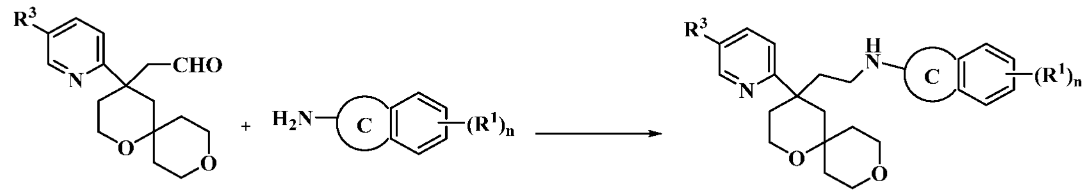

(III-A-1) (III-B-1) (III)

conducting a reductive amination reaction of a compound of general formula (III-A-1) with a compound of general formula (III-B-1) or a pharmaceutically acceptable salt thereof to give the compound of general formula (III), the stereoisomer thereof, the tautomer thereof, or the pharmaceutically acceptable salt thereof;

Method II:

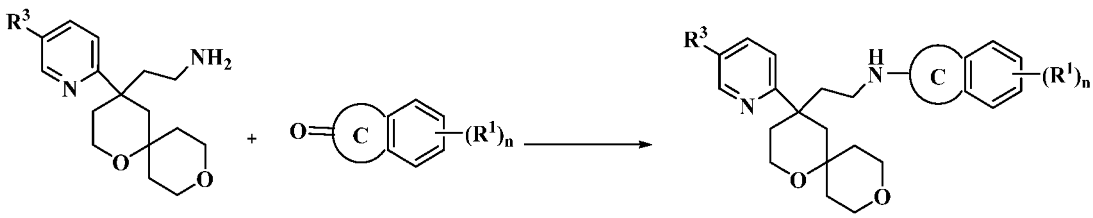

(III-A-2) (III-B-2) (III)

conducting a reductive amination reaction of a compound of general formula (III-A-2) with a compound of general formula (III-B-2) or a pharmaceutically acceptable salt thereof to give the compound of general formula (III), the stereoisomer thereof, the tautomer thereof, or the pharmaceutically acceptable salt thereof;

wherein ring C, $R^1$, $R^3$, and n are as described in general formula (III).

A preferred embodiment of the present invention aims to provide a method for preparing the compound of general formula (IV), the stereoisomer thereof, the tautomer thereof, or the pharmaceutically acceptable salt thereof described above, comprising:

Method I:

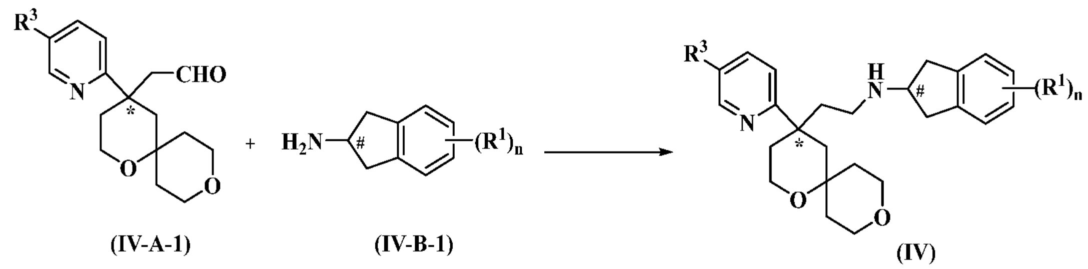

(IV-A-1) (IV-B-1) (IV)

conducting a reductive amination reaction of a compound of general formula (IV-A-1) with a compound of general formula (IV-B-1) or a pharmaceutically acceptable salt thereof to give the compound of general formula (IV), the stereoisomer thereof, the tautomer thereof, or the pharmaceutically acceptable salt thereof;

Method II:

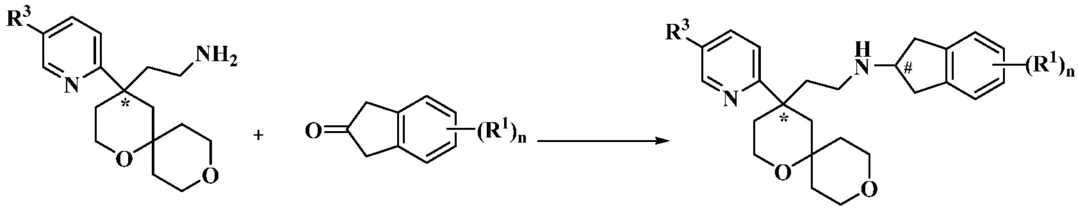

(IV-A-2) (IV-B-2) (IV)

conducting a reductive amination reaction of a compound of general formula (IV-A-2) with a compound of general formula (IV-B-2) or a pharmaceutically acceptable salt thereof to give the compound of general formula (IV), the stereoisomer thereof, the tautomer thereof, or the pharmaceutically acceptable salt thereof;
wherein $R^1$, $R^3$, and n are as described in general formula (IV).

A preferred embodiment of the present invention aims to provide a method for preparing the compound of general formula (V), the stereoisomer thereof, the tautomer thereof, or the pharmaceutically acceptable salt thereof described above, comprising:

Method I:

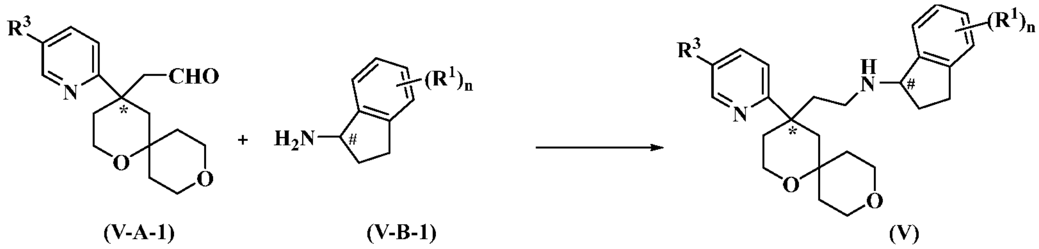

(V-A-1) (V-B-1) (V)

conducting a reductive amination reaction of a compound of general formula (V-A-1) with a compound of general formula (V-B-1) or a pharmaceutically acceptable salt thereof to give the compound of general formula (V), the stereoisomer thereof, the tautomer thereof, or the pharmaceutically acceptable salt thereof;
Method II:

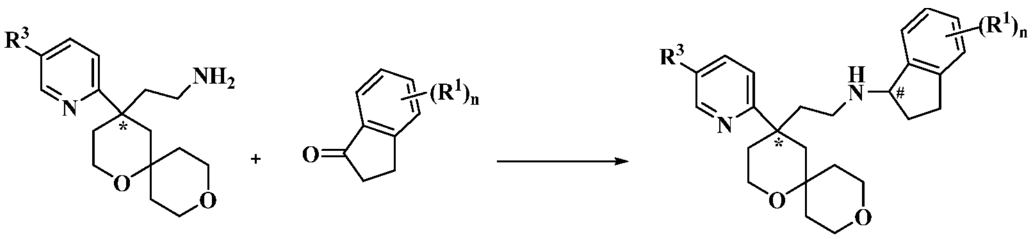

(V-A-2) (V-B-2) (V)

conducting a reductive amination reaction of a compound of general formula (V-A-2) with a compound of general formula (V-B-2) or a pharmaceutically acceptable salt thereof to give the compound of general formula (V), the stereoisomer thereof, the tautomer thereof, or the pharmaceutically acceptable salt thereof;
wherein $R^1$, $R^3$, and n are as described in general formula (V).

The present invention further provides a preferred embodiment, which relates to a pharmaceutical composition comprising a therapeutically effective amount of the compound of each of the general formulas described above, the stereoisomer thereof, the tautomer thereof, or the pharmaceutically acceptable salt thereof, and at least one pharmaceutical adjuvant of a pharmaceutically acceptable carrier, diluent, or excipient.

In some preferred embodiments of the present invention, the pharmaceutical composition may be administered in any of the following ways: oral administration, spray, inhalation, rectal administration, nasal administration, buccal administration, topical administration, parenteral administration such as subcutaneous, intravenous, intramuscular, intraperitoneal, intrathecal, intraventricular, intrasternal, or intracranial injection or infusion, or administration by means of an implantable reservoir, preferably oral, intraperitoneal, or intravenous administration.

When being administered orally, the compound of the present application may be prepared into any orally acceptable formulation forms, including, but not limited to, tablets, capsules, aqueous solutions, aqueous suspensions, etc. Carriers for the tablets generally include lactose and corn starch, and additionally, a lubricant such as magnesium stearate may be added. Diluents used in capsule formulations generally include lactose and dried corn starch. Aqueous suspension formulations are generally used by mixing the active ingredient with suitable emulsifying and suspending agents. If needed, sweetening, flavoring, or coloring agents may be added to the above oral formulation forms.

The tablets, for example, include but not limited to, buccal tablets, sublingual tablets, buccal patches, chewable tablets, dispersible tablets, effervescent tablets, immediate-release or sustained-release or controlled-release tablets, enteric-coated tablets, and the like.

When being administered topically, particularly for treating affected surfaces or organs easily reached by topical external application, such as eyes, skin or lower intestinal neurological diseases, the compound of the present application may be prepared into different topical formulation forms according to different affected surfaces or organs. The specific description is as follows.

When being administered topically to the eye, the compound of the present application may be formulated into a micronized suspension or solution, the carrier used is isotonic sterile saline with a certain pH, with or without the addition of preservatives such as chlorinated benzyl alkanolate. For ophthalmic use, the compound may also be formulated into ointments, such as vaseline.

When being administered topically to the skin, the compound of the present application may be formulated into a suitable ointment, lotion or cream formulation in which the active ingredient is suspended or dissolved in one or more carriers. Carriers that can be used in the ointment formulations include, but are not limited to: mineral oil, liquid vaseline, white vaseline, propylene glycol, polyethylene oxide, polypropylene oxide, emulsifying wax, and water; carriers that can be used in the lotions or creams include, but are not limited to: mineral oil, sorbitan monostearate, Tween 60, cetyl ester wax, hexadecene aryl alcohol, 2-octyldodecanol, benzyl alcohol, and water.

The present invention further provides a preferred embodiment, which relates to use of the compounds of each of the general formulas, the stereoisomers thereof, the tautomers thereof, or the pharmaceutically acceptable salts thereof, or the pharmaceutical composition in preparing a medicament for preventing and/or treating a related disease mediated by a μ-opioid receptor agonist.

The related disease mediated by a μ-opioid receptor agonist described in the above use is selected from one or more of pain, immune dysfunction, inflammation, esophageal reflux, neurological and psychiatric diseases, urological and reproductive diseases, cardiovascular diseases, and respiratory diseases, preferably pain.

The present invention further provides a preferred embodiment, which relates to use of the compounds of each of the general formulas, the stereoisomers thereof, the tautomers thereof, or the pharmaceutically acceptable salts thereof, or the pharmaceutical composition in preparing a medicament for preventing and/or treating pain or a pain-related disease.

The present invention further relates to a method for treating a related disease mediated by a μ-opioid receptor agonist, comprising administering to a mammal a therapeutically effective amount of the compound of the present invention, the stereoisomer thereof, the tautomer thereof, or the pharmaceutically acceptable salt thereof.

In some embodiments of the present invention, the present invention relates to treatment methods for pain, for example.

The related disease mediated by a μ-opioid receptor agonist described in the above method is selected from one or more of pain, immune dysfunction, inflammation, esophageal reflux, neurological and psychiatric diseases, urological and reproductive diseases, cardiovascular diseases, and respiratory diseases, preferably pain.

In some embodiments of the present invention, the pain is selected from one or more of postsurgical pain, cancer-induced pain, neuropathic pain, traumatic pain, and inflammation-induced pain.

In some embodiments of the present invention, the cancer is selected from one or more of breast cancer, endometrial cancer, cervical cancer, skin cancer, prostate cancer, ovarian cancer, fallopian tube tumor, hemophilia, and leukemia.

The treatment methods provided herein comprise administering to a subject a therapeutically effective amount of the compound of the present invention. In one embodiment, the present invention further provides a method for treating a related disease mediated by a μ-opioid receptor agonist in a mammal. The method comprises administering to the mammal a therapeutically effective amount of the compound of the present invention, the stereoisomer thereof, the tautomer thereof, or the pharmaceutically acceptable salt thereof.

## Detailed Description of the Invention

Unless otherwise stated, the terms used in the specification and claims have the following meanings.

The compounds of the present invention may exist in a form of a specific geometric isomer or stereoisomer. All such compounds are contemplated herein, including *cis* and *trans* isomers, (-)-and (+)-enantiomers, (R)- and (S)-enantiomers, diastereoisomers, (D)-isomers, (L)-isomers, and racemic mixtures and other mixtures thereof, such as an enantiomer or diastereomer enriched mixture, all of which are encompassed within the scope of the present invention. Additional asymmetric carbon atoms may be present in substituents such as an alkyl group. All these isomers and mixtures thereof are encompassed within the scope of the present invention. In certain embodiments, preferred compounds are those isomer compounds that exhibit superior biological activity. Purified or partially purified isomers and stereoisomers, or racemic or diastereomeric mixtures of the compound of the present invention are also encompassed within the scope of the present invention. Purification and isolation of such materials can be accomplished by standard techniques known in the art.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereomer" refers to stereoisomers whose molecules have two or more chiral centers and are not mirror images of each other.

Unless otherwise stated, "(D)" or "(+)" stands for dextrorotation, "(L)" or "(-)" stands for levorotation, and "(DL)" or "(±)" stands for racemization.

Unless otherwise stated, the absolute configuration of a stereogenic center is represented by a wedged solid bond (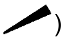) and a wedged dashed bond (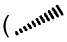), and the relative configuration of a stereogenic center is represented by a straight solid bond (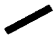) and a straight dashed bond (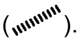). A wavy line (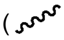) represents a wedged solid bond (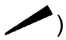) or a wedged dashed bond (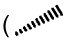), or a wavy line (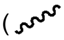) represents a straight solid bond (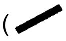) and a straight dashed bond (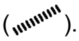).

The compound of the present invention may be present in a particular form. Unless otherwise stated, the term "tautomer" or "tautomeric form" means that different functional isomers are in dynamic equilibrium at room temperature and can be rapidly converted into each other. If tautomers are possible (e.g., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence tautomer includes the interconversion by recombination of some bonding electrons. A specific example of the keto-enol tautomerization is the interconversion between tautomers pentane-2,4-dione and 4-hydroxypent-3-en-2-one.

The term "alkyl" refers to a saturated aliphatic hydrocarbyl group which is a linear or branched chain group containing 1 to 20 carbon atoms, preferably an alkyl group containing 1 to 8 carbon atoms, more preferably an alkyl group of 1 to 6 carbon atoms, most preferably an alkyl group of 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, and 2,2-diethylhexyl and various branched isomers thereof, and the like. More preferred is an alkyl group having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl may be substituted or unsubstituted, and when it is substituted, the substituent may be substituted at any available point of attachment, and the substituent is preferably one or more of the following groups independently being alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, oxo, carboxyl and carboxylate group, wherein methyl, ethyl, isopropyl, tert-butyl, haloalkyl, alkoxy-substituted alkyl, and hydroxy-substituted alkyl are preferable in the present invention. When the alkyl is substituted with a substituent, the substituent is not further substituted.

The term "alkylene" means that one hydrogen atom of alkyl is further substituted. For example, "methylene" refers to $-CH_2-$, "ethylene" refers to $-(CH_2)_2-$, "propylene" refers to $-(CH_2)_3-$, "butylene" refers to $-(CH_2)_4-$, etc.

The term "alkenyl" refers to alkyl as defined above consisting of at least two carbon atoms and at least one carbon-carbon double bond, for example, vinyl, 1-propenyl, 2-propenyl, 1-, 2- or 3-butenyl, and the like. The alkenyl may be substituted or unsubstituted, and when it is substituted, the substituent is preferably one or more of the following groups independently being alkyl, alkenyl, alkynyl, alkoxy, alkylthio, alkylamino, halogen, mercapto, hydroxy, nitro, amino,

cycloalkyl, heterocycloalkyl, aryl, heteroaryl, cycloalkoxy, heterocycloalkoxy, cycloalkylthio and heterocycloalkylthio. When the alkylene is substituted with a substituent, the substituent is not further substituted.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl groups include spiro, fused and bridged cycloalkyl groups, preferably cyclopropyl, cyclobutyl, cyclohexyl, cyclopentyl, and cycloheptyl.

The term "spirocycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and is more preferably 7- to 10-membered. According to the number of spiro atoms shared among the rings, spirocycloalkyl may be monospirocycloalkyl, bispirocycloalkyl or polyspirocycloalkyl, preferably monospirocycloalkyl and bispirocycloalkyl. 4-membered/4-membered, 4-membered/5-membered, 4-membered/6-membered, 5-membered/5-membered or 5-membered/6-membered monospirocycloalkyl is more preferred.

Non-limiting examples of spirocycloalkyl include:

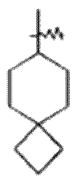 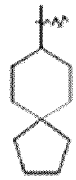 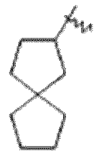 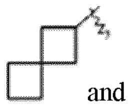 and 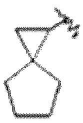 ;

Spirocycloalkyl in which monospiro cycloalkyl shares a spiro atom with heterocycloalkyl is also included, and non-limiting examples include:

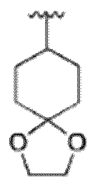 and 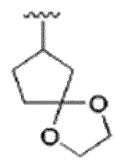 .

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of the rings has a fully conjugated π-electron system. It is preferably 6- to 14-membered, and is more preferably 7- to 10-membered. According to the number of constituent rings, fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicycloalkyl. Non-limiting examples of fused cycloalkyl include:

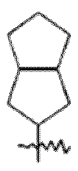 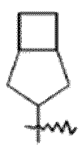 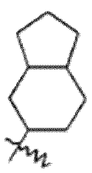 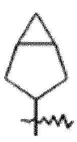 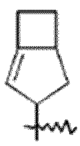 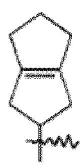 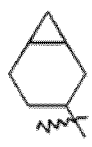 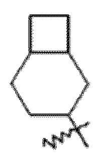 and 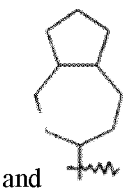 .

The term "bridged cycloalkyl" refers to a 5- to 20-membered all-carbon polycyclic group in which any two rings share two carbon atoms that are not directly connected to each other. It may contain one or more double bonds, but none of the rings has a fully conjugated π-electron system. It is preferably 6- to 14-membered, and is more preferably 7- to 10-membered. According to the number of constituent rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

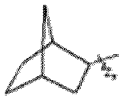 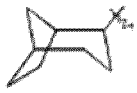 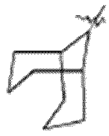 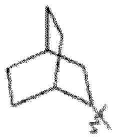 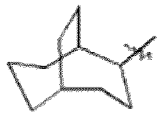

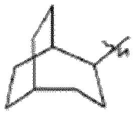 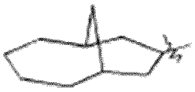 and 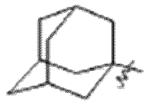.

The cycloalkyl ring may be fused to an aryl, heteroaryl or heterocycloalkyl ring, wherein the ring attached to the parent structure is cycloalkyl; non-limiting examples include indanyl, tetrahydronaphthyl, benzocycloheptyl, and the like. Cycloalkyl may be optionally substituted or unsubstituted, and when it is substituted, the substituent is preferably one or more of the following groups independently being alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, oxo, carboxyl and carboxylate group. When the cycloalkyl is substituted with a substituent, the substituent is not further substituted.

The term "aryl" refers to a 6- to 14-membered, preferably 6- to 10-membered all-carbon monocyclic or fused polycyclic (i.e., rings that share a pair of adjacent carbon atoms) group having a conjugated electron system, such as phenyl and naphthyl, more preferably, phenyl. The aryl ring may be fused to a heteroaryl, heterocyclyl or cycloalkyl ring and includes benzo 3- to 8-membered cycloalkyl and benzo 3- to 8-membered heterocyclyl, preferably benzo 3- to 6-membered cycloalkyl and benzo 3- to 6-membered heterocyclyl, wherein the heterocyclyl is heterocyclyl containing 1-3 nitrogen atoms, oxygen atoms and sulfur atoms, or further includes a three-membered nitrogen-containing fused ring containing a benzene ring.

The ring attached to the parent structure is the aryl ring; its non-limiting examples include:

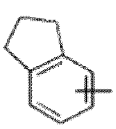 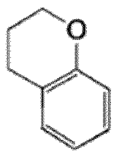 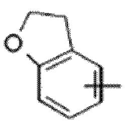 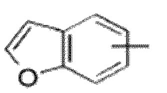 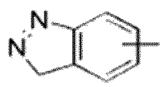 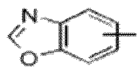 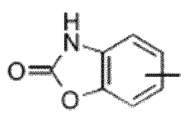

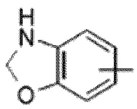 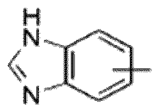 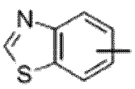 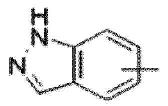 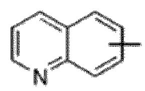 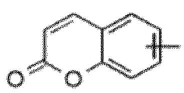

and

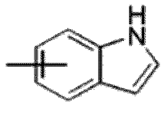 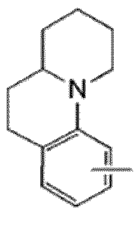.

Aryl may be substituted or unsubstituted, and when it is substituted, the substituent is preferably one or more of the following groups independently being alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, carboxyl or carboxylate group. When the aryl is substituted with a substituent, the substituent is not further substituted.

The term "heteroaryl" refers to a heteroaromatic system containing 1 to 4 heteroatoms and 5 to 14 ring atoms, wherein the heteroatoms are selected from one or more of oxygen, sulfur, and nitrogen. The heteroaryl is preferably 5-

to 10-membered, more preferably 5- or 6-membered, such as imidazolyl, furyl, thienyl, thiazolyl, pyrazolyl, oxazolyl, pyrrolyl, triazolyl, tetrazolyl, pyridinyl, pyrimidinyl, thiadiazole, pyrazinyl, and the like, preferably triazolyl, thienyl, imidazolyl, pyrazolyl or pyrimidinyl, and thiazolyl, more preferably triazolyl, pyrrolyl, thienyl, thiazolyl, and pyrimidinyl. The heteroaryl ring may be fused to an aryl, heterocyclyl or cycloalkyl ring, wherein the ring attached to the parent structure is the heteroaryl ring; its non-limiting examples include:

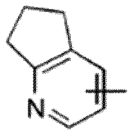 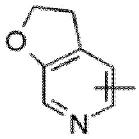 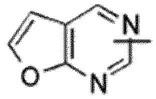 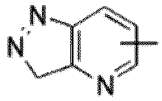 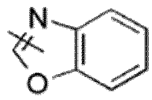 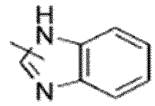

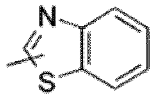 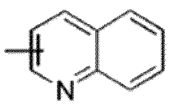 and 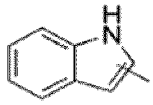 .

The heteroaryl may be optionally substituted or unsubstituted, and when it is substituted, the substituent is preferably one or more of the following groups independently being alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, carboxyl, or carboxylate group. When the heteroaryl is substituted with a substituent, the substituent is not further substituted.

The term "alkoxy" refers to -O-(alkyl) and -O-(unsubstituted cycloalkyl), wherein the alkyl is as defined above, preferably alkoxy containing 1 to 8 carbon atoms, more preferably alkoxy of 1 to 6 carbon atoms, most preferably alkoxy of 1 to 3 carbon atoms. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently being alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, carboxyl or carboxylate group. When the alkoxy is substituted with a substituent, the substituent is not further substituted.

"Haloalkyl" refers to alkyl substituted with one or more halogens, wherein the alkyl is as defined above. Non-limiting examples of halomethyl include: fluoromethyl, chloromethyl, bromomethyl, iodomethyl, difluoromethyl, chlorofluoromethyl, dichloromethyl, bromofluoromethyl, trifluoromethyl, chlorodifluoromethyl, dichlorofluoromethyl, trichloromethyl, bromodifluoromethyl, bromochlorofluoromethyl, dibromofluoromethyl, and the like, preferably fluoromethyl, difluoromethyl, and trifluoromethyl. Non-limiting examples of haloethyl include: 2-fluoroethyl, 2-chloroethyl, 2-bromoethyl, 2,2-difluoroethyl, 2-chloro-2-fluoroethyl, 2,2-dichloroethyl, 2-bromo-2-fluoroethyl, 2,2,2-trifluoroethyl, 2-chloro-2,2-difluoroethyl, 2,2-dichloro-2-fluoroethyl, 2,2,2-trichloroethyl, 2-bromo-2,2-difluoroethyl, 2-bromo-2-chloro-2-fluoroethyl, 2-bromo-2,2-dichloroethyl, 1,1,2,2-tetrafluoroethyl, pentafluoroethyl, 1-chloro-1,2,2,2-tetrafluoroethyl, 2-chloro-1,1,2,2-tetrafluoroethyl, 1,2-dichloro-1,2,2-trifluoroethyl, 2-bromo-1,1,2,2-tetrafluoroethyl, and the like, preferably 2-fluoroethyl, 2-chloroethyl, 2-bromoethyl, and 2,2-difluoroethyl.

"Haloalkoxy" refers to alkoxy substituted with one or more halogens, wherein the alkoxy is as defined above. Non-limiting examples of halomethoxy include: fluoromethoxy, chloromethoxy, bromomethoxy, iodomethoxy, difluoromethoxy, chlorofluoromethoxy, dichloromethoxy, bromofluoromethoxy, trifluoromethoxy, chlorodifluoromethoxy, dichlorofluoromethoxy, trichloromethoxy, bromodifluoromethoxy, bromochlorofluoromethoxy, dibromofluoromethoxy, and the like, preferably fluoromethoxy, difluoromethoxy, and trifluoromethoxy. Non-limiting examples of haloethoxy include: 2-fluoroethoxy, 2-chloroethoxy, 2-bromoethoxy, 2,2-difluoroethoxy, 2-chloro-2-fluoroethoxy, 2, 2-dichloroethoxy, 2-bromo-2-fluoroethoxy, 2,2,2-trifluoroethoxy, 2-chloro-2,2-difluoroethoxy, 2,2-dichloro-2-fluoroethoxy, 2,2,2-trichloroethoxy, 2-bromo-2,2-difluoroethoxy, 2-bromo-2-chloro-2-fluoroethoxy, 2-bromo-2,2-dichloroethoxy, 1,1,2,2-tetrafluoroethoxy, pentafluoroethoxy, 1-chloro-1,2,2,2-tetrafluoroethoxy, 2-chloro-1,1,2,2-tetrafluoroethoxy, 1,2-dichloro-1,2,2-trifluoroethoxy, 2-bromo-1,1,2,2-tetrafluoroethoxy, and the like, preferably 2-fluoroethoxy, 2-chloroethoxy, 2-bromoethoxy, and 2,2-difluoroethoxy.

"Alkenyl" refers to an alkenyl group, also referred to as an alkene group, wherein the alkenyl may be further substituted with other related groups, such as alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, carboxyl, or carboxylate group.

"Alkynyl" refers to (CH=C-), wherein the alkynyl may be further substituted by other related groups, such as alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, carboxyl, or carboxylate group. "Hydroxy" refers to -OH group.

"Halogen" refers to fluorine, chlorine, bromine or iodine.

"Amino" refers to $-NH_2$.

"Cyano" refers to -CN.

"Nitro" refers to $-NO_2$.

"Carboxyl" refers to -C(O)OH.

The terms "comprise", "include", "have", "contain", or "relate" and other variations thereof herein are inclusive or open-ended and do not exclude additional unrecited elements or method steps. It should be understood by those skilled in the art that terms described above such as "comprise" encompass the meaning of "consist of'.

The term "one or more" or similar expressions "at least one" may indicate, for example, 1, 2, 3, 4, 5, 6, 7, 8, 9, 10 or more.

When the lower and upper limits of a range of values are disclosed, any value falling within the range and any included range are specifically disclosed. In particular, each range of values disclosed herein is to be understood as indicating each value and range encompassed within the broader range.

As used herein, "Z" and "-Z-" both refer to the same particular group, which may be used interchangeably.

The expression "m-n" as used herein refers to the range of m to n as well as to sub-ranges consisting of point values therein and the point values. For example, the expression "C2-C8" or "C2-8" encompasses a range of 2-8 carbon atoms and should be understood as also encompassing any sub-range and each point value therein, e.g., C2-C5, C3-C4, C2-C6, C3-C6, C4-C6, C4-C7, C4-C8, C2-C5, etc., and C2, C3, C4, C5, C6, C7, C8, etc. For example, the expression "C3-C10" or "C3-10" should also be understood in a similar manner, for example, it can encompass any sub-range and point value therein, e.g., C3-C9, C6-C9, C6-C8, C6-C7, C7-C10, C7-C9, C7-C8, C8-C9, etc., and C3, C4, C5, C6, C7, C8, C9, C10, etc. For another example, the expression "C1-C6" or "C1-6" encompasses a range of 1-6 carbon atoms and should be understood as also encompassing any sub-range and each point value therein, e.g., C2-C5, C3-C4, C1-C2, C1-C3, C1-C4, C1-C5, C1-C6, etc., and C1, C2, C3, C4, C5, C6, etc. For another example, the expression "three to ten membered" should be understood as encompassing any sub-range and each point value therein, e.g., three to five membered, three to six membered, three to seven membered, three to eight membered, four to five membered, four to six membered, four to seven membered, four to eight membered, five to seven membered, five to eight membered, six to seven membered, six to eight membered, nine to ten membered, etc., and three membered, four membered, five membered, six membered, seven membered, eight membered, nine membered, ten membered, etc. Other similar expressions herein should also be understood in a similar manner.

The expressions "X is selected from A, B, or C", "X is selected from A, B, and C", "X is A, B, or C", "X is A, B, and C", and the like as used herein all carry the same meaning, i.e., X may be any one or more of A, B, and C.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily exist and that the description includes instances where the cycloalkyl is or is not substituted with alkyl.

The terms "substitution" and "substituted" mean that one or more (e.g., 1, 2, 3, or 4) hydrogens on a specified atom is replaced with a selection from the designated group, with the proviso that the normal valency of the atom specified is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed.

If a substituent is described as "optionally substituted", the substituent may be unsubstituted or may be substituted. If an atom or group is described as optionally substituted with one or more substituents in the list of substituents, one or more hydrogens on the atom or group can be replaced with an independently selected and optional substituent. When the substituent is oxo (namely =O), it means that two hydrogen atoms are replaced. Unless otherwise indicated, as used herein, the connecting point of a substituent may be from any suitable position of the substituent. When a bond of a substituent is shown to pass through a bond connecting two atoms in the ring, the substituent may be bonded to any one of the ring-forming atoms in the substitutable ring. When any variable (e.g., R), as well as labeled variables (e.g., R1, R2, R3, R4, R5, R6, R7, etc.) occurs more than once in a composition or structure of a compound, the variable is independently defined in each case at each occurrence. For example, if a group is substituted with 0, 1, 2, 3 or 4 R substituents, the group can be optionally substituted with up to four R substituents, and each R substituent is independently defined in each case.

The term "pharmaceutically acceptable" substance refers to those substances which are, within the scope of sound medical judgment, suitable for use in contact with the tissues of patients without undue toxicity, irritation, allergic

response and other problems, commensurate with a reasonable benefit to risk ratio, and effective for their intended use.

The term "pharmaceutically acceptable salt" refers to salts of the compounds of the present invention, which are safe and effective for use in the body of a mammal and possess the requisite biological activities.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers or excipients. The pharmaceutical composition is intended to promote the administration to an organism, so as to facilitate the absorption of the active ingredient, thereby exerting biological activity.

The term "pharmaceutically acceptable carrier" refers to those substances which do not have a significant irritating effect on organisms and do not impair the biological activity and properties of the active compound. The "pharmaceutically acceptable carrier" includes, but is not limited to a glidant, a sweetener, a diluent, a preservative, a dye/colorant, a flavoring agent, a surfactant, a wetting agent, a dispersant, a disintegrant, a stabilizer, a solvent or an emulsifier.

The terms "administration" or "administering" and the like refer to those methods which enable a compound or composition to be delivered to a desired site of biological action. Those methods include, but are not limited to, oral administration or parenteral (including intraventricular, intravenous, subcutaneous, intraperitoneal, intramuscular, intravascular injection or infusion), topical, rectal administrations, and the like. Those methods especially include injection or oral administration.

As used herein, the term "treat", "treating" or "treatment" includes alleviating, reducing, or ameliorating a disease or symptom; preventing other symptoms; ameliorating or preventing metabolic factors underlying a symptom; inhibiting a disease or symptom, e.g., arresting the development of a disease or symptom; alleviating a disease or symptom; promoting the alleviation of a disease or symptom; or halting the signs of a disease or symptom, and extends to include prevention. The "treat", "treating" or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, and amelioration of the underlying disease in the subject is observed, although the subject may still be afflicted with the underlying disease. The prophylactic benefit refers to the use of a composition by a patient to prevent the risk of a disease or the administration of a composition by a patient when the patient develops one or more physiological conditions of a disease, although the disease has not yet been diagnosed.

The term "active ingredient", "therapeutic agent", "active substance" or "active agent" refers to a chemical entity that is effective in treating a target disorder, disease, or condition. The term "nervous and mental disease" refers to a general term for neurological diseases and psychiatric diseases, including neurological diseases and/or psychiatric diseases.

For a drug, drug unit or active ingredient, the term "effective amount", "therapeutically effective amount" or "prophylactically effective amount" refers to an amount of a drug or agent that is sufficient to provide the desired effect with acceptable side effects. The determination of the effective amount varies from person to person. It depends on the age and general condition of a subject, as well as the particular active substance used. The appropriate effective amount in a case may be determined by those skilled in the art in the light of routine tests.

As used herein, an "individual" includes a human or non-human animal. An exemplary human individual includes a human individual (referred to as patients) with a disease (e.g., a disease described herein) or a normal individual. As used herein, a "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The following detailed description is intended to illustrate non-limiting embodiments and to enable others skilled in the art to more fully understand the technical scheme of the present invention, its principles, and its practical application, so that others skilled in the art may modify and implement the present invention in various forms to allow it to be optimally adapted to the particular use contemplated.

Beneficial Effects

*In vitro* receptor functional assays show that the compounds of the present invention have an agonistic effect on MOR receptors and have an effect on preventing and/or treating a related disease mediated by a μ-opioid receptor agonist, such as pain, immune dysfunction, inflammation, esophageal reflux, neurological and psychiatric diseases, urological and reproductive diseases, cardiovascular diseases, and respiratory diseases. The compounds of the present invention have relatively high inhibitory activity against cAMP and a relatively high Emax value. In addition, the compounds of the present invention have a relatively low Emax value for β-arrestin, which indicates that the compounds are safer and more effective in the clinical use process, and compared with the oral drug oxycodone, the compounds have better analgesic effect and can greatly improve the compliance of patients to take medicine.

## BRIEF DESCRIPTION OF THE DRAWING

FIG. 1 is a graph showing the percentage of the analgesic effect in the tail flick test of mice in Test Example 3.

## DETAILED DESCRIPTION

Embodiments of the present invention will be described in detail below with reference to examples, but those skilled in the art will appreciate that the following examples are only illustrative of the present invention and should not be construed as limiting the scope of the present invention. Experimental procedures without specified conditions in the examples are conducted according to conventional conditions or conditions recommended by the manufacturer. Reagents or instruments without specified manufacturers used herein are conventional products that are commercially available. The proportions or percentages used herein are calculated by weight, unless otherwise specified.

## EXAMPLES

The compound structure of the present invention is determined by nuclear magnetic resonance (NMR) and/or liquid chromatography-mass spectrometry (LC-MS).

The NMR chemical shift (δ) is given in parts per million (ppm). The NMR determination was conducted by using an AVANCE III600 nuclear magnetic resonance apparatus, with deuterated dimethyl sulfoxide (DMSO-$d_6$), deuterated methanol ($CD_3OD$), and deuterated chloroform ($CDCl_3$) as determination solvents, and tetramethylsilane (TMS) as an internal standard.

The LC-MS determination was conducted by using a Japan Shimadzu LCMS2020 mass spectrometer. HPLC analysis was performed by using a Japan Shimadzu LC20A liquid chromatograph.

The Yantai Jiangyou silica gel plate was adopted as a thin layer chromatography silica gel plate. The specification adopted by the TLC was 0.2 mm ± 0.03 mm, and the specification adopted by the thin layer chromatography for product separation and purification was 0.4 mm-0.5 mm.

### Intermediate 1

### 2-(4-(5-fluoropyridin-2-yl)-1,9-dioxaspiro[5.5]undecan-4-yl)ethyl-1-amine 1a

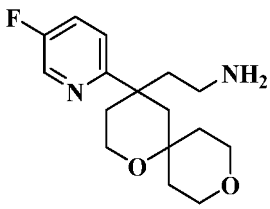

1a

Synthesis scheme:

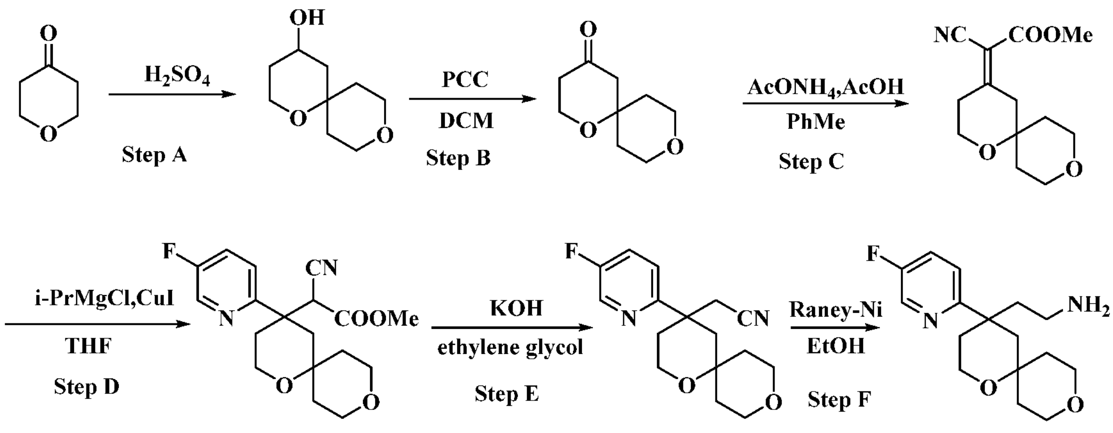


1a

**Step A: preparation of 1,9-dioxaspiro[5.5]undecan-4-ol**

3-Buten-1-ol (10.0 g, 142 mmol) and tetrahydropyranone (7.1 g, 71 mmol) were added to a flask, the mixture was cooled to 0 °C, 75% sulfuric acid (40 mL) was slowly added dropwise to the reaction solution, and the mixture was gradually warmed to room temperature and reacted overnight. Water (100 mL) was added to the reaction system, the pH was adjusted to 8 with a 10% sodium hydroxide solution, and the mixture was extracted with ethyl acetate (150 mL × 3). The ethyl acetate layer was washed with water (50 mL × 3) and saturated brine (50 mL), dried over anhydrous sodium sulfate, concentrated, and purified by silica gel column chromatography to give the target product as a pale yellow oil (4.8 g, 40% yield).

$^1$H NMR (600 MHz, DMSO-$d_6$): δ4.62 (d, $J$ = 4.5 Hz, 1H), 3.78-3.73 (m, 1H), 3.67 (m, 1H), 3.62-3.58 (m, 1H), 3.55-3.47 (m, 4H), 1.85-1.81 (m, 1H), 1.78-1.69 (m, 2H), 1.63-1.52 (m, 1H), 1.48-1.44 (m, 2H), 1.30-1.21 (m, 1H), 1.10 (dd, J= 12.6, 10.5 Hz, 1H).

**Step B: preparation of 1,9-dioxaspiro[5.5]undecan-4-one**

1,9-Dioxaspiro[5.5]undecan-4-ol (4.8 g, 28 mmol) was dissolved in dichloromethane (50 mL) in a flask, the mixture was cooled to 0 °C, pyridinium chlorochromate (9.0 g, 42 mmol) was added slowly, and the mixture was warmed to room temperature and reacted overnight. The mixture was subjected to suction filtration and concentration, and purified by column chromatography (petroleum ether:ethyl acetate = 5:1) to give the target product as a pale yellow oil (4.0 g, 85% yield).

$^1$H NMR (600 MHz, DMSO-$d_6$): δ3.93 (t, $J$ = 6.1 Hz, 2H), 3.60-3.53 (m, 4H), 2.42-2.30 (m, 4H), 1.77 -1.61 (m, 2H), 1.60-1.48 (m, 2H).

**Step C: preparation of methyl (Z)-2-cyano-2-(1,9-dioxaspiro[5.5]undecan-4-ylidene)acetate**

1,9-Dioxaspiro[5.5]undecan-4-one (4.0 g, 23.5 mmol), ammonium acetate (530 mg,6.8 mmol), acetic acid (270 mg,4.6 mmol), and methyl cyanoacetate (2.6 g, 26.2 mmol) were dissolved in toluene (50 mL) in a flask, and the mixture was refluxed and reacted for 8 h. The organic phase was washed with water (50 mL × 3), washed with saturated brine (50 mL), dried over anhydrous sodium sulfate, subjected to suction filtration and concentration, and purified by column chromatography (petroleum ether:ethyl acetate = 5:1) to give the target product (5.6 g, 90% yield).

LC-MS: MS Found: 252 $[M+H]^+$.

$^1$H NMR (600 MHz, DMSO-$d_6$): δ4.02 (s, 1H), 3.86 (t, $J$ = 5.7 Hz, 1H), 3.78-3.76 (m, 3H), 3.57-3.54 (m, 4H), 3.08-3.06 (m, 2H), 2.75-2.63 (m, 2H), 1.68-1.63 (m, 2H), 1.61-1.47 (m, 2H).

**Step D: preparation of methyl 2-cyano-2-(4-(5-(fluoropyridin-2-yl)-1,9-dioxaspiro[5.5]undecan-4-yl)acetate**

5-Fluoro-2-bromopyridine (5.35 g, 40 mmol) was dissolved in anhydrous tetrahydrofuran (15 mL), and the mixture was slowly added dropwise to an isopropyl magnesium chloride solution (20 mL, 40 mmol) at 0 °C and reacted at room temperature for 3 h. Cuprous iodide (0.76 g, 4 mmol) was added, and the mixture was reacted for another 1 h. Methyl (Z)-2-cyano-2-(1,9-dioxaspiro[5.5]undecane-4-ylidene)acetate (5.0 g, 20 mmol) was dissolved in tetrahydrofuran (15 mL) and added dropwise therein, and the mixture was reacted overnight at room temperature. A saturated ammonium

chloride solution (20 mL) was added, and the mixture was extracted with ethyl acetate (50 mL × 3). The organic phase was washed with water (50 mL), washed with saturated brine (50 mL), dried over anhydrous sodium sulfate, subjected to suction filtration and concentration, and purified by column chromatography (petroleum ether:ethyl acetate = 3:1) to give the target product (3.3 g, 50% yield).

LC-MS: MS Found: 349 $[M+H]^+$.

$^1H$ NMR (600 MHz, $CDCl_3$) δ8.47 (dd, *J* = 7.0, 2.8 Hz, 1H), 7.64-7.27 (m, 2H), 3.91-3.63 (m, 8H), 3.46-3.33 (m, 2H), 2.94-2.58 (m, 2H), 2.15 (dd, *J* = 4.9, 1.8 Hz, 2H), 1.80-1.69 (m, 2H), 1.32-1.19 (m, 2H).

**Step E: preparation of 2-(4-(5-fluoropyridin-2-yl)-1,9-dioxaspiro[5.5]undecan-4-yl)acetonitrile**

Methyl 2-cyano-2-(4-(5-(fluoropyridin-2-yl)-1,9-dioxaspiro[5.5]undecan-4-yl)acetate (3.0 g, 86 mmol) was dissolved in ethylene glycol (10 mL). Potassium hydroxide (1 g, 17 mmol) was added, and the mixture was reacted at 110 °C for 3 h. Water (10 mL) was added to the reaction system, and the mixture was extracted with ethyl acetate (20 mL ×3). The ethyl acetate layer was washed with water (10 mL), washed with saturated brine (10 mL), dried over anhydrous sodium sulfate, concentrated, and purified by column chromatography (petroleum ether:ethyl acetate = 3:1) to give the pale yellow target product (1.7 g, 70% yield).

LC-MS: MS Found: 291 $[M+H]^+$.

$^1H$ NMR (600 MHz, $CDCl_3$): δ8.47 (d, *J* = 2.6 Hz, 1H), 7.50-7.34 (m, 2H), 4.25-3.81 (m, 2H), 3.78-3.65 (m, 4H), 3.41-3.27 (m, 2H), 2.70-2.59 (m, 2H), 1.95-1.83 (m, 2H), 1.70 (m, 2H), 0.97-0.94 (m, 2H).

**Step F: preparation of 2-(4-(5-fluoropyridin-2-yl)-1,9-dioxaspiro[5.5]undecan-4-yl)ethyl-1-amine**

2-(4-(5-Fluoropyridin-2-yl)-1,9-dioxaspiro[5.5]undecan-4-yl)acetonitrile (1.5 g, 5.2 mmol) was dissolved in ethanol (30 mL) and methanol (5 mL). After ammonia water (2 mL) was added dropwise, 5 spoons of Raney nickel were added, and the mixture was stirred at room temperature overnight under hydrogen atmosphere. After the reaction was completed, the mixture was filtered through celite, and the filtrate was concentrated to give a yellow oily product **1a** (1.3 g, 85% yield).

LC-MS(m/z): 295.1 $[M+H]^+$.

**Intermediate 2**

**2-(4-(5-fluoropyridin-2-yl)-1,9-dioxaspiro[5.5]undecan-4-yl)acetaldehyde 1b**

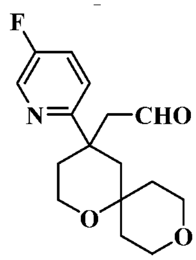

**1b**

Synthesis scheme:

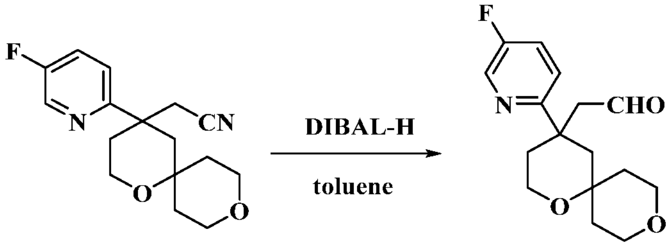


1b

2-(4-(5-Fluoropyridin-2-yl)-1,9-dioxaspiro[5.5]undecan-4-yl)acetonitrile (1.0 g, 3.8 mmol) was added to dry toluene (10 mL), and the mixture was cooled to -78 °C under nitrogen atmosphere. diisobutylaluminium hydride (7.6 mL, 7.6 mmol) was added dropwise, and after the dropwise addition, the mixture was stirred at the temperature for 2 h. After the reaction was completed as detected by LC-MS, a saturated ammonium chloride solution (10 mL) was added to the reaction solution, and the mixture was warmed to room temperature, stirred for 1 h, and extracted with ethyl acetate (15 mL × 3). The organic phases were combined, washed with water (15 mL), washed with saturated brine (15 mL), dried over anhydrous sodium sulfate, and concentrated to give the target product **1b** (0.8 g, 80% yield).

LC-MS(m/z): 294.1 $[M+H]^+$.

**Intermediate 3**

**(*R*)-2-(4-(5-fluoropyridin-2-yl)-1,9-dioxaspiro[5.5]undecan-4-yl)acetonitrile 1c**

Synthesis scheme:

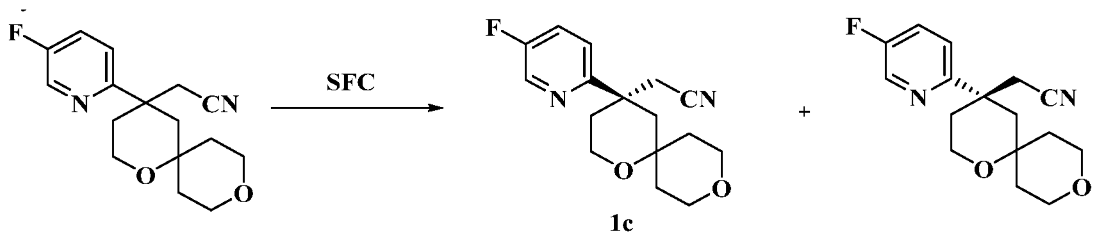


1c

2-(4-(5-Fluoropyridin-2-yl)-1,9-dioxaspiro[5.5]undecan-4-yl)acetonitrile (100 g) was separated to give two optical isomers:

Compound (*R*)-2-(4-(5-fluoropyridin-2-yl)-1,9-dioxaspiro[5.5]undecan-4-yl)acetonitrile 1c, 0.97 min, 45.1 g, ee% = 96%, [a]27.3 = -6.75 (C = 2 g/100 mL, MeOH), LC-MS (m/z): 291.2$[M+H]^+$;
compound (S)-2-(4-(5-fluoropyridin-2-yl)-1,9-dioxaspiro[5.5]undecan-4-yl)acetonitrile, 0.58 min, 46.5 g, ee% = 96%, [a]27.3 = 2.04 (C = 2 g/100 mL, MeOH).

Separation conditions: instrument: Waters SFC 150; column: DAICELCHIRALPAK®AS, 250 × 40 mm 10 μm; mobile phase: A: supercritical $CO_2$, B: MeOH (0.1% ammonia in MeOH); gradient: A:B = 75:25; flow rate: 120 mL/min; back pressure: 100 bar; column temperature: 25 °C; wavelength: 214 nm; cycle: 6 min; the compound to be separated was dissolved in MeOH (1000 mL); injection: 8 mL.

**Example 1**

**(1*S*)-6-fluoro-*N*-(2-(4-(5-fluoropyridin-2-yl)-1,9-dioxaspiro[5.5]undecan-4-yl)ethyl)-2,3-dihydro-1*H*-inden-1-amine 1-1**

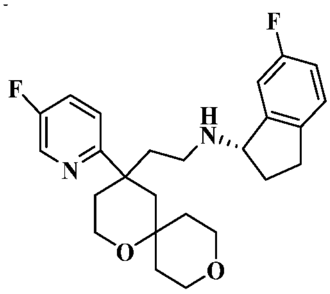

1-1

Synthesis scheme:

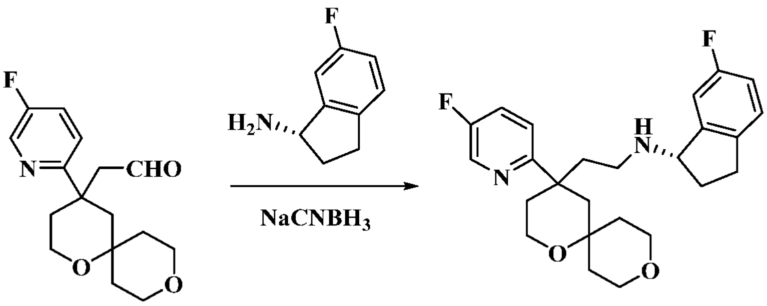


1b 1-1

2-(4-(5-Fluoropyridin-2-yl)-1,9-dioxaspiro[5.5]undecan-4-yl)acetaldehyde (100 mg, 0.34 mmol) and (S)-6-fluoro-2,3-dihydro-1H-inden-1-amine (47 mg, 0.34 mmol) were dissolved in methanol (1 mL), acetic acid (0.1 mL) was added, sodium cyanoborohydride (62 mg, 1.0 mmol) was added, and the mixture was reacted at room temperature for 16 h. After the reaction was completed as detected by LC-MS, the mixture was filtered, concentrated, and purified by preparative HPLC to give the target compound 1-1 (25.3 mg, 11% yield).

LC-MS(m/z): 429.2 $[M+H]^+$.

$^1H$ NMR (600 MHz, DMSO-$d_6$) δ9.28 (s, 1H), 8.55 (d, J= 3.0 Hz, 1H), 7.76-7.71 (m, 1H), 7.66-7.63 (m, 1H), 7.47 (d, *J* = 9.0 Hz, 1H), 7.39-7.29 (m, 1H), 7.24-7.11 (m, 1H), 4.69-4.52 (m, 1H), 3.72-3.70 (m, 1H), 3.64-3.44 (m, 3H), 3.33-3.16 (m, 2H), 2.98 (m, 1H), 2.89-2.70 (m, 2H), 2.58-2.56 (m, 2H), 2.37-2.16 (m, 2H), 2.13-1.79 (m, 3H), 1.66-1.37 (m, 4H), 0.85-0.82 (m, 2H).

Example 2

**(1S)-4-fluoro-N-(2-(4-(5-fluoropyridin-2-yl)-1,9-dioxaspiro [5.5] undecan-4-yl)ethyl)-2,3-dihydro-1*H*-inden-1-amine 2-1**

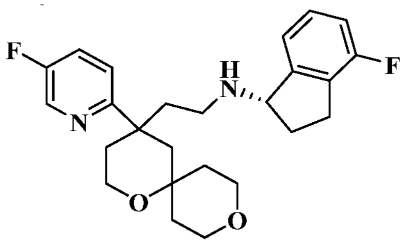

2-1

Synthesis scheme:

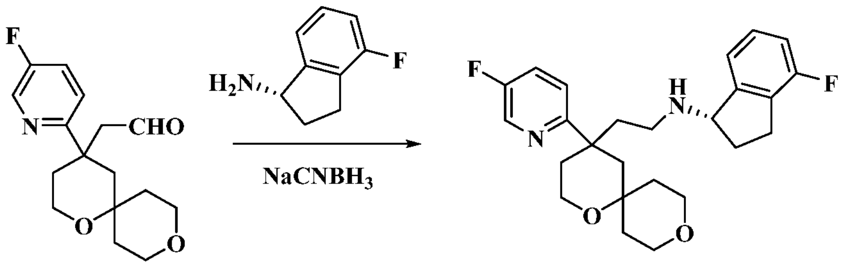


1b 2-1

The starting material (*S*)-6-fluoro-2,3-dihydro-1*H*-inden-1-amine was replaced by (S)-4-fluoro-2,3-dihydro-1*H*-inden-1-amine to prepare the target compound 2-1 according to the synthesis method of Example 1.

LC-MS(m/z): 429.2 $[M+H]^+$.

$^1H$ NMR (600 MHz, DMSO-$d_6$) δ8.80 (s, 1H), 8.58-8.56 (m, 1H), 7.78-7.71 (m, 1H), 7.66-7.62 (m, 1H), 7.34-7.30 (m, 1H), 7.25 (d, J = 7.8 Hz, 1H), 7.19 (t, J = 8.4 Hz, 1H), 4.73-4.66 (m, 1H), 3.71-3.69 (m, 1H), 3.59-3.51 (m, 3H), 3.28-3.20 (m, 2H), 3.03-2.97 (m, 1H), 2.87-2.81 (m, 2H), 2.57-2.52 (m, 2H), 2.40-2.34 (m, 1H), 2.31-2.25 (m, 1H), 2.10-1.91 (m, 2H), 1.79-1.71 (m, 1H), 1.61-1.54 (m, 2H), 1.48 (dd, *J* = 12.0, 6.0 Hz, 1H), 1.42-1,40 (m, 1H), 0.84-0.81 (m, 2H).

Example 3

**(1S)-N-(2-(-4-(5-fluoropyridin-2-yl)-1,9-dioxaspiro [5.5] undecan-4-yl)ethyl)-2,3-dihydro-1H-inden-1-amine** 3-1

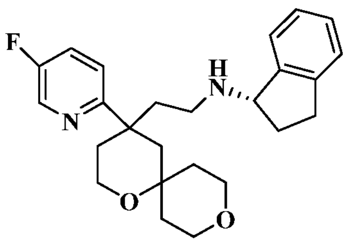

3-1

Synthesis scheme:

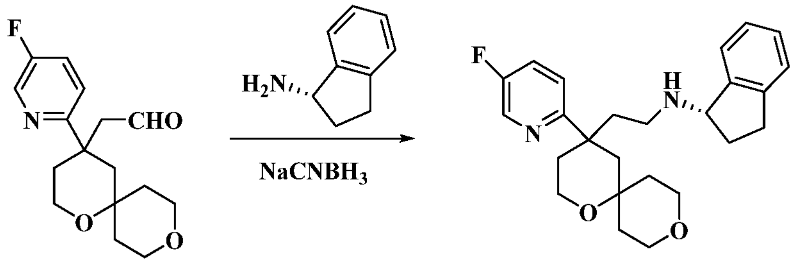


1b 3-1

The starting material (*S*)-6-fluoro-2,3-dihydro-1*H*-inden-1-amine was replaced by (5)-2,3-dihydro-1*H*-inden-1-amine to prepare the target compound 3-1 according to the synthesis method of Example 1.

LC-MS(m/z): 411.2 $[M+H]^+$.

$^1$H NMR (600 MHz, $CDCl_3$) δ8.39 (d, *J* = 2.4 Hz, 1H), 7.38-7.31 (m, 2H), 7.23-7.10 (m, 4H), 4.65-4.62 (m, 1H), 4.17-4.01 (m, 1H), 3.83-3.59 (m, 5H), 3.48-3.23 (m, 2H), 2.99-2.85 (m, 1H), 2.81-2.68 (m, 1H), 2.63-2.50 (m, 2H), 2.46 (d, *J* = 13.7 Hz, 1H), 2.31-2.21 (m, 1H), 2.17 (m, 1H), 1.96-1.85 (m, 1H), 1.82-1.63 (m, 4H), 1.50 (m, 1H), 0.92-0.87 (m, 2H).

**Example 4**

**6-chloro-N-(2-(4-(5-fluoropyridin-2-yl)-1,9-dioxaspiro [5.5] undecan-4-yl)ethyl)-2,3-dihydro-1*H*-inden-1-amine 4**

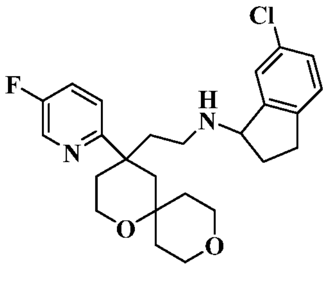

**4**

Synthesis scheme:

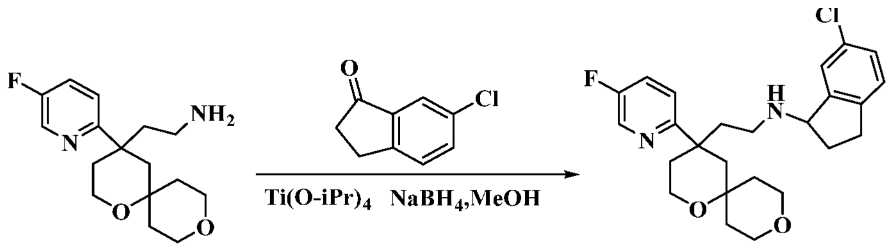


**1a** **4**

2-(4-(5-Fluoropyridin-2-yl)-1,9-dioxaspiro[5.5]undecan-4-yl)ethyl-1-amine (150 mg, 0.51 mmol) and 6-chloro-2,3-dihydro-1H-inden-1-one (84.69 mg, 0.51 mmol) were dissolved in titanium tetraisopropoxide (2 mL), and the mixture was stirred at 80 °C for 4 h. Then methanol (3 mL) was added for dilution, sodium borohydride (37.83 mg, 1.0 mmol) was added, and the mixture was stirred at room temperature for 0.5-1 h, after which water was added to quench the reaction. The reaction solution was filtered through celite, the filter cake was washed with ethyl acetate, and the resulting filtrate was washed with saturated brine, dried and concentrated, and purified by reversed-phase C18 column (water/acetonitrile = 25%) to give the target compound **4** (39 mg, 17% yield).

LC-MS(m/z): 445.2 $[M+H]^+$.

$^1$H NMR (600 MHz, DMSO-$d_6$) δ8.69 (s, 1H), 8.57 (dd, *J* = 6.6, 3.0 Hz, 1H), 7.77-7.72 (m, 1H), 7.66-7.64 (m, 1H), 7.45 (d, J= 6.0 Hz, 1H), 7.39 (dd, *J* = 7.8, 1.8 Hz, 1H), 7.34 (dd, *J* = 7.8, 3.0 Hz, 1H), 4.68-4.59 (m, 1H), 3.72-3.69 (m, 1H), 3.60-3.51 (m, 3H), 3.29-3.20 (m, 2H), 3.00-2.93 (m, 1H), 2.90-2.77 (m, 2H), 2.59-2.52 (m, 1H), 2.37-2.22 (m, 3H), 2.05-1.91 (m, 2H), 1.81-1.70 (m, 1H), 1.62-1.55 (m, 2H), 1.51-1.48 (m, 1H), 1.43-1.40 (m, 1H), 0.85-0.81 (m, 2H).

**Example 5**

**(1*S*)-4-chloro-*N*-(2-(4-(5-fluoropyridin-2-yl)-1,9-dioxaspiro [5.5] undecan-4-yl)ethyl)-2,3-dihydro-1*H*-inden-1-amine 5-1**

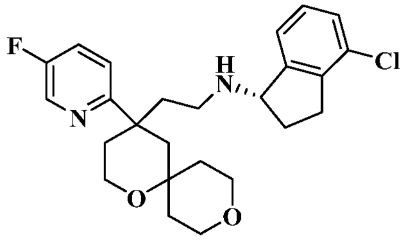

5-1

Synthesis scheme:

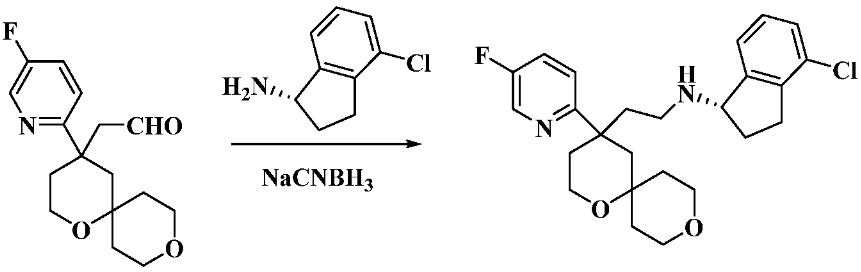


1b 5-1

The starting material (S)-6-fluoro-2,3-dihydro-1H-inden-1-amine was replaced by (S)-4-chloro-2,3-dihydro-1*H*-inden-1-amine to prepare the target compound 5-1 according to the synthesis method of Example 1.

LC-MS(m/z): 445.2 $[M+H]^+$.

$^1H$ NMR (600 MHz, DMSO-$d_6$) δ8.70 (s, 1H), 8.57 (d, *J* = 2.4 Hz, 1H), 7.74 (d, *J* = 6.0 Hz, 1H), 7.68-7.61 (m, 1H), 7.45 (d, *J* = 7.8 Hz, 1H), 7.38 (dd, *J* = 7.2, 3.6 Hz, 1H), 7.33-7.29 (m, 1H), 4.88-4.48 (m, 1H), 3.70-3.69 (m, 1H), 3.57-3.54 (m, 4H), 3.27-3.21 (m, 2H), 3.00 (dd, *J* = 16.2, 8.4 Hz, 1H), 2.87-2.84 (m, 2H), 2.57-2.55 (m, 1H), 2.38-2.35 (m, 2H), 2.07-1.92 (m, 2H), 1.75-1.72 (m, 1H), 1.59-1.55 (m, 2H), 1.48 (dd, *J* = 13.8, 6.0 Hz, 1H), 1.42-1,40 (m, 1H), 0.84-0.80 (m, 2H).

Example 6

**(1S)-N-(2-(4-(5-fluoropyridin-2-yl)-1,9-dioxaspiro [5.5] undecan-4-yl)ethyl)-4-methyl-2,3-dihydro-1*H*-inden-1-amine 6-1**

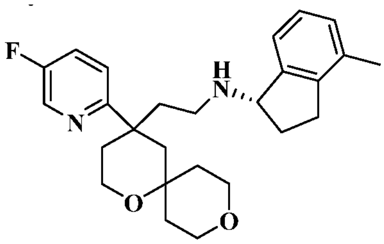

6-1

Synthesis scheme:

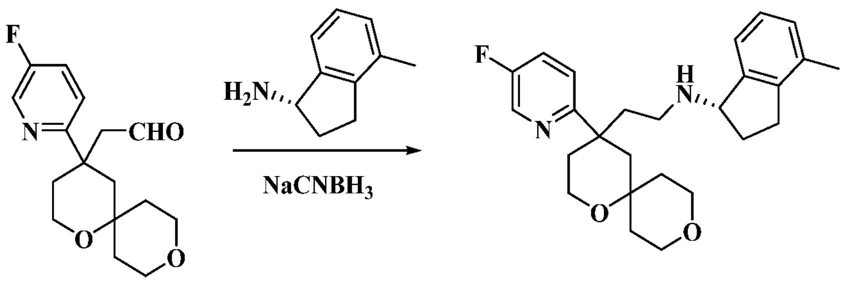


1b 6-1

The starting material (*S*)-6-fluoro-2,3-dihydro-1*H*-inden-1-amine was replaced by (S)-4-methyl-2,3-dihydro-1*H*-inden-1-amine to prepare the target compound 6-1 according to the synthesis method of Example 1.

LC-MS(m/z): 425.2 [M+H]$^+$.

$^1$H NMR (600 MHz, DMSO-$d_6$) δ9.25-8.51 (m, 2H), 7.78-7.68 (m, 1H), 7.66-7.62 (m, 1H), 7.23-7.11 (m, 3H), 4.64-4.61 (m, 1H), 3.70-3.69 (m, 1H), 3.59-3.54 (m, 4H), 3.26-3.22 (m, 2H), 2.92-2.75 (m, 3H), 2.57-2.52 (m, 1H), 2.34-2.28 (m, 2H), 2.22 (s, 3H), 2.04-1.84 (m, 2H), 1.79-1.71 (m, 1H), 1.60-1.55 (m, 2H), 1.50-1.46 (m, 1H), 1.42-1.40 (m, 1H), 0.83-0.80 (m, 2H).

## Example 7

**(1*S*)-4-bromo-*N*-(2-(4-(5-fluoropyridin-2-yl)-1,9-dioxaspiro [5.5] undecan-4-yl)ethyl)-2,3-dihydro-1*H*-inden-1-amine 7-1**

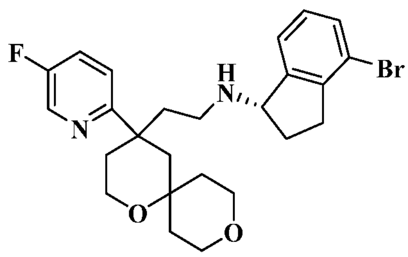

7-1

Synthesis scheme:

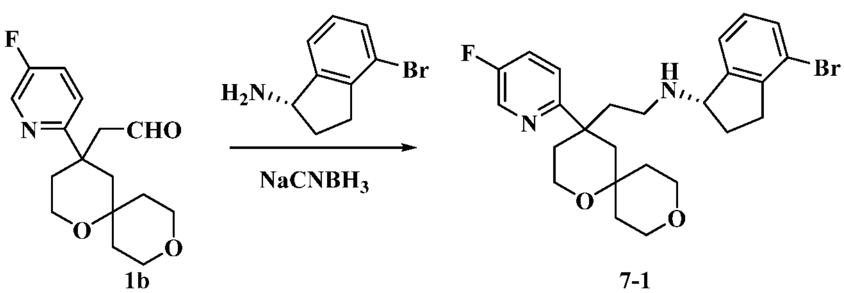


1b 7-1

The starting material (S)-6-fluoro-2,3-dihydro-1H-inden-1-amine was replaced by (*S*)-4-bromo-2,3-dihydro-1*H*-inden-1-amine to prepare the target compound 7-1 according to the synthesis method of Example 1.

LC-MS(m/z): 489.1 [M+H]$^+$.

$^1$H NMR (600 MHz, DMSO-$d_6$) δ8.70 (s, 1H), 8.57 (d, *J* = 2.4 Hz, 1H), 7.77-7.72 (m, 1H), 7.66-7.63 (m, 1H), 7.59 (d, *J* = 7.8 Hz, 1H), 7.41 (dd, *J* = 7.8, 3.6 Hz, 1H), 7.25-7.21 (m, 1H), 4.76-4.73 (m, 1H), 3.70-3.69 (m, 1H), 3.59-3.54 (m, 4H), 3.26-3.22 (m, 2H), 3.00-2.94 (m, 1H), 2.87-2.81 (m, 2H), 2.57-2.55 (m, 1H), 2.38-2.35 (m, 2H), 2.07-1.92 (m,

2H), 1.75-1.72 (m, 1H), 1.59-1.55 (m, 2H), 1.48 (dd, *J* = 13.8, 6.0 Hz, 1H), 1.42-1,40 (m, 1H), 0.84-0.80 (m, 2H).

Example 8

***N*-(2-(4-(5-fluoropyridin-2-yl)-1,9-dioxaspiro[5.5]undecan-4-yl)ethyl)-2,3-dihydro-1*H*-inden-2-amine** 8

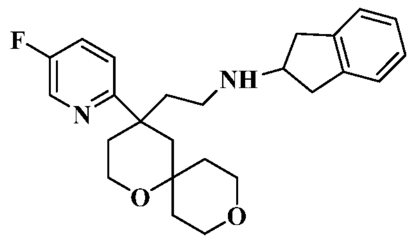

**8**

Synthesis scheme:

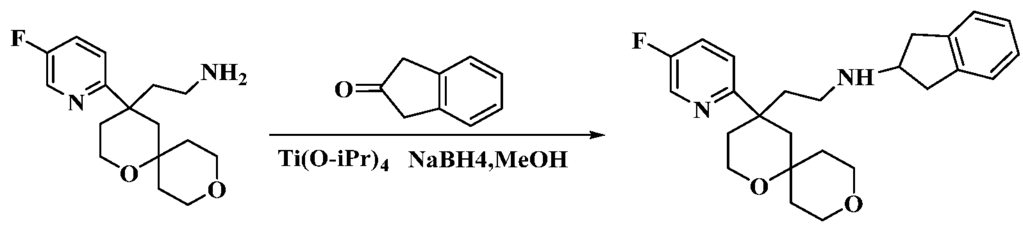


**1a** **8**

The starting material 6-chloro-2,3-dihydro-1H-inden-1-one was replaced by 1,3-dihydro-2H-inden-2-one to prepare the target compound **8** according to the synthesis method of Example 4. LC-MS(m/z): 411 $[M+H]^+$.

$^1H$ NMR (600 MHz, DMSO-$d_6$) δ8.57 (d, J = 3.0 Hz, 1H), 7.78-7.74 (m, 1H), 7.64 (dd, *J* = 9.0, 4.2 Hz, 1H), 7.23-7.17 (m, 4H), 4.54-4.51 (m, 1H), 3.91-3.86 (m, 1H), 3.72-3.70 (m, 1H), 3.62-3.50 (m, 3H), 3.27-3.15 (m, 4H), 2.94-2.82 (m, 2H), 2.56-2.53 (m, 2H), 2.29-2.27 (m, 1H), 1.96-1.94 (m, 1H), 1.78-1.74 (m, 1H), 1.61-1.39 (m, 5H), 0.81-0.76 (m, 2H).

**Example 9**

**(*R*)-*N*-(2-(4-(5-fluoropyridin-2-yl)-1,9-dioxaspiro[5.5]undecan-4-yl)ethyl)-2,3-dihydro-1*H***-inden-2-amine 8-1

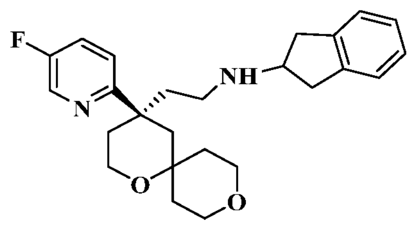

**8-1**

Synthesis scheme:

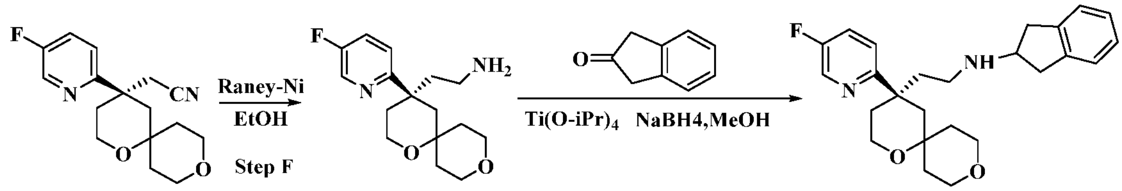


1c 1a-1 8-1

**Step A: preparation of (R)-2-(4-(5-fluoropyridin-2-yl)-1,9-dioxaspiro[5.5]undecan-4-yl)ethyl-1-amine**

(*R*)-2-(4-(5-Fluoropyridin-2-yl)-1,9-dioxaspiro[5.5]undecan-4-yl)acetonitrile **1c** (1.5 g, 5.2 mmol) was dissolved in ethanol (30 mL) and methanol (5 mL). After ammonia water (2 mL) was added dropwise, 5 spoons of Raney nickel were added, and the mixture was stirred at room temperature overnight under hydrogen atmosphere. After the reaction was completed, the mixture was filtered through celite, and the filtrate was concentrated to give a yellow oily product of (*R*)-2-(4-(5-fluoropyridin-2-yl)-1,9-dioxaspiro[5.5]undecan-4-yl)ethyl-1-amine **1a-1** (1.4 g, 91% yield).

LC-MS(m/z): 295.1 $[M+H]^+$.

**Step B: preparation of (*R*)-N-(2-(4-(5-fluoropyridin-2-yl)-1,9-dioxaspiro[5.5]undecan-4-yl)ethyl)-2,3-dihydro-1H-inden-2-amine**

(*R*)-2-(4-(5-Fluoropyridin-2-yl)-1,9-dioxaspiro[5.5]undecan-4-yl)ethyl-1-amine **1a-1** (150 mg, 0.51 mmol) and 1,3-dihydro-2*H*-inden-2-one (67.32 mg, 0.51 mmol) were dissolved in titanium tetraisopropoxide (2 mL), and the mixture was stirred at 80 °C for 4 h. Then methanol (3 mL) was added for dilution, sodium borohydride (37.83 mg, 1.0 mmol) was added, and the mixture was stirred at room temperature for 0.5-1 h, after which water was added to quench the reaction. The reaction solution was filtered through celite, the filter cake was washed with ethyl acetate, and the resulting filtrate was washed with saturated brine, dried and concentrated, and purified by reversed-phase C18 column (water/acetonitrile = 25%) to give the target compound **8-1** (41 mg, 19% yield).

LC-MS(m/z): 411 $[M+H]^+$.

$^1H$ NMR (400 MHz, MeOD) δ 8.44 (d, J = 2.4 Hz, 1H), 7.61 - 7.54 (m, 2H), 7.16 - 7.05 (m, 4H), 3.81 - 3.75 (m, 2H), 3.73 (dd, J = 11.0, 2.9 Hz, 1H), 3.68 - 3.60 (m, 1H), 3.45 - 3.33 (m, 4H), 3.03 (dd, J = 15.7, 7.3 Hz, 2H), 2.68 - 2.49 (m, 5H), 1.98 (td, J = 12.5, 6.0 Hz, 2H), 1.69 (ddt, *J* = 11.8, 7.6, 2.6 Hz, 3H), 1.59 (d, J = 13.9 Hz, 1H), 1.53 - 1.45 (m, 1H), 0.97 (dd, J = 7.4, 4.4 Hz, 2H).

**Example 10**

**5-fluoro-*N*-(2-(4-(5-fluoropyridin-2-yl)-1,9-dioxaspiro[5.5]undecan-4-yl)ethyl)-2,3-dihydro-1*H*-inden-2-amine 9**

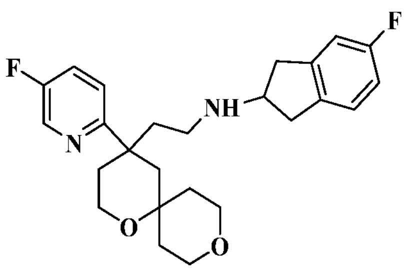

9

Synthesis scheme:

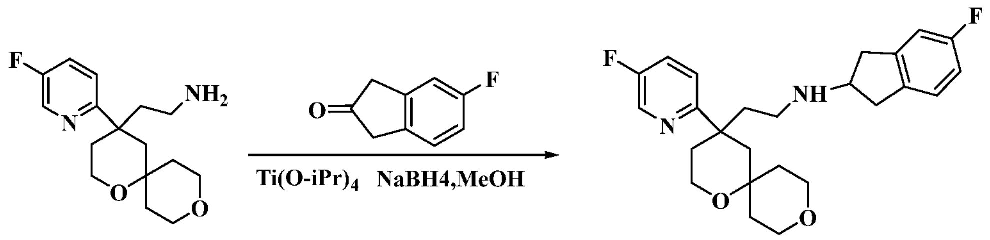


1a 9

The starting material 6-chloro-2,3-dihydro-1H-inden-1-one was replaced by 5-fluoro-1,3-dihydro-2*H*-inden-2-one to prepare the target compound 9 according to the synthesis method of Example 4.

LC-MS(m/z): 429 $[M+H]^+$.

$^1$H NMR (600 MHz, DMSO-$d_6$) δ8.57 (d, *J* = 3.0 Hz, 1H), 7.75 (td, *J* = 6.0, 3.0 Hz, 1H), 7.64 (dd, *J* = 9.0, 4.2 Hz, 1H), 7.25-7.23 (m, 1H), 7.08 (d, *J* = 9.0 Hz, 1H), 7.00 (t, *J* = 9.6 Hz, 1H), 4.58-4.55 (m, 1H), 3.95-3.91 (m, 1H), 3.72-3.70 (m, 1H), 3.60-3.52 (m, 3H), 3.27-3.12 (m, 4H), 2.95-2.82 (m, 3H), 2.55-2.53 (m, 2H), 2.28-2.26 (m, 1H), 1.98-1.93 (m, 1H), 1.77-1.73 (m, 1H), 1.61-1.41 (m, 4H), 0.82-0.80 (m, 2H).

**Example 11**

**4-bromo-*N*-(2-(4-(5-fluoropyridin-2-yl)-1,9-dioxaspiro[5.5]undecan-4-yl)ethyl)-2,3-dihydro-1*H*-inden-2-amine 10**

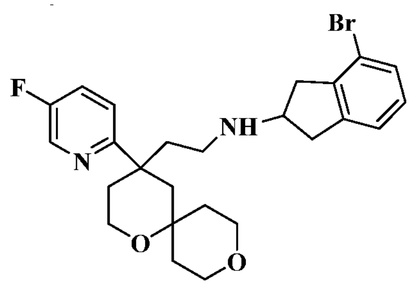

10

Synthesis scheme:

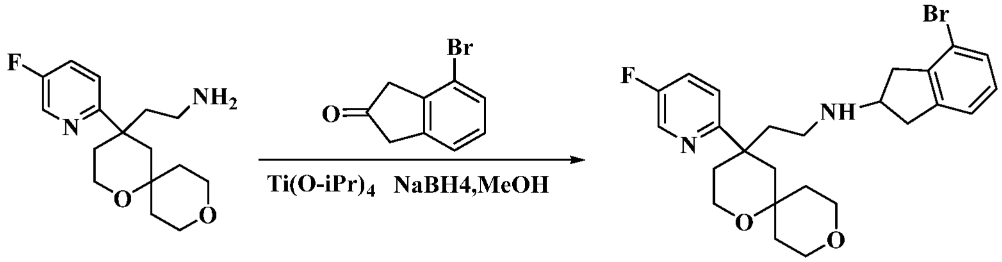


1a 10

The starting material 6-chloro-2,3-dihydro-1*H*-inden-1-one was replaced by 4-bromo-1,3-dihydro-2*H*-inden-2-one to prepare the target compound 10 according to the synthesis method of Example 4.

LC-MS(m/z): 490 $[M+H]^+$.

$^1H$ NMR (600 MHz, DMSO-$d_6$) δ8.57 (d, J = 3.0 Hz, 1H), 7.77-7.74 (m, 1H), 7.66-7.63 (m, 1H), 7.41 (d, J = 7.8 Hz, 1H), 7.25 (d, J = 7.8 Hz, 1H), 7.17-7.14 (m, 1H), 3.96-3.94 (m, 1H), 4.53-4.49 (m, 1H), 3.72-3.70 (m, 1H), 3.60-3.52 (m, 3H), 3.33-3.17 (m, 4H), 3.05-2.84 (m, 3H), 2.54-2.52 (m, 2H), 2.34-2.26 (m, 1H), 1.95-1.92 (m, 1H), 1.76-1.74 (m, 1H), 1.60-1.40 (m, 4H), 0.82-0.80 (m, 2H).

## Example 12

**(S)-4-bromo-N-(2-((R)-4-(5-fluoropyridin-2-yl)-1,9-dioxaspiro [5.5] undecan-4-yl)ethyl)-2,3-dihydro-1H-inden-2-amine 10-1-1 and (*R*)-4-bromo-*N*-(2-((*R*)-4-(5-fluoropyridin-2-yl)-1,9-dioxaspiro[5.5]undecan-4-yl)ethyl)-2,3-dihydro-1H-inden-2-amine 10-1-2**

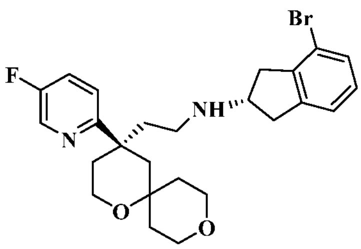

10-1-1

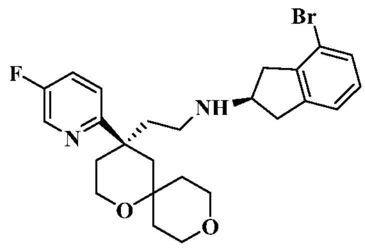

10-1-2

Synthesis scheme:

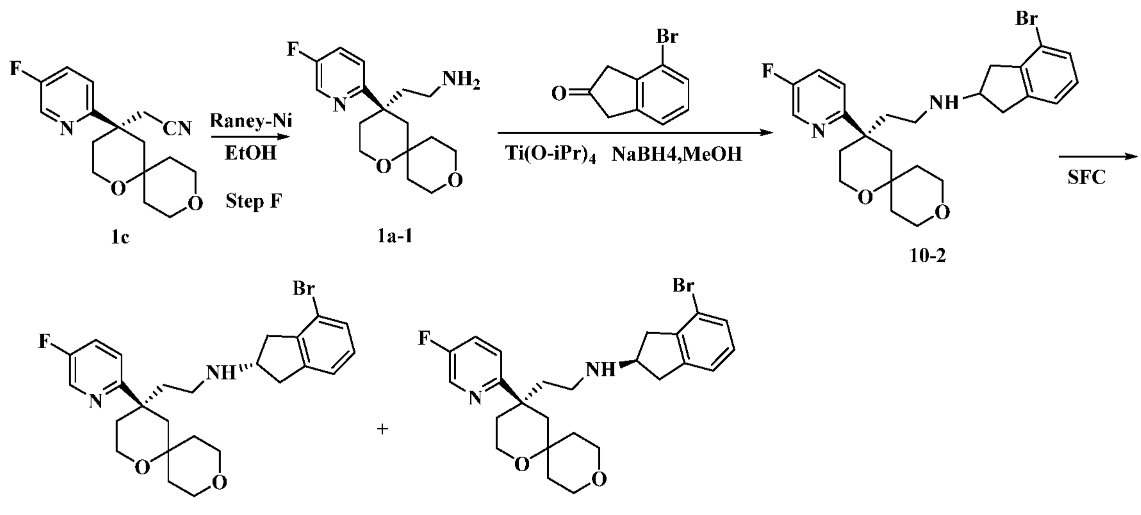


1c 1a-1 10-2

+

10-1-1 10-1-2

The starting material 1,3-dihydro-2*H*-inden-2-one was replaced by 4-bromo-1,3-dihydro-2*H*-inden-2-one to prepare the target compound **10-2** according to the synthesis method of Example 9.

Compound **10-2** was separated to give two optical isomers:

(*S*)-4-bromo-*N*-(2-((R)-4-(5-fluoropyridin-2-yl)-1,9-dioxaspiro[5.5]undecan-4-yl)ethyl)-2,3-dihydro-1*H*-inden-2-amine **10-1-1,** 2.08 min, 0.16 g, ee% = 96%;

LC-MS(m/z): 490.5 $[M+H]^+$.

$^1H$ NMR (400 MHz, DMSO) δ 8.45 (d, J = 2.9 Hz, 1H), 7.60 (td, J = 8.7, 3.0 Hz, 1H), 7.53 (dd, J = 8.9, 4.4 Hz, 1H), 7.22 (d, J = 7.9 Hz, 1H), 7.07 (d, J = 7.4 Hz, 1H), 6.97 (t, J = 7.6 Hz, 1H), 3.60 (ddd, J = 12.1, 5.0, 2.5 Hz,

1H), 3.55 - 3.42 (m, 3H), 3.24 - 3.10 (m, 4H), 2.94 (dd, J = 16.1, 7.0 Hz, 1H), 2.83 (dd, J = 16.3, 7.1 Hz, 1H), 2.51 (ddd, J = 15.9, 14.8, 4.0 Hz, 2H), 2.43 - 2.35 (m, 2H), 2.28 (td, J = 11.0, 5.0 Hz, 1H), 1.82 (td, J = 11.0, 4.7 Hz, 1H), 1.75 - 1.67 (m, 1H), 1.52 - 1.43 (m, 3H), 1.39 (d, J = 13.8 Hz, 1H), 1.32 (dt, J = 13.6, 3.6 Hz, 1H), 0.75 (d, J = 4.9 Hz, 2H).

(R)-4-bromo-N-(2-((R)-4-(5-fluoropyridin-2-yl)-1,9-dioxaspiro[5.5]undecan-4-yl)ethyl)-2,3-dihydro-1H-inden-2-amine **10-1-2,** 2.98 min, 0.15 g, ee% = 96%;

LC-MS(m/z): 490.5 $[M+H]^+$.
$^1$H NMR (400 MHz, DMSO-$d_6$) δ 8.52 (d, J = 2.9 Hz, 1H), 7.67 (td, J = 8.8, 3.0 Hz, 1H), 7.60 (dd, J = 8.9, 4.4 Hz, 1H), 7.29 (d, J = 7.8 Hz, 1H), 7.13 (d, J = 7.3 Hz, 1H), 7.04 (t, J = 7.6 Hz, 1H), 3.68 (ddd, J = 12.1, 4.9, 2.4 Hz, 1H), 3.64 - 3.46 (m, 3H), 3.32 - 3.15 (m, 5H), 3.01 (dd, J = 16.0, 7.0 Hz, 1H), 2.89 (dd, J = 16.3, 7.0 Hz, 1H), 2.62 - 2.52 (m, 2H), 2.49 - 2.43 (m, 1H), 2.36 (td, J = 10.9, 5.0 Hz, 1H), 1.92 - 1.73 (m, 2H), 1.56 (dq, J = 8.6, 4.1, 3.3 Hz, 2H), 1.52 (d, J = 5.2 Hz, 1H), 1.49 - 1.34 (m, 2H), 0.81 (p, J = 4.7, 3.8 Hz, 2H).

Separation conditions: instrument: Waters SFC 150; column: DAICELCHIRALCEL®OZ, 250 × 25 mm 10 μm; mobile phase: A: supercritical $CO_2$, B: MeOH (0.1% ammonia in MeOH); gradient: A:B = 50:50; flow rate: 70 mL/min; back pressure: 100 bar; column temperature: 25 °C; wavelength: 214 nm; cycle: 6 min; the compound to be separated was dissolved in MeOH (40 mL); injection: 1.8 mL.

**Example 13**

**N-(3-chlorobenzyl)-2-(4-(5-fluoropyridin-2-yl)-1,9-dioxaspiro[5.5]undecan-4-yl)ethan-1-amine 11**

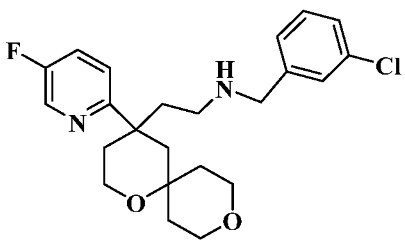

11

Synthesis scheme:

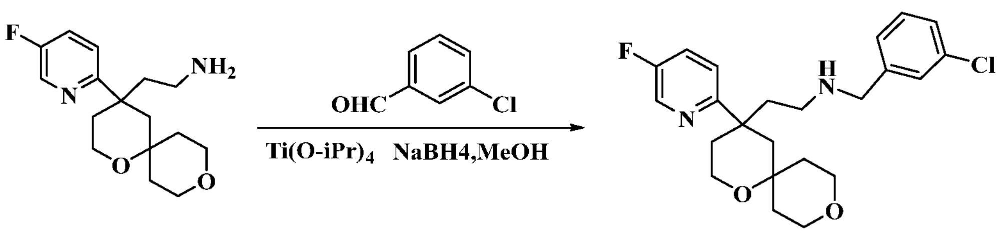


1a 11

The starting material 6-chloro-2,3-dihydro-1H-inden-1-one was replaced by 3-chlorobenzaldehyde to prepare the target compound 11 according to the synthesis method of Example 4.
LC-MS(m/z): 419.2 $[M+H]^+$.
$^1$H NMR (600 MHz, $CD_3OD$) δ8.48-8.40 (m, 1H), 7.62-7.52 (m, 2H), 7.45-7.36 (m, 3H), 7.31 (d, J= 7.4 Hz, 1H), 4.58-4.54 (m, 1H), 4.12-4.01 (m, 2H), 3.79-3.57 (m, 4H), 3.37-3.31 (m, 2H), 3.02-2.94 (m, 1H), 2.65-2.55 (m, 2H), 2.42 (td, *J* = 12.5, 4.4 Hz, 1H), 2.14-2.03 (m, 1H), 1.89-1.80 (m, 1H), 1.75-1.62 (m, 2H), 1.56 (d, J= 13.9 Hz, 1H), 1.45 (dd, *J* = 19.9, 8.8 Hz, 1H), 0.96-0.87 (m, 2H).

**Example 14**

***N*-(3-chloro-2-methylbenzyl)-2-(4-(5-fluoropyridin-2-yl)-1,9-dioxaspiro[5.5]undecan-4-yl)ethan-1-amine 12**

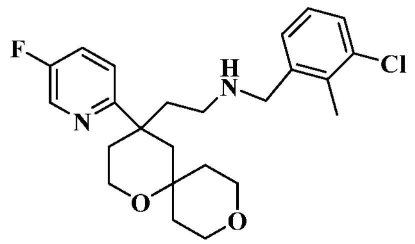

**12**

Synthesis scheme:

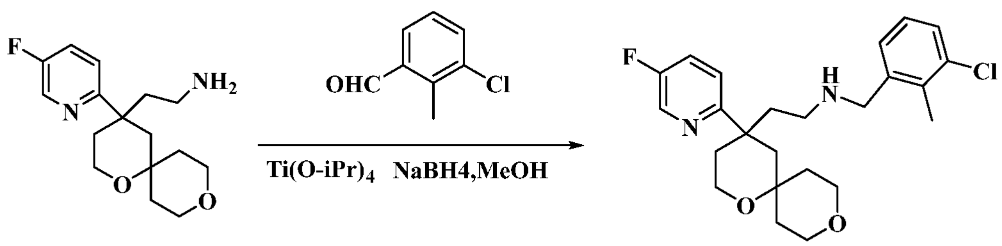


**1a** **12**

The starting material 6-chloro-2,3-dihydro-1*H*-inden-1-one was replaced by 3-chloro-2-methylbenzaldehyde to prepare the target compound **12** according to the synthesis method of Example 4.

LC-MS(m/z): 433.2 $[M+H]^+$.

$^1H$ NMR (600 MHz, $CD_3OD$) δ8.48 (d, *J* = 2.0 Hz, 1H), 7.64-7.54 (m, 2H), 7.45 (dd, *J* = 7.7, 1.2 Hz, 1H), 7.31-7.14 (m, 2H), 4.26-4.10 (m, 2H), 4.56-4.52 (m, 1H), 3.83-3.68 (m, 3H), 3.61 (m, 1H), 3.42-3.33 (m, 2H), 3.04 (td, *J* = 12.6, 4.5 Hz, 1H), 2.63 (m, 2H), 2.52-2.46 (m, 1H), 2.39 (s, 3H), 2.09 (td, *J* = 12.9, 4.5 Hz, 1H), 1.84 (td, *J* = 12.8, 4.6 Hz, 1H), 1.76-1.64 (m, 2H), 1.56 (d, J= 13.8 Hz, 1H), 1.46 (d, *J*= 13.7 Hz, 1H), 1.00-0.87 (m, 2H).

**Example 15**

**(*R*)-*N*-(3-chloro-2-methylbenzyl)-2-(4-(5-fluoropyridin-2-yl)-1,9-dioxaspiro[5.5]undecan-4-yl)ethan-1-amine 12-1**

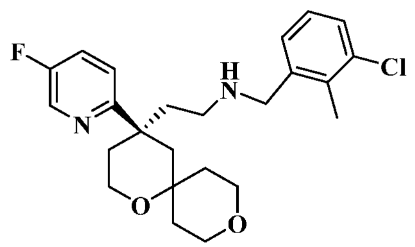

**12-1**

Synthesis scheme:

The starting material 1,3-dihydro-2*H*-inden-2-one was replaced by 3-chloro-2-methylbenzaldehyde to prepare the target compound **12-1** according to the synthesis method of Example 9.

LC-MS(m/z): 433.2 $[M+H]^+$.

$^1$H NMR (400 MHz, Methanol-d4) δ 8.29 (t, J = 1.8 Hz, 1H), 7.45 - 7.38 (m, 2H), 7.14 (dd, J = 5.6, 3.8 Hz, 1H), 6.99 - 6.93 (m, 2H), 3.67 - 3.56 (m, 3H), 3.56 - 3.46 (m, 3H), 3.28 - 3.22 (m, 2H), 2.47 (ddd, J = 14.3, 10.0, 2.3 Hz, 2H), 2.38 (td, J = 12.2, 11.7, 5.1 Hz, 1H), 2.19 (s, 3H), 1.92 - 1.80 (m, 2H), 1.64 - 1.50 (m, 3H), 1.46 (d, J = 14.1 Hz, 1H), 1.41 - 1.32 (m, 1H), 0.84 (dd, J = 7.3, 4.5 Hz, 2H).

## Example 16

***N*-(2,3-dichlorobenzyl)-2-(4-(5-fluoropyridin-2-yl)-1,9-dioxaspiro[5.5]undecan-4-yl)ethan-1-amine 13**

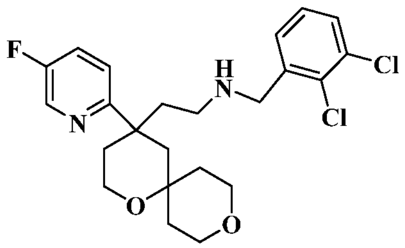

13

Synthesis scheme:

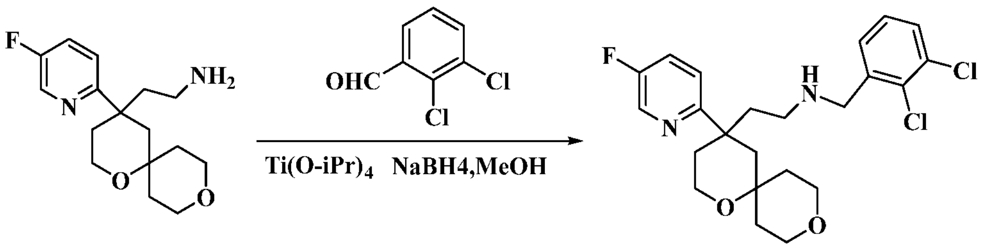


1a 13

The starting material 6-chloro-2,3-dihydro-1*H*-inden-1-one was replaced by 2,3-dichlorobenzaldehyde to prepare the target compound **13** according to the synthesis method of Example 4.

LC-MS(m/z): 453.2 $[M+H]^+$.

$^1$H NMR (600 MHz, $CD_3OD$) δ8.48-8.40 (m, 1H), 7.62-7.52 (m, 2H), 7.45-7.36 (m, 3H), 7.31 (d, *J* = 7.4 Hz, 1H), 4.12-4.01 (m, 2H), 3.79-3.57 (m, 4H), 3.37-3.31 (m, 2H), 3.02-2.94 (m, 1H), 2.65-2.55 (m, 2H), 2.42 (td, *J* = 12.5, 4.4 Hz, 1H), 2.14-2.03 (m, 1H), 1.89-1.80 (m, 1H), 1.75-1.62 (m, 2H), 1.56 (d, *J* = 13.9 Hz, 1H), 1.45 (dd, *J* = 19.9, 8.8 Hz, 1H), 0.96-0.87 (m, 2H).

## Example 17

**(*R*)-*N*-(2,3-dichlorobenzyl)-2-(4-(5-fluoropyridin-2-yl)-1,9-dioxaspiro[5.5]undecan-4-yl)ethan-1-amine 13-1**

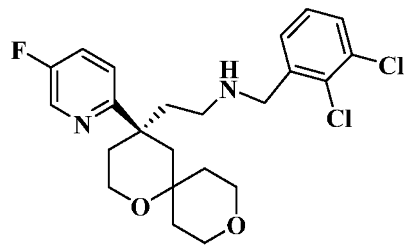

13-1

The starting material 1,3-dihydro-2*H*-inden-2-one was replaced by 2,3-dichlorobenzaldehyde to prepare the target compound **13-1** according to the synthesis method of Example 9.

LC-MS(m/z): 454 $[M+H]^+$.

$^1H$ NMR (600 MHz, Deuterium Oxide) δ 8.37 (d, J = 2.8 Hz, 1H), 7.67 (td, J = 8.4, 2.9 Hz, 1H), 7.59 (dd, J = 9.0, 4.3 Hz, 1H), 7.55 (p, J = 4.2 Hz, 1H), 7.26 - 7.21 (m, 2H), 4.24 - 4.16 (m, 2H), 3.74 (dd, J = 12.8, 4.5 Hz, 1H), 3.68 (t, J = 12.4 Hz, 1H), 3.61 (dd, J = 8.6, 2.7 Hz, 2H), 3.28 (tp, J = 11.7, 4.0 Hz, 2H), 2.98 (td, J = 12.6, 4.8 Hz, 1H), 2.44 (dd, J = 20.9, 14.6 Hz, 2H), 2.25 (td, J = 12.7, 4.3 Hz, 1H), 2.04 - 1.96 (m, 2H), 1.84 (td, J = 12.9, 4.8 Hz, 1H), 1.73 - 1.59 (m, 3H), 1.44 (d, J = 14.1 Hz, 1H), 0.87 (dd, J = 9.2, 4.6 Hz, 2H).

**Example 18**

***N*-(3-chloro-2-fluorobenzyl)-2-(4-(5-fluoropyridin-2-yl)-1,9-dioxaspiro [5.5] undecan-4-yl)ethan-1-amine 14**

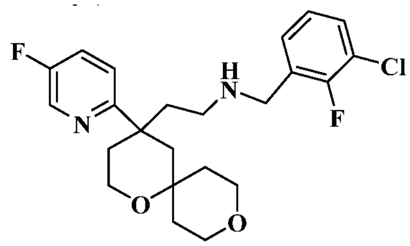

14

Synthesis scheme:

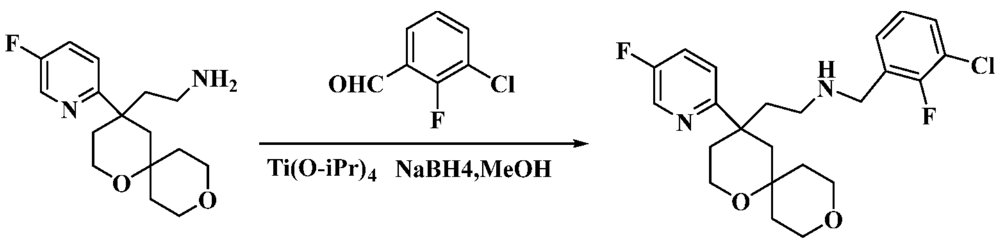


1a 14

The starting material 6-chloro-2,3-dihydro-1*H*-inden-1-one was replaced by 3-chloro-2-fluorobenzaldehyde to prepare the target compound **14** according to the synthesis method of Example 4.

LC-MS(m/z): 438 $[M+H]^+$.

$^1H$ NMR (600 MHz, DMSO-$d_6$) δ8.57 (d, J = 3.0 Hz, 1H), 7.74 (td, *J* = 9.0, 3.0 Hz, 1H), 7.67-7.62 (m, 2H), 7.43 (t, *J* = 6.0 Hz, 1H), 7.28 (t, *J* = 7.8 Hz, 1H), 4.43-4.39 (m, 1H), 4.14-4.12 (m, 2H), 3.71-3.69 (m, 1H), 3.59-3.51 (m, 3H), 3.27-3.21 (m, 2H), 2.88-2.86 (m, 1H), 2.56-2.53 (m, 2H), 2.31-2.30 (m, 1H), 1.96-1.94 (m, 1H), 1.78-1.75 (m, 1H), 1.59-1.40 (m, 4H), 0.82-0.80 (m, 2H).

**Example 19**

***N*-(2-(4-(pyridin-2-yl)-1,9-dioxaspiro[5.5]undecan-4-yl)ethyl)-2,3-dihydro-1H-inden-1-amine 15**

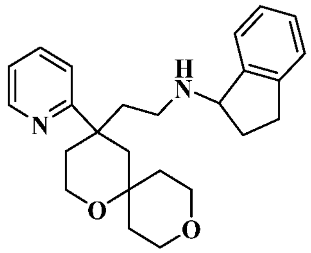

15

Synthesis scheme:

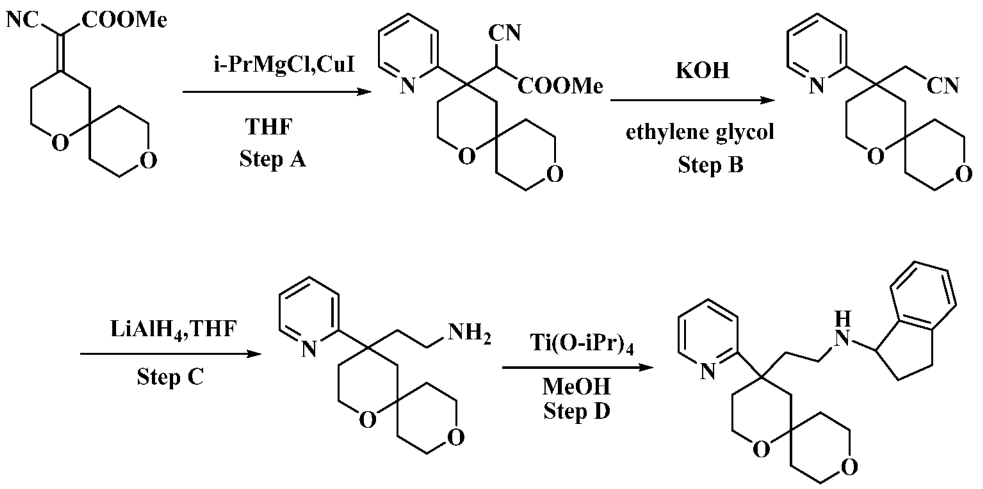


15

**Step A: synthesis of methyl 2-cyano-2-(4-(pyridin-2-yl)-1,9-dioxaspiro[5.5]undecan-4-yl)acetate**

2-Bromopyridine (4.74 g, 30 mmol) was dissolved in anhydrous tetrahydrofuran (20 mL), and the mixture was slowly added dropwise to an isopropyl magnesium chloride solution (15 mL, 30 mmol) at 0 °C and reacted at room temperature for 3 h. Cuprous iodide (0.57 g, 3 mmol) was added, and the mixture was reacted for another 1 h. Methyl (*Z*)-2-cyano-2-(1,9-dioxaspiro[5.5]undecane-4-ylidene)acetate (5.0 g, 20 mmol) was dissolved in tetrahydrofuran (15 mL) and added dropwise therein, and the mixture was reacted overnight at room temperature. A saturated ammonium chloride solution (50 mL) was added, and the mixture was extracted with ethyl acetate (50 mL × 3). The organic phase was washed with water (50 mL), washed with saturated brine (50 mL), dried over anhydrous sodium sulfate, subjected to suction filtration and concentration, and purified by column chromatography (petroleum ether:ethyl acetate = 3:1) to give the target product (3.3 g, 50% yield).

LC-MS(m/z): 331 $[M+H]^+$.

**Step B: synthesis of 2-(4-(pyridin-2-yl)-1,9-dioxaspiro[5.5]undecan-4-yl)acetonitrile**

Methyl 2-cyano-2-(4-(pyridin-2-yl)-1,9-dioxaspiro[5.5]undecan-4-yl)acetate (3.0 g, 9.1 mmol) was dissolved in ethylene glycol (10 mL). Potassium hydroxide (1 g, 17 mmol) was added, and the mixture was reacted at 110 °C for 3 h. Water (10 mL) was added to the reaction system, and the mixture was extracted with ethyl acetate (20 mL × 3). The ethyl acetate layer was washed with water (10 mL), washed with saturated brine (10 mL), dried over anhydrous sodium

sulfate, concentrated, and purified by column chromatography (petroleum ether:ethyl acetate = 3:1) to give the pale yellow target product (1.8 g, 73% yield).

LC-MS(m/z): 273 $[M+H]^+$.

**Step C: synthesis of 2-(4-(pyridin-2-yl)-1,9-dioxaspiro[5.5]undecan-4-yl)ethan-1-amine**

2-(4-(Pyridin-2-yl)-1,9-dioxaspiro[5.5]undecan-4-yl)acetonitrile (1.8 g, 6.6 mmol) was dissolved in tetrahydrofuran (15 mL), lithium aluminum hydride (750 mg, 19.8 mmol) was added under an ice bath, and the mixture was reacted at room temperature for 2 h. After the reaction was completed, water (0.75 mL), a 15% sodium hydroxide solution (2 mL), water (2 mL), and ethyl acetate (10 mL) were added sequentially, and the mixture was dried over anhydrous sodium sulfate and concentrated to dryness under reduced pressure to give the oily target product (1.5 g, 85% yield).

LC-MS(m/z): 277 $[M+H]^+$.

**Step D: synthesis of *N*-(2-(4-(pyridin-2-yl)-1,9-dioxaspiro[5.5]undecan-4-yl)ethyl)-2,3-dihydro-1*H*-inden-1-amine**

2-(4-(Pyridin-2-yl)-1,9-dioxaspiro[5.5]undecan-4-yl)ethan-1-amine (100 mg, 0.36 mmol) and 1-indanone (50 mg, 0.36 mmol) were dissolved in tetraisopropyl titanate (1 mL), and the mixture was reacted at 80 °C for 5 h and cooled to room temperature. Methanol (1 mL) was added, sodium borohydride (38 mg, 1.0 mmol) was added, and the mixture was reacted for 1 h. After the reaction was completed as detected by LC-MS, the mixture was filtered, concentrated, and purified by preparative liquid chromatography to give the target compound **15** (25.3 mg, 18% yield).

LC-MS(m/z): 393 $[M+H]^+$.

$^1H$ NMR (600 MHz, DMSO-$d_6$)δ8.59 (s, 1H), 7.82-7.80 (m, 2H), 7.57 (dd, *J* = 7.8, 3.0 Hz, 1H), 7.39-7.30 (m, 2H), 7.29 (dd, *J* = 7.2, 4.8 Hz, 1H), 7.25-7.23 (m, 1H), 4.65-4.61 (m, 1H), 4.48-4.45 (m, 1H), 3.77-3.64 (m, 1H), 3.64-3.44 (m, 3H), 3.32-3.13 (m, 2H), 3.01-2.97 (m, 1H), 2.94-2.76 (m, 2H), 2.62-2.54 (m, 2H), 2.34-2.25(m, 2H), 2.05-1.91 (m, 2H), 1.80-1.74 (m, 1H), 1.62-1.41 (m, 4H), 0.88-0.75 (m, 2H).

**Example 20**

**4-chloro-*N*-(2-(4-(pyridin-2-yl)-1,9-dioxaspiro[5.5]undecan-4-yl)ethyl)-2,3-dihydro-1*H*-inden-1-amine 16**

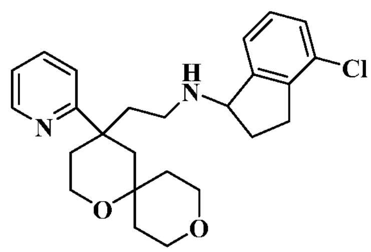

16

The starting material 1-indanone was replaced by 4-chloro-1-indanone to prepare the target compound **16** according to the synthesis method of Example 16.

LC-MS(m/z): 428 $[M+H]^+$.

$^1H$ NMR (600 MHz, DMSO-$d_6$) δ8.59-8.58 (m, 1H), 8.04-7.73 (m, 1H), 7.56 (dd, *J* = 7.8, 4.2 Hz, 1H), 7.44 (d, *J* = 7.2 Hz, 1H), 7.38-7.27 (m, 3H), 4.80-4.56 (m, 1H), 4.49-4.46 (m, 1H), 3.72-3.70 (m, 1H), 3.63-3.41 (m, 3H), 3.35-3.11 (m, 2H), 3.02-2.97 (m, 1H), 2.94-2.74 (m, 2H),2.61-2.54 (m, 2H), 2.39-2.25 (m, 2H), 2.07-1.95 (m, 2H), 1.76-1.73 (m, 1H), 1.66-1.46 (m, 4H),0.82-0.80 (m, 2H).

**Example 21**

**4-chloro-*N*-(2-(4-(5-fluoropyridin-2-yl)-1,9-dioxaspiro [5.5] undecan-4-yl)ethyl)-2,3-dihydro-1*H*-inden-2-amine 17**

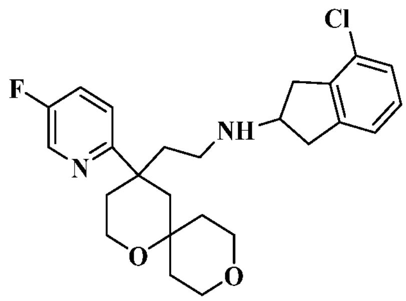

**17**

The starting material 6-chloro-2,3-dihydro-1*H*-inden-1-one was replaced by 4-chloro-1,3-dihydro-2*H*-inden-2-one to prepare the target compound **17** according to the synthesis method of Example 4.

LC-MS(m/z): 447 $[M+H]^+$.

$^1H$ NMR (600 MHz, MeOD) δ 8.50 (t, J = 3.1 Hz, 1H), 7.63 - 7.58 (m, 2H), 7.21 (ddd, J = 15.5, 7.8, 4.3 Hz, 3H), 4.04 - 3.95 (m, 1H), 3.81 - 3.70 (m, 3H), 3.66 - 3.59 (m, 1H), 3.38 (ddt, J = 11.0, 7.2, 2.6 Hz, 4H), 3.10 - 3.02 (m, 1H), 2.97 (ddd, J = 19.4, 17.4, 5.4 Hz, 2H), 2.64 (dd, J = 28.9, 13.9 Hz, 2H), 2.44 (dtd, J = 16.9, 12.5, 4.5 Hz, 1H), 2.10 - 2.02 (m, 1H), 1.81 (td, J = 12.8, 4.7 Hz, 1H), 1.76 - 1.66 (m, 2H), 1.57 (d, J = 13.8 Hz, 1H), 1.47 (d, J = 13.8 Hz, 1H), 1.31 (dd, J = 15.3, 8.0 Hz, 1H), 0.95 (d, J = 2.5 Hz, 2H).

**Example 22**

**(*S*)-4-chloro-*N*-(2-((*R*)-4-(5-fluoropyridin-2-yl)-1,9-dioxaspiro[5.5]undecan-4-yl)ethyl)-2,3-dihydro-1*H*-inden-2-amine 17-1-1 and (*R*)-4-chloro-*N*-(2-((*R*)-4-(5-fluoropyridin-2-yl)-1,9-dioxaspiro[5.5]undecan-4-yl)ethyl)-2,3-dihydro-1*H*-inden-2-amine 17-1-2**

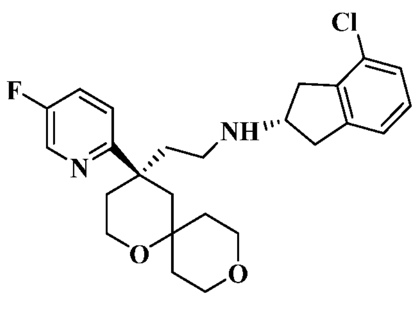

**17-1-1**

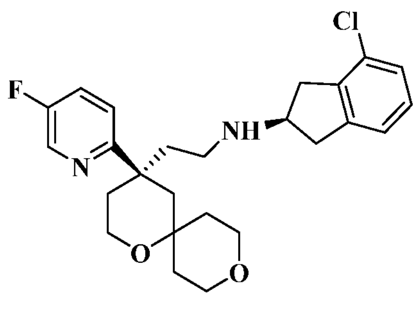

**17-1-2**

Synthesis scheme:

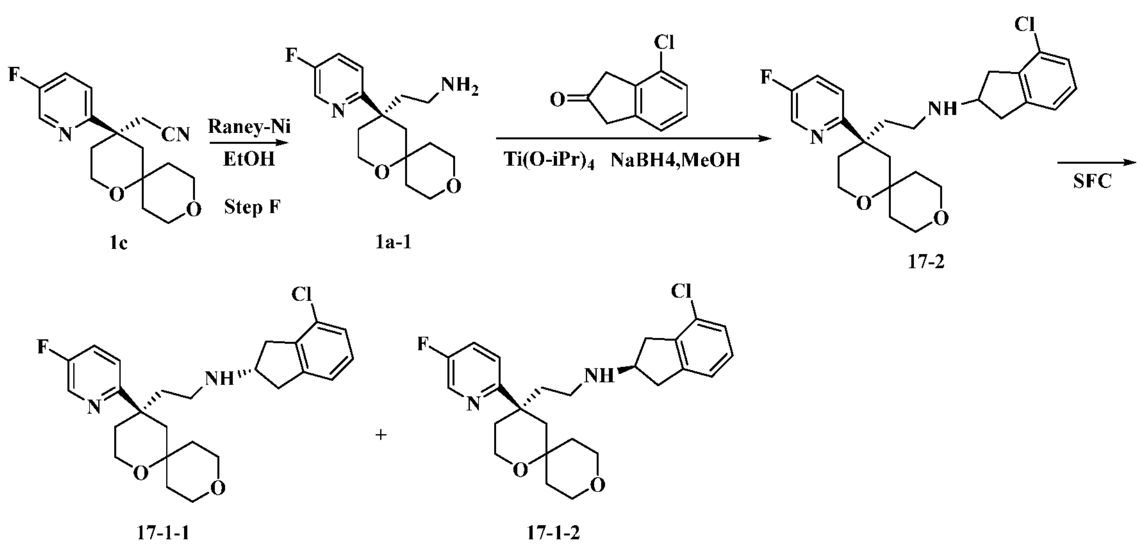


1c 1a-1 17-2

17-1-1 + 17-1-2

The starting material 1,3-dihydro-2*H*-inden-2-one was replaced by 4-chloro-1,3-dihydro-2*H*-inden-2-one to prepare the target compound **17-2** according to the synthesis method of Example 9.

Compound **17-2** was separated to give two optical isomers:

(*S*)-4-chloro-*N*-(2-((*R*)-4-(5-fluoropyridin-2-yl)-1,9-dioxaspiro[5.5]undecan-4-yl)ethyl)-2,3-dihydro-1*H*-inden-2-amine **17-1-1,** 2.10 min, ee% = 97%;

LC-MS(m/z): 445 $[M+H]^+$.

$^1$H NMR (600 MHz, DMSO) δ 8.56 (s, 1H), 7.74 (d, *J* = 16.6 Hz, 1H), 7.62 (s, 1H), 7.18 (d, *J* = 36.2 Hz, 4H), 3.83 - 3.76 (m, 1H), 3.70 (d, *J* = 11.9 Hz, 1H), 3.60 - 3.51 (m, 4H), 3.21 (d, *J* = 11.3 Hz, 1H), 3.12 (dd, *J* = 16.1, 7.8 Hz, 1H), 2.94 - 2.83 (m, 2H), 2.76 (dd, *J* = 11.8, 8.6 Hz, 1H), 2.54 (d, *J* = 13.9 Hz, 2H), 2.21 (dd, *J* = 11.7, 8.1 Hz, 1H), 1.96 (td, *J* = 12.5, 4.1 Hz, 1H), 1.91 (s, 1H), 1.77 (dt, *J* = 12.3, 6.2 Hz, 1H), 1.64 - 1.53 (m, 2H), 1.48 (d, *J* = 14.0 Hz, 1H), 1.41 (d, *J* = 13.5 Hz, 1H), 0.81 (s, 2H).

(*R*)-4-chloro-*N*-(2-((*R*)-4-(5-fluoropyridin-2-yl)-1,9-dioxaspiro[5.5]undecan-4-yl)ethyl)-2,3-dihydro-1*H*-inden-2-amine **17-1-2,** 3.1 min, ee% = 97%;

LC-MS(m/z): 445 $[M+H]^+$.

$^1$HNMR (600 MHz, DMSO) δ 8.55 (s, 1H), 7.71 (td, *J* = 8.7, 2.5 Hz, 1H), 7.62 (dd, *J* = 8.8, 4.1 Hz, 1H), 7.19 (dt, *J* = 16.8, 7.5 Hz, 3H), 3.74 - 3.66 (m, 2H), 3.61 - 3.49 (m, 4H), 3.22 (d, *J* = 6.1 Hz, 1H), 3.15 (dd, *J* = 16.4, 7.5 Hz, 1H), 3.09 (dd, *J* = 16.5, 7.6 Hz, 1H), 2.88 - 2.76 (m, 2H), 2.67 (s, 1H), 2.55 (d, *J* = 14.3 Hz, 2H), 2.08 (d, *J* = 14.0 Hz, 1H), 2.08 (d, *J* = 14.0 Hz, 1H), 1.90 (t, *J* = 10.4 Hz, 1H), 1.69 (t, *J* = 10.3 Hz, 1H), 1.62 - 1.52 (m, 2H), 1.48 (d, *J* = 13.8 Hz, 1H), 1.40 (d, *J* = 13.2 Hz, 1H), 0.80 (s, 2H).

Separation conditions: instrument: Waters SFC 150; column: DAICELCHIRALCEL®OZ, 250 × 25 mm 10 μm; mobile phase: A: supercritical $CO_2$, B: MeOH (0.1% ammonia in MeOH); gradient: A:B = 50:50; flow rate: 70 mL/min; back pressure: 100 bar; column temperature: 25 °C; wavelength: 214 nm; cycle: 6 min; the compound to be separated was dissolved in MeOH (40 mL); injection: 1.8 mL.

**Example 23**

**(*R*)-*N*-(2-chlorobenzyl)-2-(4-(5-fluoropyridin-2-yl)-1,9-dioxaspiro[5.5]undecan-4-yl)ethan-1-amine 18-1**

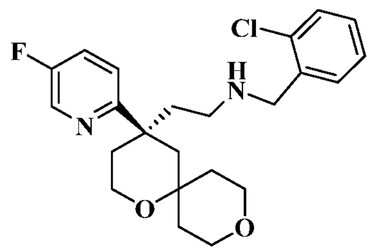

18-1

The starting material 1,3-dihydro-2*H*-inden-2-one was replaced by 2-chlorobenzaldehyde to prepare the target compound **18-1** according to the synthesis method of Example 9.

LC-MS(m/z): 419 $[M+H]^+$.

$^1H$ NMR (600 MHz, DMSO) δ 9.30 (d, J = 57.3 Hz, 1H), 8.55 (d, J = 3.0 Hz, 1H), 7.73 (td, J = 8.8, 3.0 Hz, 1H), 7.67 - 7.61 (m, 2H), 7.51 (dd, J = 7.8, 1.2 Hz, 1H), 7.40 (dtd, J = 19.3, 7.4, 1.5 Hz, 2H), 4.12 (t, J = 6.0 Hz, 2H), 3.75 - 3.67 (m, 1H), 3.54 (ddd, J = 15.4, 14.9, 7.9 Hz, 3H), 3.30 - 3.18 (m, 2H), 2.89 - 2.78 (m, 1H), 2.54 (d, J = 13.2 Hz, 2H), 2.34 - 2.24 (m, 1H), 2.06 (td, J = 12.8, 4.5 Hz, 1H), 1.88 (td, J = 12.7, 4.2 Hz, 1H), 1.62 - 1.53 (m, 2H), 1.48 (d, J = 13.9 Hz, 1H), 1.42 (d, J = 13.5 Hz, 1H), 0.86 - 0.77 (m, 2H).

**Example 24**

**(*R*)-*N*-(2-(difluoromethoxy)benzyl)-2-(4-(5-fluoropyridin-2-yl)-1,9-dioxaspiro[5.5]undecan-4-yl)ethan-1-amine 19-1**

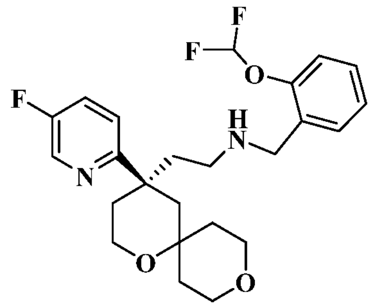

19-1

The starting material 1,3-dihydro-2*H*-inden-2-one was replaced by 2-difluoromethoxybenzaldehyde to prepare the target compound **19-1** according to the synthesis method of Example 9.

LC-MS(m/z): 451 $[M+H]^+$.

$^1H$ NMR (600 MHz, DMSO) δ 8.53 (d, *J* = 2.9 Hz, 1H), 7.72 (d, *J* = 2.9 Hz, 1H), 7.66 - 7.57 (m, 2H), 7.44 (s, 1H), 7.38 - 7.07 (m, 3H), 3.97 (t, *J* = 5.8 Hz, 2H), 3.76 - 3.62 (m, 1H), 3.53 (dd, *J* = 16.1, 3.7 Hz, 3H), 3.22 (dd, *J* = 10.8, 9.0 Hz, 2H), 2.75 (dd, *J* = 8.0, 4.0 Hz, 1H), 2.52 (d, *J* = 14.9 Hz, 2H), 2.48 (s, 1H), 2.28 - 2.15 (m, 1H), 2.11 - 2.01 (m, 1H), 1.89 (d, *J* = 4.1 Hz, 1H), 1.55 (ddd, *J* = 13.3, 8.3, 3.7 Hz, 2H), 1.46 (d, *J* = 13.8 Hz, 2H), 0.80 (s, 2H).

**Evaluation of biological activity**

The present invention is further described below using test examples, but these examples are not intended to limit the scope of the present invention. The structure and source of oxycodone in the test examples are shown in Table 1 below:

Table 1

| Name | Structural formula | Source |
|---|---|---|
| | | |

(continued)

| Name | Structural formula | Source |
|---|---|---|
| Oxycodone | 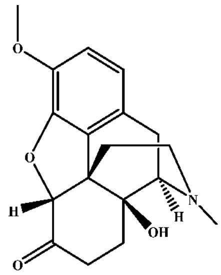 | Provided by Jiangsu Nhwa Pharmaceutical Co., Ltd. |

**Test Example 1**

1.1 Objective

The objective of this experiment was to test the agonistic effect of the compound of the present invention on MOR, and to evaluate the *in vitro* activity of the compound according to $EC_{50}$ and Emax.

1.2 Experimental materials are shown below

Table 2

| Cell | Growth medium | Cryopreserved medium |
|---|---|---|
| CHO-K1/OP3 | Ham's F-12, 10% FBS, 0.4 mg/ml G418 | 45% culture medium, 45% FBS, 10% DMSO |

Table 3

| Reagent | Brand | Cat. No. | Specification |
|---|---|---|---|
| cAMP Assay Kit | Cisbio | 62AM9PEJ | 100000 tests |
| IBMX | Sigma | 15879 | 1 g |
| HBSS | Gibco | 14175103 | 10×500 mL |
| Forskolin | Sigma | F6886 | 50 mg |
| DMSO | Sigma | D8418 | 500 mL |

1.3 Experimental procedures

1.3.1 Reagent preparation

1.3.1.1 Preparation of experimental buffer

5× stimulation buffer was diluted to 1× with $ddH_2O$, an appropriate amount of 500 mM IBMX (generally, the IBMX stock solution was prepared to be 500 mM, namely 1000X) was added to enable the final concentration of the IBMX to be 0.5 mM, and the mixture was mixed well for later use (after the IBMX was added, that precipitation occurred was normal, and the precipitate was dissolved by well mixing). It must be prepared right before use.

1.3.1.2 IBMX was dissolved with DMSO to prepare a 500 mM stock solution, which was aliquoted into 20 μL/tube and cryopreserved at -80 °C to avoid repetition of freezing and thawing.

1.3.1.3 Forskolin was dissolved with DMSO to prepare a 10 mM stock solution, which was aliquoted into 20 μL/tube and cryopreserved at -20 °C for later use.

1.3.1.4 cAMP standard substance preparation

The cAMP standard substance in the kit returned to room temperature, and an equivalent amount of the experimental buffer was added according to the volume marked on the bottle label. The mixture was vortexed for mixing, aliquoted according to 50 μL/tube, and cryopreserved at -20 °C for later use.

1.3.1.5 Preparation of detection reagent stock solution (20000 tests packaging)

5 mL $ddH_2O$ was added to a bottle of cAMP-d2 and a bottle of anti-cAMP-cryptate lyophilized powder, respectively, and the mixture was mixed well by gently turning the mixture upside down, aliquoted according to 125, 62.5, and 30 μL, and the like, and cryopreserved in the dark at - 80 °C.

1.3.2 Experimental procedures of agonist test

1.3.2.1 Positive compound DAMGO and the test compound were serially diluted 4-fold to obtain 10 concentrations with Bravo using experimental buffer in a compound plate (Greiner-781280) at a starting concentration of 2 μM (from DMSO stock solution diluted with experimental buffer) and 20 μM (from DMSO stock solution diluted with experimental buffer), respectively.

1.3.2.2 Cells cryopreserved were thawed in a water bath at 37 °C, the cell suspension was added to a centrifuge tube containing 10 mL of HBSS buffer, and the mixture was centrifuged at 750 rpm for 5 min. The supernatant was discarded, the pellet was resuspended in an appropriate amount of experimental buffer, and 20 μL of the pellet was counted using a cell counter. An appropriate amount of the cell suspension was diluted to $0.4 \times 10^6$ cells/mL, and 5 μL of the cell suspension was added to each well of a cell plate (the cell density was 2000 cells/well) and centrifuged at 1000 rpm for 1 min. 5 μL of the diluted compound was transferred to a cell plate (PerkinElmer-6008280) with Bravo, 5 μL of 2 μM DAMGO (the final concentration was 1 μM) was transferred to positive control wells, and an equal volume of experimental buffer was transferred to negative control wells, and the cell plate was centrifuged at 1000 rpm for 1 min. The cell plate was sealed and incubated at room temperature for 15 min. Forskolin was diluted to 0.2 mM with DMSO, 25.1 nL of which was transferred to a cell plate with Tecan-D300e, and the plate was centrifuged at 1000 rpm for 1 min, sealed, and incubated at room temperature for 45 min. Forskolin had a final concentration of 1 μM.

1.3.2.3 Preparation of cAMP (the stock solution had a concentration of 2848 nM) standard curve: the stock solution was serially diluted 4-fold to obtain 8 concentrations from a starting concentration of 1424 nM, 10 μL of which was added to a cell plate. The final concentration of the initial point was 712 nM.

1.3.2.4 Preparation of detection reagent: an appropriate amount of a cAMP-d2 stock solution and an anti-cAMP-cryptate stock solution was diluted with a lysis buffer according to the ratio of 1:20, the two solutions were then mixed well by turning the solutions upside down according to the ratio of 1:1, and vortexing was prohibited. 10 μL of the prepared detection reagent was added to a cell plate and centrifuged at 1000 rpm for 1 min. The cell plate was incubated at room temperature for 1 h in the dark. After the cell plate was centrifuged at 1000 rpm for 1 min, the plate was read using Envision. Excitation light at 340 nm, emission light at 620 nm and 665 nm.

1.3.3 Data analysis

The formula for calculating the compound activation rate in the agonist test:

$$\text{Activity\%} = 100 - (\text{Readout} - \text{LC}) / (\text{HC-LC}) \times 100$$

HC (high control): the average value of readings from DMSO + 2.5 μM Forskolin wells
LC (low control): the average value of readings from 1 μM Dopamine + 2.5 μM Forskolin wells
Readout: the reading of the compound

1.3.4 Test results

Changes in the effect of the compound of the present invention on the downstream cAMP level by agonizing MOR were determined by the above experiment, and the $EC_{50}$ measured is shown in Table 4 below (maximal effect of DAMGO is 100%).

Table 4. $EC_{50}$ and Emax of the compound of the present invention agonizing MOR receptor to affect cAMP level

| Example No. | $EC_{50}$ (nM) | Emax |
|---|---|---|
| Compound 1-1 | 0.44 | 103.2% |
| Compound 2-1 | 23.5 | 101.4% |
| Compound 3-1 | 0.79 | 99.9% |
| Compound 4 | 2.19 | 102.8% |
| Compound 5-1 | 1.33 | 111% |
| Compound 6-1 | 1.56 | 118% |
| Compound 7-1 | 1.69 | 116% |
| Compound 8 | 8.62 | 72.0% |
| Compound 8-1 | 4.1 | 92.8% |
| Compound 10 | 2.79 | 80.9% |
| Compound 10-1-1 | 0.8 | 104% |
| Compound 11 | 7.75 | 100.3% |
| Compound 12 | 7.63 | 89.8% |
| Compound 12-1 | 1.29 | 93.8% |
| Compound 13 | 17.3 | 78.9% |
| Compound 13-1 | 18.7 | 78.6% |
| Compound 14 | 24.2 | 88.1% |
| Compound 15 | 30.7 | 96.9% |
| Compound 16 | 2.40 | 101.6% |
| Compound 17 | 2.14 | 93.8% |
| Compound 17-1-1 | 0.64 | 101.8% |
| oxycodone | 64.7 | 95.2% |

**Conclusion**: the results described above show that the compound of the present invention has relatively strong agonistic activity on MOR; the compound provided by the present invention can reduce the administration dose while maintaining the analgesic effect, thereby reducing the side effects in the process of clinical use, so that the compound of the present invention has broad prospects in analgesic clinical use.

**Test Example 2**

2.1 Objective

The experiment was conducted to test the agonistic effect of the compound on MOR beta-arrestin, and to evaluate the *in vitro* activity of the compound according to $EC_{50}$ and Emax.

2.2 Experimental consumables, instruments, and equipment are shown below

Table 5

| Consumables, instruments, and equipment | Supplier | Cat. No. |
|---|---|---|
| 384-well cell plate | Coming | #3570 |
| 384-well Echo compound plate | Labcyte | #LP-0200 |
| 384-well compound plate | PE | #6008590 |
| Echo 550 | Labcyte | / |

(continued)

| Consumables, instruments, and equipment | Supplier | Cat. No. |
|---|---|---|
| Bravo | Agilent | / |
| Envision Plate Reader | PerkinElmer | / |

2.3 Experimental reagents are shown below

Table 6

| Reagent | Supplier | Cat. No. |
|---|---|---|
| Human Mu opioid β-arrestin U2OS/OPRM1 cell line | DiscoveRX | #93-0213C3 |
| MEM | Gibco | #11095-080 |
| FBS | Invitrogen | #10099-141 |
| 0.25% Trpysin/EDTA | Invitrogen | #25200-072 |
| Hygromycin B | Invitrogen | #10687-010 |
| GlutaMAX | Invitrogen | #35050-079 |
| DPBS | Invitrogen | #14200-075 |
| DAMGO | Tocris | #1171 |
| Path Hunter Detection Kit | DiscoveRX | #93-0001L |
| Cell complete medium: MEM + 10% FBS + 1% PS + 250 μg/mL Hygromycin B + 500 μg/mL G418 + 1X GlutaMAX<br>Cell inoculation medium: MEM + 10% FBS + 1% PS<br>Experimental buffer: 1× DPBS + 0.1% BSA<br>Detection reagent: Galacton Star:Emerald II:PathHunter cell assay buffer = 1:5:19 | | |

2.4 Experimental methods

2.4.1 Preparation of compounds

i) Compound samples were dissolved in DMSO to a stock concentration of 10 mM;
ii) A sample dilution sequence on a 384-well LDV plate was prepared, wherein the first concentration point of the sample was 10 mM (FAC = 30 μM), and the sample was serially diluted 3.162-fold to obtain a total of 11 concentration points;
iii) The sample dilution sequence and HPE and ZPE were transferred to a compound plate (PE #6008590) using an Echo machine, with each well 90 nL transferred.

2.4.2 Experimental procedures

i) The MOR beta-arrestin cell strain was cultured in a cell complete medium at 37 °C with 5% $CO_2$ to a confluence of 70-90%;
ii) the cells were digested and resuspended in the cell inoculation medium, and 20 μL of the MOR beta-arrestin cell suspension was added to each well of an assay plate (Corning #3570) at 7500 cells/well and incubated in an incubator at 37 °C with 5% $CO_2$ for 24 h;
iii) 30 μL of an experimental buffer was added to a compound plate (PE #6008590), and the plate was centrifuged at 1000 rpm for 5 min;
iv) after 24 h, the assay plate was taken out, the medium was removed from the plate, and then 20 μL of the compound was transferred from each corresponding well of the compound plate to the assay plate with Bravo;
v) the assay plate was centrifuged at 500 rpm for 30 s and then placed into an incubator for incubation at 37 °C with

5% $CO_2$ for 90 min;
vi) 10 μL of the beta-arrestin detection reagent was added to each well of the assay plate;
vii) after 1 h of incubation at room temperature in the dark, the plate was read on Envision.

2.5 Data processing

The experimental data of the percent activation rate and concentrations of 11 points were fitted using XLFit to a parameter nonlinear logic
Use formula to calculate the $EC_{50}$ value of the compound, and the results are shown in Table 7 (maximal effect of DAMGO is 100%).

Table 7. Results of activity of compound of the present invention for activating beta-arrestin signaling pathway

| Example No. | $EC_{50}$ (nM) | Emax |
|---|---|---|
| Compound 8 | 364 | 2.1% |
| Compound 8-1 | >30000 | 3.0% |
| Compound 10 | 4801 | 4.5% |
| Compound 12 | >30000 | 6.3% |
| Compound 13-1 | >30000 | 4.2% |
| Compound 15 | 1784 | 7.2% |

**Conclusion**: the results described above show that the compound of the present invention has almost no activation effect on the beta-arrestin signaling pathway; the compound provided by the present invention has no obvious side effects, and is safer and more effective in the process of clinical use.

**Test Example 3**

3.1 Objective

The experiment was to measure the effects of each compound sample on the pain threshold of a mouse by using a mouse thermal radiation tail-flick method.

3.2 Experimental reagents are shown below

Table 8

| Reagent | Supplier | Cat. No. | Specification |
|---|---|---|---|
| Methylcellulose | Sigma | SLCH2339 | 250 G |
| DMSO | Sinopharm Chemical Reagent Co., Ltd. | 30072418 | 500 mL |

3.3 Experimental methods

3.3.1 Dose and route of administration:

Intragastric administration at a dose of 10 mg/kg;

3.3.2 Experimental procedures

ICR mice (normal grade, weighed 25-35 g, purchased from Shanghai Slac Laboratory Animal Co., Ltd.) were first mounted in a special plastic cylinder, their tails were exposed and allowed to droop naturally, and the measurements were made after the animals were calm. The radiation heat source could be an 8.75 mm projection lamp (32 W, adjustable). A light beam with the diameter of about 4 mm was emitted after being focused by a lens, which irradiated the skin at the

junction of the middle third and the lower third of the tail (the light source and the skin at the tail must be closely attached); an electronic timer connected with the light source in parallel was used to synchronously record the irradiation duration, that is, when the irradiation started, a stopwatch was automatically started at the same time. When the tail of the animal obviously evaded, the timing was automatically stopped. The measured time interval was the tail flick latency. Mice with tail flick latency of 2-6 seconds were screened to participate in a grouping administration experiment. If the animal had an analgesic effect for more than 15 seconds, the irradiation was stopped, and 15 seconds were used as the upper limit of the tail flick latency so as to prevent the skin from being burnt by the irradiation for too long. After a dose of a drug was administered to the mice, the pain threshold of the mice was measured at different times.

3.4 Detection index and statistical method:

After a dose of a drug was administered to the mice, the pain threshold of the mice was measured at 0.5 h, 1 h, 2 h, and 3 h (effect view of the analgesic effect is shown in FIG. 1). The formula for calculating the maximum analgesic percentage (%MPE) after administration was as follows: %MPE = (post-administration pain threshold - pre-administration pain threshold)/(15 - pre-administration pain threshold) × 100%.

Table 9. Maximum analgesic percentage (%MPE, Mean ± SEM, n = 9-10) at different times after administration of each compound

| Example No. | 0.5h | 1h | 2h | 3h |
|---|---|---|---|---|
| Compound 8-1 | 80.64±9.27** | 85.69±5.95*** | 69.94±10.43***### | 87.16±6.80***### |
| Compound 12-1 | 99.48±0.52*** | 86.20±9.32*** | 89.98±10.02***### | 78.89±10.05***### |
| Oxycodone | 37.87±12.44 | 28.70±9.24 | 3.63±8.14 | -0.23±6.48 |
| Note: compared to oxycodone, *p < 0.05, **p < 0.01, ***p < 0.001. | | | | |

**Conclusion**: the results described above show that the compounds of the present invention, especially compound 8-1 and compound 12-1, have better drug effect than oxycodone at the same dose, wherein compared with oxycodone, compound 8-1 and compound 12-1 have an analgesic effect that is maintained for a longer period of time, and the maintenance of the analgesic effect has statistical advantages.

Although specific embodiments of the present invention have been described in detail, various modifications and substitutions can be made to the details of the technical solutions of the present invention by those skilled in the art according to all the teachings that have been disclosed, and these changes are all encompassed within the protection scope of the present invention. The full scope of the present invention is given by the appended claims and any equivalent thereof.

## Claims

1. A compound of general formula (I), a stereoisomer thereof, a tautomer thereof, or a pharmaceutically acceptable salt thereof,

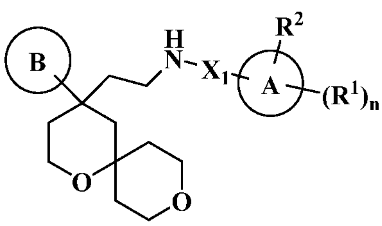

(I)

wherein:

ring A is $C_{6-10}$ aryl or 5- to 6-membered heteroaryl, wherein the 5- to 6-membered heteroaryl is 5- to 6-membered heteroaryl containing 1-3 heteroatoms selected from a nitrogen atom, an oxygen atom, and a sulfur atom;

$R^1$ are the same or different, and $R^1$ and $R^2$ are each independently hydrogen, halogen, hydroxy, amino, nitro, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl is optionally further substituted with one or more substituents selected from $C_{3-6}$ cycloalkyl, $C_{1-6}$ alkyl, and halogen;
ring B is phenyl or pyridinyl, optionally further substituted with 1-4 $R^3$;
$R^3$ are the same or different and are each independently hydrogen, halogen, hydroxy, amino, nitro, cyano, $C_{1-6}$ alkyl, $C_{1-6}$ haloalkyl, $C_{1-6}$ alkoxy, or $C_{1-6}$ haloalkoxy;
$X_1$ is $CR^aR^b$ or $CR^aR^bCH_2$;
$R^a$ and $R^b$ are each independently hydrogen, halogen, or $C_{1-3}$ alkyl;
or $R^a$ or $R^b$ is linked to $R^2$ to form one cyclopentyl or cyclohexyl and is optionally further substituted with one or more substituents selected from fluorine, chlorine, bromine, methyl, ethyl, propyl, methoxy, ethoxy, halomethyl, haloethyl, halomethoxy, and haloethoxy;
n is 0, 1, 2, or 3.

**2.** The compound, the stereoisomer thereof, the tautomer thereof, or the pharmaceutically acceptable salt thereof according to claim 1, wherein one or more of the following conditions are met:

(1) ring A is phenyl;
(2) $X_1$ is $CR^aR^b$ or $CR^aR^bCH_2$, wherein $R^a$ and $R^b$ are each independently hydrogen, halogen, or $C_{1-3}$ alkyl, preferably hydrogen; or $R^a$ or $R^b$ is linked to $R^2$ to form one cyclopentyl or cyclohexyl, preferably cyclopentyl;
(3) ring B is pyridinyl and is optionally further substituted with one $R^3$, preferably

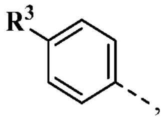

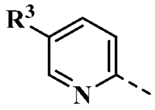 , or 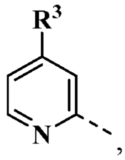

more preferably

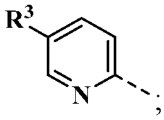

(4) $R^1$ and $R^2$ are each independently hydrogen, halogen, $C_{1-6}$ alkyl, or $C_{1-6}$ haloalkoxy, preferably hydrogen, halogen, $C_{1-3}$ alkyl, or $C_{1-3}$ haloalkoxy, more preferably hydrogen, fluorine, chlorine, bromine, methyl, ethyl, propyl, or halomethoxy;
(5) $R^3$ is hydrogen or halogen, preferably hydrogen, fluorine, chlorine, or bromine, more preferably hydrogen or fluorine;
(6) n is 0, 1, or 2.

**3.** The compound, the stereoisomer thereof, the tautomer thereof, or the pharmaceutically acceptable salt thereof according to claim 2, wherein one or more of the following conditions are met:

(1) $X_1$ is $CR^aR^b$, preferably $CH_2$;
(2) ring B is

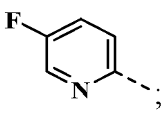

(3) $R^1$ is hydrogen, halogen, $C_{1-6}$ alkyl, $C_{1-6}$ alkoxy, or $C_{1-6}$ haloalkoxy, preferably hydrogen, halogen, $C_{1-3}$ alkyl, or $C_{1-3}$ haloalkoxy, more preferably hydrogen, fluorine, chlorine, bromine, methyl, ethyl, propyl, or halomethoxy, further preferably hydrogen, fluorine, chlorine, methyl, or fluoromethoxy;
(4) $R^2$ is hydrogen, halogen, $C_{1-3}$ alkyl, or $C_{1-3}$ haloalkoxy, preferably hydrogen.

**4.** The compound, the stereoisomer thereof, the tautomer thereof, or the pharmaceutically acceptable salt thereof according to claim 2, wherein one or more of the following conditions are met:

(1) $X_1$ is $CH(R^b)$ or $CH(R^b)CH_2$, wherein $R^b$ is linked to $R^2$ to form one cyclopentyl or cyclohexyl, preferably cyclopentyl;
(2) ring B is

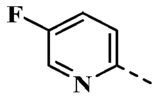 or 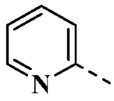,

preferably

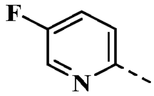;

(3) $R^1$ is independently hydrogen, halogen, or $C_{1-6}$ alkyl, preferably hydrogen, halogen, or $C_{1-3}$ alkyl, more preferably hydrogen, fluorine, chlorine, bromine, methyl, ethyl, or propyl, further preferably hydrogen, fluorine, chlorine, bromine, or methyl.

**5.** The compound, the stereoisomer thereof, the tautomer thereof, or the pharmaceutically acceptable salt thereof according to claim 1, wherein one or more of the following conditions are met:

(1) $R^1$ and $R^2$ are each independently hydrogen, halogen, hydroxy, $C_{1-3}$ alkyl, $C_{1-3}$ haloalkyl, $C_{1-3}$ alkoxy, or $C_{1-3}$ haloalkoxy, preferably hydrogen, fluorine, chlorine, bromine, hydroxy, methyl, ethyl, propyl, methoxy, ethoxy, halomethyl, haloethyl, or halomethoxy, more preferably hydrogen, fluorine, chlorine, bromine, methyl, methoxy, or halomethoxy, further preferably hydrogen, fluorine, chlorine, bromine, methyl, or methoxy;
(2) $R^3$ is each independently hydrogen, fluorine, chlorine, bromine, or $C_{1-3}$ alkyl, preferably hydrogen, fluorine, chlorine, or bromine;
(3) $X_1$ is $CH_2$, $CH(R^b)$, or $CH(R^b)CH_2$;
(4) $R^a$ or $R^b$ is linked to $R^2$ to form one cyclopentyl.

**6.** The compound, the stereoisomer thereof, the tautomer thereof, or the pharmaceutically acceptable salt thereof according to claim 1, wherein general formula (I) further has the structure represented by general formula (II):

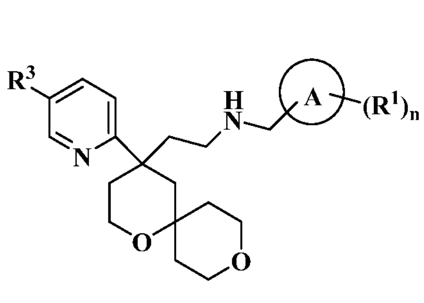

**(II)**

wherein ring A, $R^1$, $R^3$, and n are as described in claim 1;
general formula (I) further preferably has the structure represented by general formula (II-1):

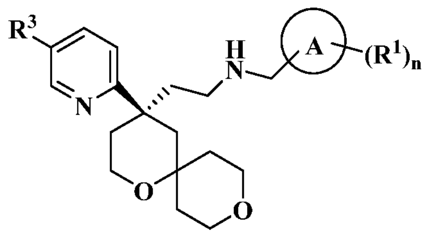

(II-1)

wherein ring A, $R^1$, $R^3$, and n are as described in claim 1.

**7.** The compound, the stereoisomer thereof, the tautomer thereof, or the pharmaceutically acceptable salt thereof according to claim 6, wherein

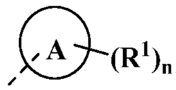

is

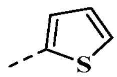,

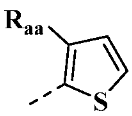, 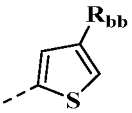, 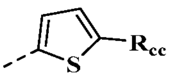, 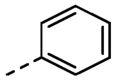, 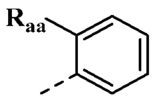, 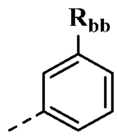, 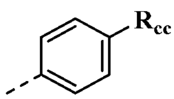,

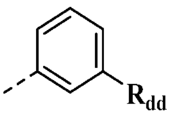, 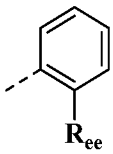, 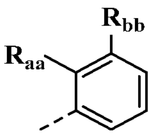, 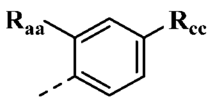, 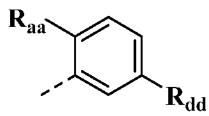, 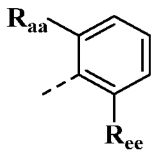,

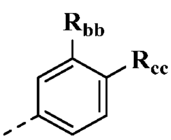, 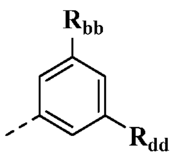, 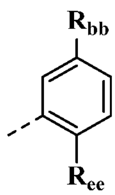, 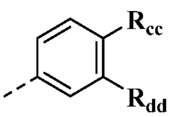, 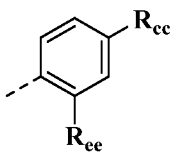, or 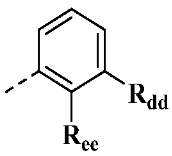;

preferably

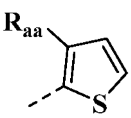, 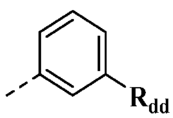, or 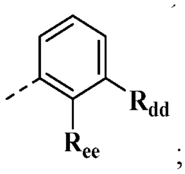;

wherein:

$R_{aa}$, $R_{bb}$, $R_{cc}$, $R_{dd}$, or $R_{ee}$ is each independently halogen, hydroxy, $C_{1-6}$ alkyl, $C_{1-6}$ haloalkyl, $C_{1-6}$ alkoxy, or $C_{1-6}$

haloalkoxy, wherein the $C_{1-6}$ alkyl is optionally further substituted with one or more substituents selected from $C_{3-6}$ cycloalkyl, $C_{1-6}$ alkyl, or halogen; preferably halogen, hydroxy, $C_{1-3}$ alkyl, $C_{1-3}$ haloalkyl, $C_{1-3}$ alkoxy, or $C_{1-3}$ haloalkoxy; more preferably fluorine, chlorine, bromine, hydroxy, methyl, ethyl, propyl, methoxy, ethoxy, halomethyl, haloethyl, or halomethoxy; further preferably fluorine, chlorine, bromine, methyl, methoxy, or halomethoxy; still further preferably fluorine, chlorine, bromine, methyl, or methoxy.

**8.** The compound, the stereoisomer thereof, the tautomer thereof, or the pharmaceutically acceptable salt thereof according to claim 7, wherein

$R_{aa}$, $R_{bb}$, $R_{cc}$, $R_{dd}$, and $R_{ee}$ are each independently halogen, $C_{1-6}$ alkyl, $C_{1-6}$ alkoxy, or $C_{1-6}$ haloalkoxy, preferably halogen, $C_{1-3}$ alkyl, or $C_{1-3}$ haloalkoxy, more preferably fluorine, chlorine, bromine, methyl, ethyl, propyl, or halomethoxy, further preferably fluorine, chlorine, bromine, methyl, or fluoromethoxy;
$R_{aa}$ is preferably halogen or $C_{1-6}$ haloalkoxy, more preferably halogen or $C_{1-3}$ haloalkoxy, further preferably fluorine, chlorine, bromine, or halomethoxy, still further preferably chlorine or fluoromethoxy;
$R_{dd}$ is preferably halogen, more preferably fluorine, chlorine, or bromine, further preferably chlorine;
$R_{ee}$ is preferably halogen or $C_{1-6}$ alkyl, more preferably halogen or $C_{1-3}$ alkyl, further preferably fluorine, chlorine, bromine, methyl, ethyl, or propyl, still further preferably fluorine, chlorine, or methyl.

**9.** The compound, the stereoisomer thereof, the tautomer thereof, or the pharmaceutically acceptable salt thereof according to any one of claims 6-8, wherein

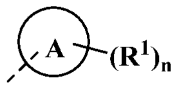

is

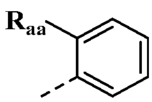 , 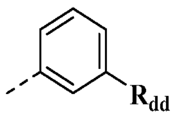 , or 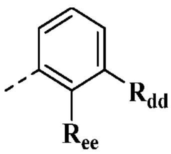 ,

preferably

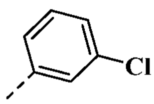 , 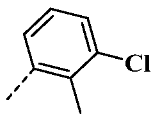 ,

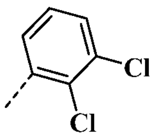 , 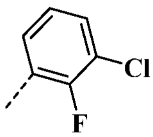 , 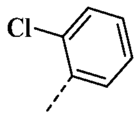 , or 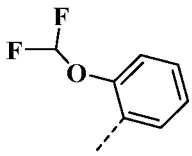 .

**10.** The compound, the stereoisomer thereof, the tautomer thereof, or the pharmaceutically acceptable salt thereof according to claim 1, wherein general formula (I) further has the structure represented by general formula (III):

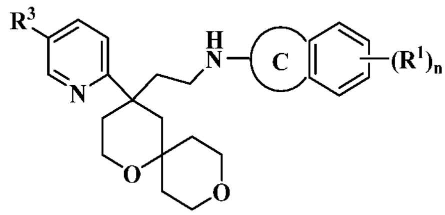

(III)

wherein ring C is cyclopentyl, and $R^1$, $R^3$, and n are as described in claim 1;
general formula (III) further preferably has the structure represented by general formula (IV) or general formula (V):

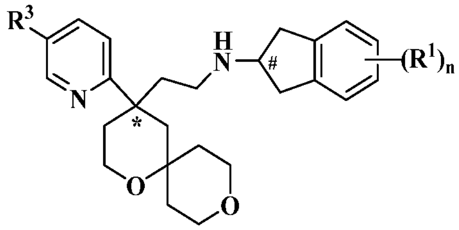

(IV)

or

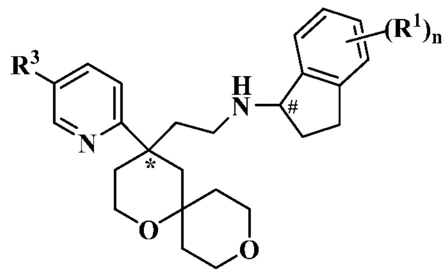

(V)

wherein $R^1$, $R^3$, and n are as described in claim 1; the carbon atom with "*" is a chiral carbon atom present in a form of a single (R) or (S) enantiomer or in a form enriched with a pair of enantiomers; the carbon atom with "#" is a chiral carbon atom present in a form of a single (R) or (S) enantiomer or in a form enriched with a pair of enantiomers;
general formula (III) even more preferably has the structure represented by general formula (IV-1), general formula (IV-2), general formula (V-l), or general formula (V-2):

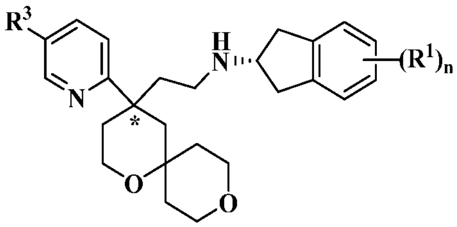

(IV-1)

,

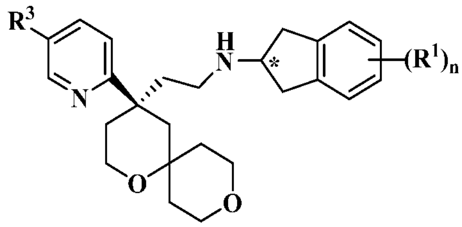

(IV-2)

,

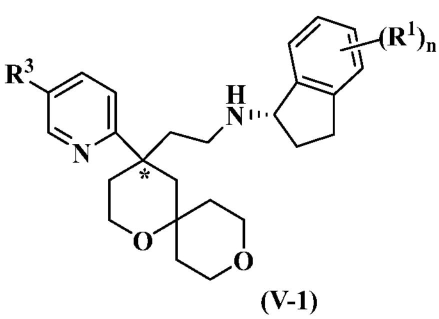

(V-1)

, or

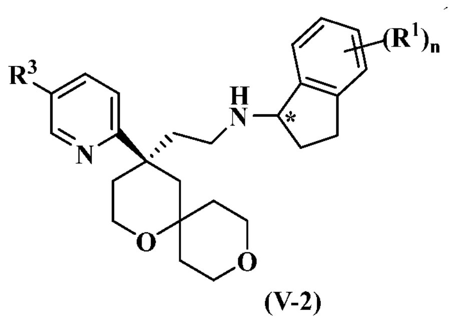

(V-2)

wherein $R^1$, $R^3$, and n are as described in claim 1; the carbon atom with "*" is a chiral carbon atom present in a form of a single (R) or (S) enantiomer or in a form enriched with a pair of enantiomers;
general formula (III) further particularly preferably has the structure represented by general formula (IV-1-1), general formula (IV-1-2), general formula (V-1-1), or general formula (V-1-2):

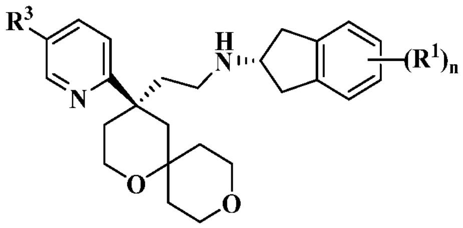

(IV-1-1) ,

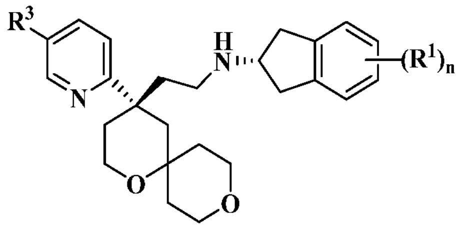

(IV-1-2) ,

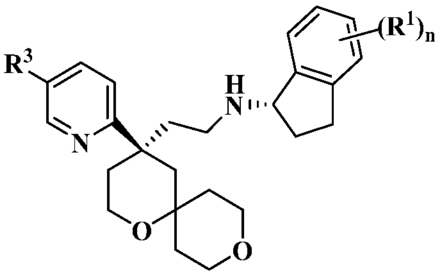

(V-1-1) , or

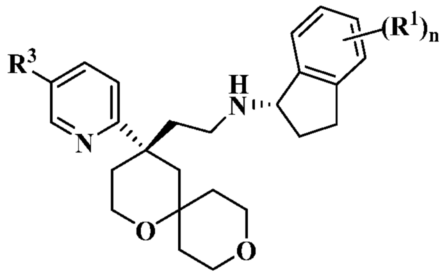

(V-1-2)

wherein $R^1$, $R^3$, and n are as described in claim 1.

**11.** The compound, the stereoisomer thereof, the tautomer thereof, or the pharmaceutically acceptable salt thereof according to claim 10, wherein

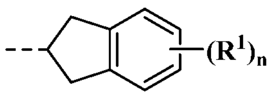

is

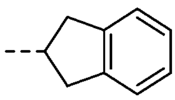 , 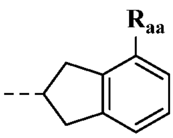 , 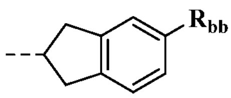 , 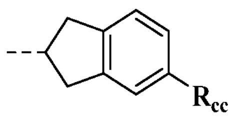 ,

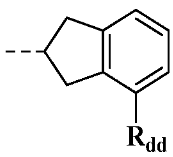 , 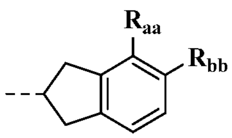 , 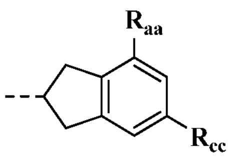 , 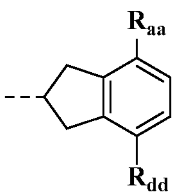 , 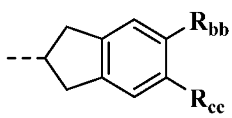 ,

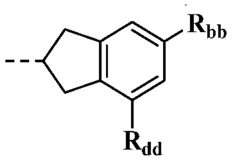 , or 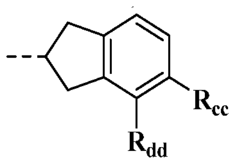 ,

preferably

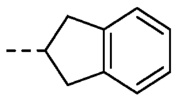, 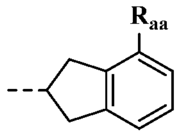,

or

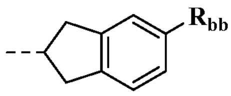;

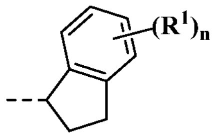

is

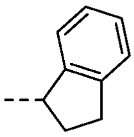, 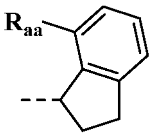, 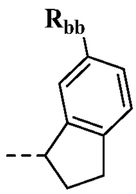, 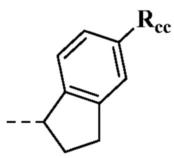, 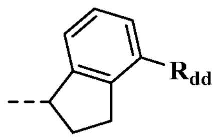,

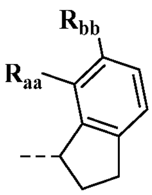, 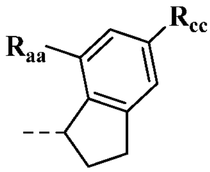, 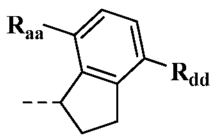, 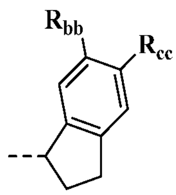, 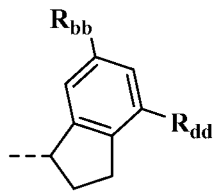,

or

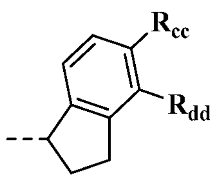,

preferably

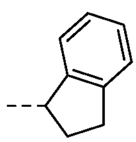, 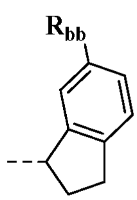, or 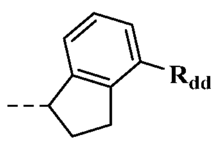;

wherein $R_{aa}$, $R_{bb}$, $R_{cc}$, and $R_{dd}$ are each independently halogen, hydroxy, $C_{1-6}$ alkyl, $C_{1-6}$ haloalkyl, $C_{1-6}$ alkoxy, or $C_{1-6}$ haloalkoxy, wherein the $C_{1-6}$ alkyl is optionally further substituted with one or more substituents selected from $C_{3-6}$ cycloalkyl, $C_{1-6}$ alkyl, or halogen; preferably halogen, hydroxy, $C_{1-3}$ alkyl, $C_{1-3}$ haloalkyl, $C_{1-3}$ alkoxy, or $C_{1-3}$ haloalkoxy; more preferably fluorine, chlorine, bromine, hydroxy, methyl, ethyl, propyl, methoxy, ethoxy, halomethyl, haloethyl, or halomethoxy; further preferably fluorine, chlorine, bromine, methyl, methoxy, or halomethoxy; still further preferably fluorine, chlorine, bromine, methyl, or methoxy.

**12.** The compound, the stereoisomer thereof, the tautomer thereof, or the pharmaceutically acceptable salt thereof according to claim 11, wherein

$R_{aa}$, $R_{bb}$, $R_{cc}$, and $R_{dd}$ are each independently halogen or $C_{1-6}$ alkyl, preferably halogen or $C_{1-3}$ alkyl, more preferably fluorine, chlorine, bromine, methyl, ethyl, or propyl, further preferably fluorine, chlorine, bromine, or methyl;
$R_{aa}$ is preferably halogen, more preferably fluorine, chlorine, or bromine, further preferably chlorine or bromine;
$R_{bb}$ is preferably halogen, more preferably fluorine, chlorine, or bromine, further preferably fluorine or chlorine;
$R_{dd}$ is preferably halogen or $C_{1-6}$ alkyl, more preferably halogen or $C_{1-3}$ alkyl, further preferably fluorine, chlorine, bromine, methyl, ethyl, or propyl, still further preferably fluorine, chlorine, bromine, or methyl.

**13.** The compound, the stereoisomer thereof, the tautomer thereof, or the pharmaceutically acceptable salt thereof according to any one of claims 10-12, wherein one or two of the following conditions are met:

(1)

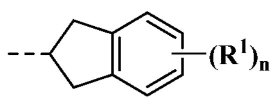

is

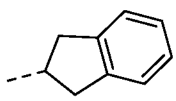, 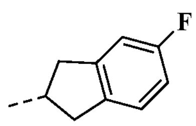, 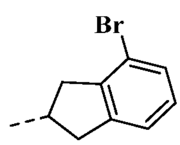, or 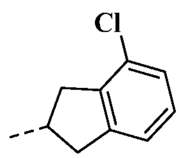;

(2)

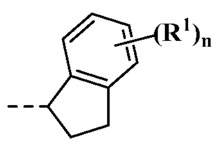

is

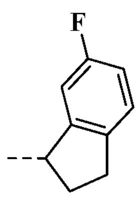, 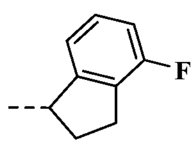, 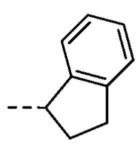, 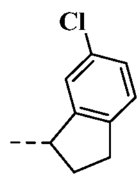, 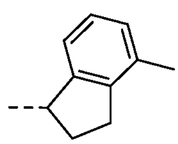,

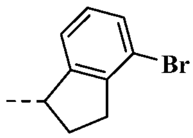, or 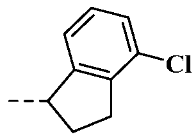.

**14.** The compound, the stereoisomer thereof, the tautomer thereof, or the pharmaceutically acceptable salt thereof according to claim 1, wherein the compound is of any one of the following structures:

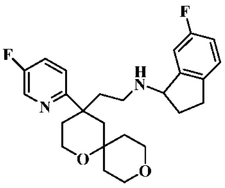
1

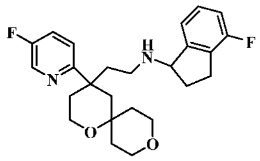
2

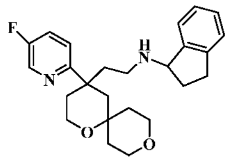
3

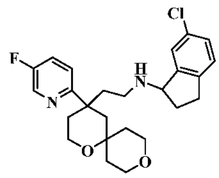
4

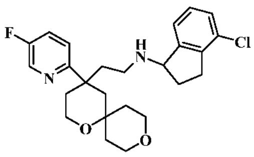
5

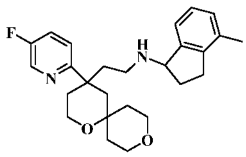
6

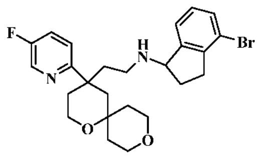
7

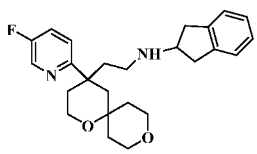
8

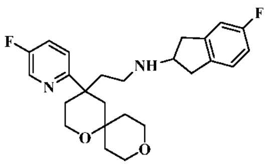
9

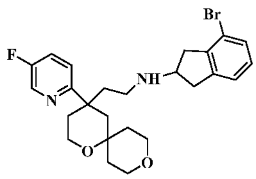
10

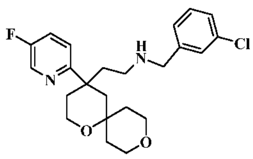
11

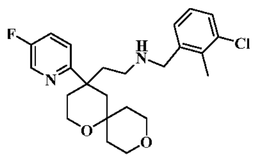
12

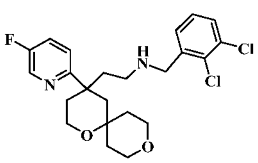
13

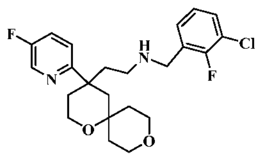
14

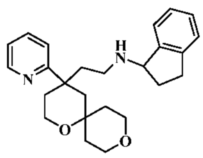
15

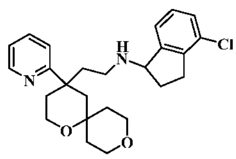
16

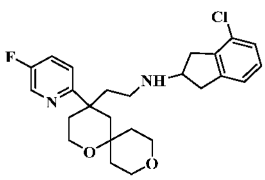
17

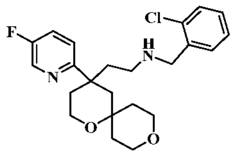
18

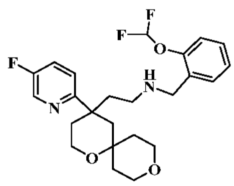
19

;

preferably, the compound is of any one of the following structures:

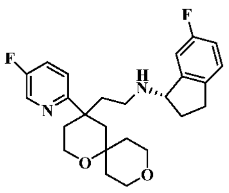
1-1

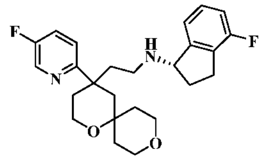
2-1

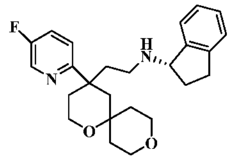
3-1

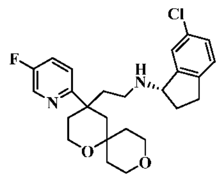
4-1

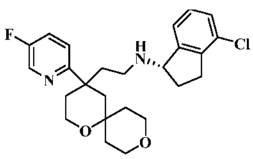
5-1

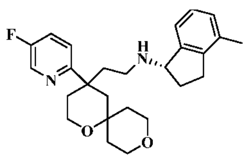
6-1

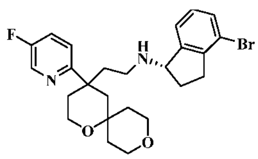
7-1

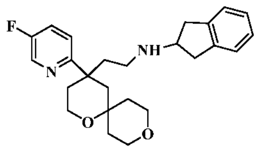
8

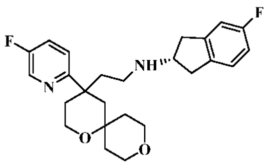
9-1

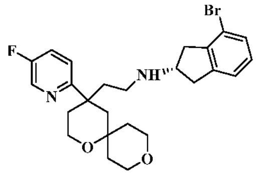
10-1

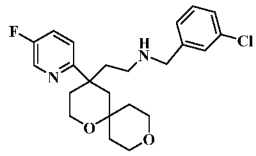
11

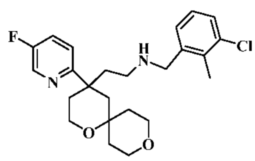
12

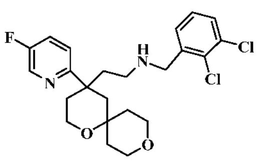
13

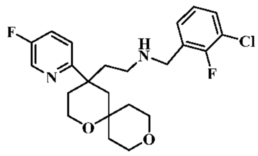
14

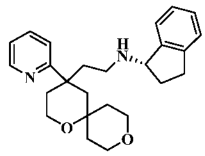
15-1

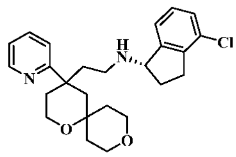
16-1

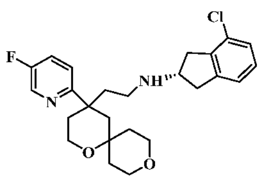
17-1

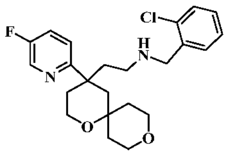
18

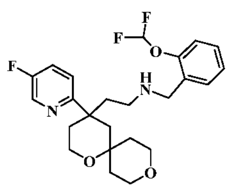
19

.

**15.** The compound, the stereoisomer thereof, the tautomer thereof, or the pharmaceutically acceptable salt thereof according to claim 14, wherein the compound is of any one of the following structures:

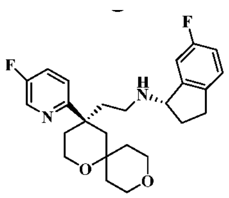
1-1-1

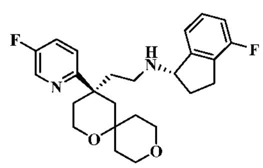
2-1-1

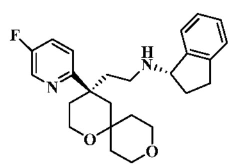
3-1-1

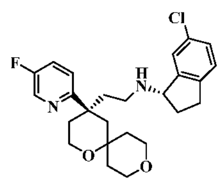
4-1-1

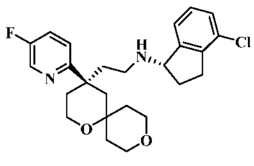
5-1-1

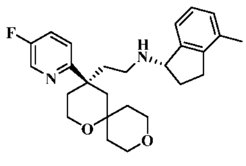
6-1-1

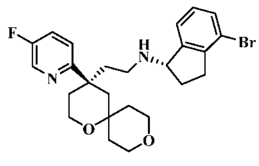
7-1-1

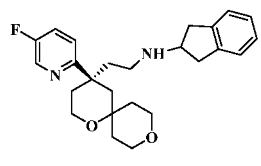
8-1

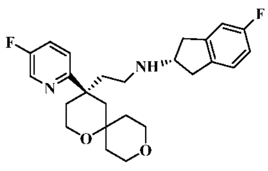
9-1-1

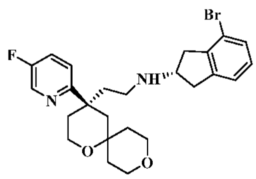
10-1-1

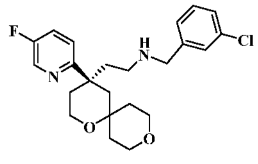
11-1

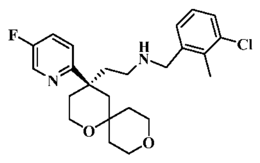
12-1

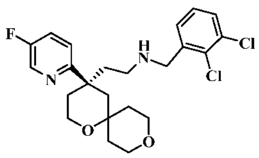
13-1

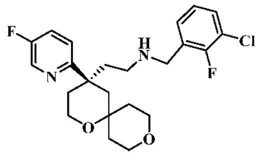
14-1

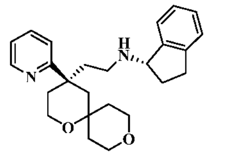
15-1-1

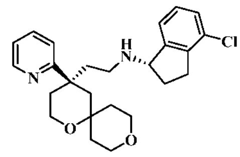
16-1-1

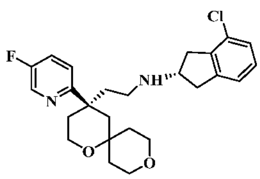
17-1-1

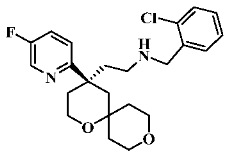
18-1

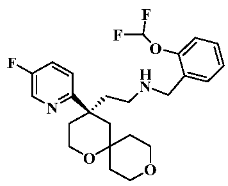
19-1

.

**16.** A method for preparing the compound of general formula (I), the stereoisomer thereof, the tautomer thereof, or the pharmaceutically acceptable salt thereof according to claim 1, comprising:

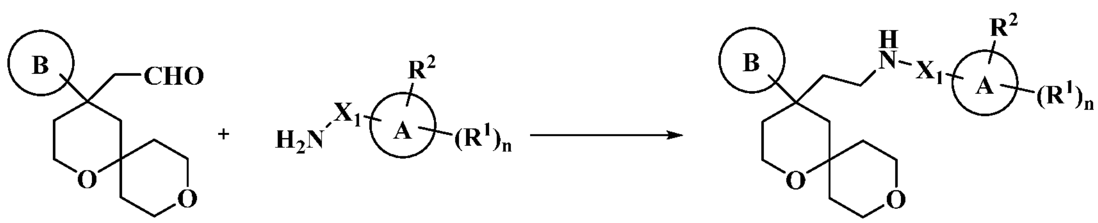
(I-A) (I-B) (I)

conducting a reductive amination reaction of a compound of general formula (I-A) with a compound of general formula (I-B) or a pharmaceutically acceptable salt thereof to give the compound of general formula (I), the stereoisomer thereof, the tautomer thereof, or the pharmaceutically acceptable salt thereof;
wherein: ring A, $R^1$, $R^2$, ring B, $X_1$, and n are as described in claim 1.

**17.** A method for preparing the compound of general formula (II), the stereoisomer thereof, the tautomer thereof, or the pharmaceutically acceptable salt thereof according to claim 6, comprising:

Method I:

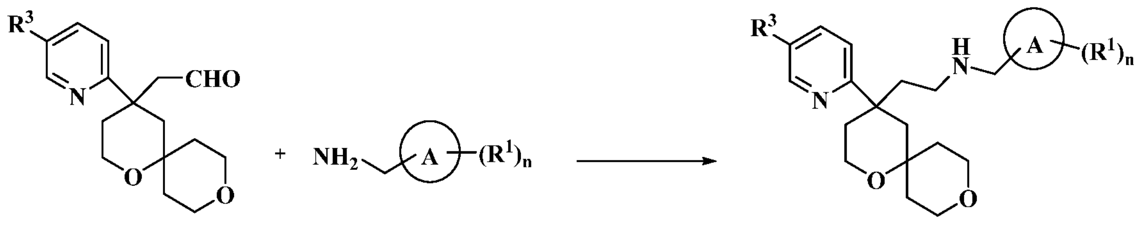

(II-A-1) (II-B-1) (II)

conducting a reductive amination reaction of a compound of general formula (II-A-1) with a compound of general formula (II-B-1) or a pharmaceutically acceptable salt thereof to give the compound of general formula (II), the stereoisomer thereof, the tautomer thereof, or the pharmaceutically acceptable salt thereof;
Method II:

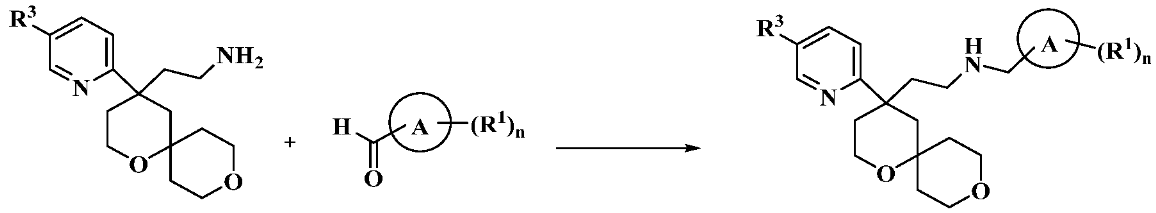

(II-A-2) (II-B-2) (II)

conducting a reductive amination reaction of a compound of general formula (II-A-2) with a compound of general formula (II-B-2) or a pharmaceutically acceptable salt thereof to give the compound of general formula (II), the stereoisomer thereof, the tautomer thereof, or the pharmaceutically acceptable salt thereof; wherein ring A, $R^1$, $R^3$, and n are as described in claim 6.

**18.** A method for preparing the compound of general formula (III), the stereoisomer thereof, the tautomer thereof, or the pharmaceutically acceptable salt thereof according to claim 10, comprising:

Method I:

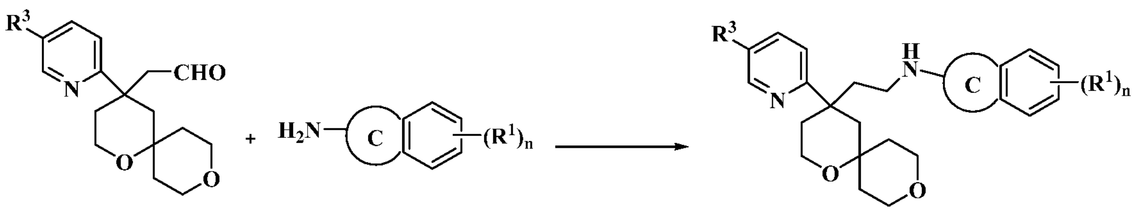

(III-A-1) (III-B-1) (III)

conducting a reductive amination reaction of a compound of general formula (III-A-1) with a compound of general formula (III-B-1) or a pharmaceutically acceptable salt thereof to give the compound of general formula (III), the stereoisomer thereof, the tautomer thereof, or the pharmaceutically acceptable salt thereof;
Method II:

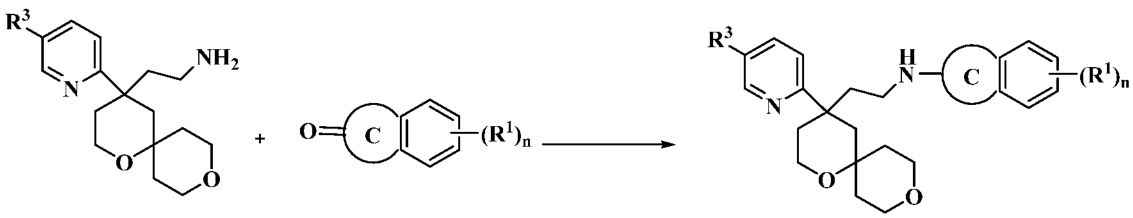

(III-A-2) (III-B-2) (III)

conducting a reductive amination reaction of a compound of general formula (III-A-2) with a compound of general formula (III-B-2) or a pharmaceutically acceptable salt thereof to give the compound of general formula (III), the stereoisomer thereof, the tautomer thereof, or the pharmaceutically acceptable salt thereof; wherein ring C, $R^1$, $R^3$, and n are as described in claim 10.

**19.** A pharmaceutical composition comprising a therapeutically effective amount of the compound, the stereoisomer thereof, the tautomer thereof, or the pharmaceutically acceptable salt thereof according to any one of claims 1-15, and at least one pharmaceutical adjuvant of a pharmaceutically acceptable carrier, diluent, or excipient.

**20.** Use of the compound, the stereoisomer thereof, the tautomer thereof, or the pharmaceutically acceptable salt thereof according to any one of claims 1-15 or the pharmaceutical composition according to claim 19 in preparing a medicament for preventing and/or treating a related disease mediated by a μ-opioid receptor agonist, wherein the related disease mediated by a μ-opioid receptor agonist is preferably selected from one or more of pain, immune dysfunction, inflammation, esophageal reflux, neurological and psychiatric diseases, urological and reproductive diseases, cardiovascular diseases, and respiratory diseases, more preferably pain.

**21.** Use of the compound, the stereoisomer thereof, the tautomer thereof, or the pharmaceutically acceptable salt thereof according to any one of claims 1-15 or the pharmaceutical composition according to claim 19 in preparing a medicament for preventing and/or treating pain or a pain-related disease, wherein the pain is preferably selected from one or more of postsurgical pain, cancer-induced pain, neuropathic pain, traumatic pain, and inflammation-induced pain.

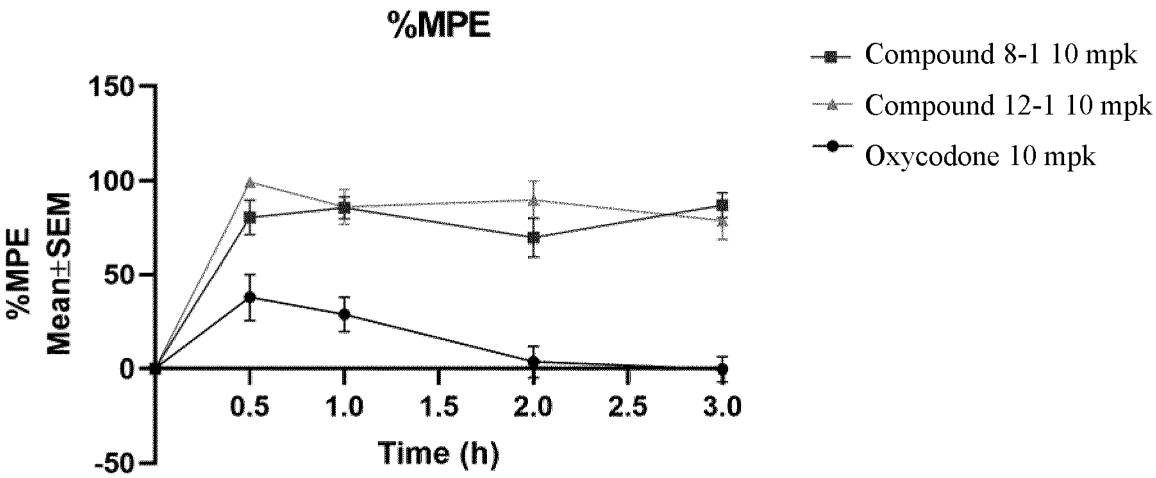
%MPE
150
100
50
0
-50
%MPE
Mean±SEM
0.5
1.0
1.5
2.0
2.5
3.0
Time (h)
Compound 8-1 10 mpk
Compound 12-1 10 mpk
Oxycodone 10 mpk

FIG. 1

**INTERNATIONAL SEARCH REPORT**

International application No.
**PCT/CN2022/109503**

**A. CLASSIFICATION OF SUBJECT MATTER**

C07D 493/10(2006.01)i; A61K 31/35(2006.01)i; A61K 31/4433(2006.01)i; A61P 25/04(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, STN: 阿片, 氧杂, 螺, 镇痛, 疼痛, 枢境, 恩华, 结构检索, structure search, oxa, spiro, opioid, MOR, GPCR, pain

**C. DOCUMENTS CONSIDERED TO BE RELEVANT**

| Category* | Citation of document, with indication, where appropriate, of the relevant passages | Relevant to claim No. |
|---|---|---|
| X | WO 2019072235 A1 (MEDSHINE DISCOVERY INC.) 18 April 2019 (2019-04-18)<br>claims 1-15, and embodiment | 1-21 |
| X | CN 109206417 A (SICHUAN HAISCO PHARMACEUTICAL CO., LTD.) 15 January 2019 (2019-01-15)<br>claims 1-23 | 1-21 |
| A | WO 2019205983 A1 (SICHUAN KELUN-BIOTECH BIOPHARMACEUTICAL CO., LTD.) 31 October 2019 (2019-10-31)<br>claims 1-22 | 1-21 |
| A | CN 107001347 A (JIANGSU HENGRUI MEDICINE CO., LTD. et al.) 01 August 2017 (2017-08-01)<br>abstract, and claims 1-16 | 1-21 |
| A | WO 2012129495 A1 (TREVENA INC. et al.) 27 September 2012 (2012-09-27)<br>claims 1-75 | 1-21 |
| A | WO 2021143801 A1 (SHANGHAI HAIYAN PHARMACEUTICAL TECH. CO., LTD. et al.) 22 July 2021 (2021-07-22)<br>claims 1-51 | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2022** | **10 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/109503**

| Patent document cited in search report | Publication date (day/month/year) | Patent family member(s) | Publication date (day/month/year) |
|---|---|---|---|
| WO 2019072235 A1 | 18 April 2019 | CN 111148515 A | 12 May 2020 |
| | | AU 2018349930 A1 | 16 April 2020 |
| | | US 2020255444 A1 | 13 August 2020 |
| CN 109206417 A | 15 January 2019 | None | |
| WO 2019205983 A1 | 31 October 2019 | CN 111836807 A | 27 October 2020 |
| CN 107001347 A | 01 August 2017 | BR 112018006448 A2 | 16 October 2018 |
| | | PL 3354649 T3 | 27 July 2020 |
| | | DK 3354649 T3 | 24 February 2020 |
| | | MX 2018004175 A | 06 June 2018 |
| | | US 2018297988 A1 | 18 October 2018 |
| | | KR 20180063251 A | 11 June 2018 |
| | | WO 2017063509 A1 | 20 April 2017 |
| | | RU 2018115569 A | 18 November 2019 |
| | | PT 3354649 T | 03 February 2020 |
| | | AU 2016339404 A1 | 24 May 2018 |
| | | HU E048032 T2 | 28 May 2020 |
| | | TW 201718558 A | 01 June 2017 |
| | | CA 3000761 A1 | 20 April 2017 |
| | | JP 2018534257 A | 22 November 2018 |
| | | ES 2772689 T3 | 08 July 2020 |
| | | EP 3354649 A1 | 01 August 2018 |
| WO 2012129495 A1 | 27 September 2012 | DK 3290415 T3 | 22 March 2021 |
| | | EP 3290415 A1 | 07 March 2018 |
| | | PL 3290415 T3 | 19 July 2021 |
| | | AU 2017200745 A1 | 23 February 2017 |
| | | NZ 615993 A | 27 November 2015 |
| | | AU 2012230761 A1 | 02 May 2013 |
| | | US 2015246904 A1 | 03 September 2015 |
| | | IL 228506 D0 | 31 December 2013 |
| | | US 2022175747 A1 | 09 June 2022 |
| | | ME 02754 B | 20 January 2018 |
| | | JP 2021176885 A | 11 November 2021 |
| | | LT 2688403 T | 25 July 2017 |
| | | US 2012245181 A1 | 27 September 2012 |
| | | SI 2688403 T1 | 31 August 2017 |
| | | EP 3872076 A1 | 01 September 2021 |
| | | NZ 713143 A | 26 May 2017 |
| | | CA 2830742 A1 | 27 September 2012 |
| | | KR 20180100453 A | 10 September 2018 |
| | | US 2017326124 A1 | 16 November 2017 |
| | | US 2013331408 A1 | 12 December 2013 |
| | | HU E032948 T2 | 28 November 2017 |
| | | HR P20171021 T1 | 22 September 2017 |
| | | RS 56111 B1 | 31 October 2017 |
| | | US 2019343819 A1 | 14 November 2019 |
| | | US 2018311222 A1 | 01 November 2018 |
| | | IL 272165 A | 31 March 2020 |
| | | PL 2688403 T3 | 29 September 2017 |
| | | ES 2632009 T3 | 07 September 2017 |
| | | BR 112013024136 A2 | 14 January 2020 |

Form PCT/ISA/210 (patent family annex) (January 2015)

**INTERNATIONAL SEARCH REPORT**
**Information on patent family members**

International application No.
**PCT/CN2022/109503**

| Patent document cited in search report | Publication date (day/month/year) | Patent family member(s) | Publication date (day/month/year) |
|---|---|---|---|
| | | EP 2688403 A1 | 29 January 2014 |
| | | CN 103702561 A | 02 April 2014 |
| | | JP 2018048193 A | 29 March 2018 |
| | | CY 1119057 T1 | 10 January 2018 |
| | | PT 3290415 T | 29 March 2021 |
| | | DK 2688403 T3 | 24 July 2017 |
| | | JP 2016155843 A | 01 September 2016 |
| | | HU E054055 T2 | 30 August 2021 |
| | | JP 2014508811 A | 10 April 2014 |
| | | HK 1250984 A1 | 18 January 2019 |
| | | JP 2020073570 A | 14 May 2020 |
| | | KR 20140047599 A | 22 April 2014 |
| | | KR 20190072665 A | 25 June 2019 |
| | | PT 2688403 T | 13 July 2017 |
| | | IL 243318 D0 | 29 February 2016 |
| | | IL 284407 A | 31 August 2021 |
| | | EA 201391332 A1 | 30 April 2014 |
| | | US 2016250199 A1 | 01 September 2016 |
| | | ES 2857549 T3 | 29 September 2021 |
| WO 2021143801 A1 | 22 July 2021 | CN 113412263 A | 17 September 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110895052X
- CN 202110888850X